# EUROPEAN PATENT SPECIFICATION

(11) **EP 2 123 759 B1**
(45) Date of publication and mention of the grant of the patent: **15.01.2014**
(21) Application number: 09010444.9
(22) Date of filing: 04.02.2005
(51) Int. Cl.: C12N 15/11, A61K 31/712, A61K 31/7125, A61K 31/713, A61K 38/00, C12N 15/113

(54) **Stabilized RNAS as transfection controls and silencing reagents**
Stabilisierte RNA als Transfektionskontrollen und Dämpfungsreagenzien
ARN stabilisés en tant que contrôles de la transfection et réactifs silencieux

(30) Priority: 06.02.2004 US 542646 P; 10.02.2004 US 543640 P; 18.05.2004 US 572270 P
(43) Date of publication of application: 25.11.2009
(62) Divisional of application: 05712710.2
(73) Proprietor: Thermo Fisher Scientific Biosciences Inc., Lafayette, CO 80026 (US)
(72) Inventor: Leake, Devin, Denver, Colorado 80238 (US); Khvorova, Anastasia, Boulder CO 80305 (US); Fedorov, Yuriy, Superior CO 80027 (US); Delaney, Michael O., Firestone CO 80504 (US); Roberson, Barbara, Boulder CO 80304 (US); Anderson, Emily, Lafayette, CO 80026 (US)
(74) Representative: Bentham, Andrew

(56) References cited:
- WO-A-02/44321
- WO-A-03/012052
- WO-A2-2004/090105
- HOLEN T ET AL: "Similar behaviour of single-strand and double-strand siRNAs suggests they act through a common RNAi pathway", NUCLEIC ACIDS RESEARCH, OXFORD UNIVERSITY PRESS, SURREY, GB, vol. 31, no. 9, 1 May 2003 (2003-05-01), pages 2401-2407, XP002283906, ISSN: 0305-1048
- AMARZGUIOUI M. ET AL.: "Tolerance for mutations and chemical modifications in a siRNA", NUCLEIC ACIDS RESEARCH, vol. 31, no. 2, 15 January 2003 (2003-01-15), pages 589-595, XP002270887, ISSN: 0305-1048
- CHIU Y.-L. AND RANA T.M.: "RNAi in human cells: basic structural and functional features of small interfering RNA", MOLECULAR CELL, CELL PRESS, CAMBRIDGE, MA, US, vol. 10, September 2002 (2002-09), pages 549-561, XP002978510, ISSN: 1097-2765
- CZAUDERNA F ET AL: "Structural variations and stabilising modifications of synthetic siRNAs in mammalian cells", NUCLEIC ACIDS RESEARCH, OXFORD UNIVERSITY PRESS, SURREY, GB, vol. 31, no. 11, 1 June 2003 (2003-06-01), pages 2705-2716, XP002270732, ISSN: 0305-1048
- ELBASHIR S.M. ET AL.: "Functional anatomy of siRNAs for mediating efficient RNAi in Drosophila melanogaster embryo lysate", EMBO JOURNAL, OXFORD UNIVERSITY PRESS, SURREY, GB, vol. 20, no. 23, 3 December 2001 (2001-12-03), pages 6877-6888, XP002225998, ISSN: 0261-4189
- HOLEN T. ET AL.: "Positional effects of short interfering RNAs targeting the human coagulation trigger Tissue Factor", NUCLEIC ACIDS RESEARCH, vol. 30, no. 8, 15 April 2002 (2002-04-15) , pages 1757-1766, XP002232890, ISSN: 0305-1048
- GAIT M.J. ET AL.: "Oligoribonucleotide synthesis", OLIGONUCLEOTIDES AND ANALOGUES - A PRACTICAL APPROACH; ECKSTEIN F., 1 January 1991 (1991-01-01), pages 25-48, XP002325585,
- NYKÄNEN A. ET AL.: "ATP requirements and small interfering RNA structure in the RNA interference pathway", CELL, vol. 107, 2 November 2001 (2001-11-02), pages 309-321, XP002969117, ISSN: 0092-8674
- HARBORTH J. ET AL.: "Sequence, chemical, and structural variation of small interfering RNAs and short hairpin RNAs and the effect on mammalian gene silencing", ANTISENSE & NUCLEIC ACID DRUG DEVELOPMENT, vol. 13, no. 2, April 2003 (2003-04), pages 83-105, XP002284355, ISSN: 1087-2906
- SONG E ET AL: "RNA interference targeting Fas protects mice from fulminant hepatitis", NATURE MEDICINE, NATURE PUBLISHING GROUP, NEW YORK, NY, US, vol. 9, no. 3, 1 March 2003 (2003-03-01), pages 347-351, XP002351920, ISSN: 1078-8956
- CHIU Y-L AND RANA T M: "siRNA function in RNAi: a chemical modification analysis", RNA, COLD SPRING HARBOR LABORATORY PRESS, US, vol. 9, 1 January 2003 (2003-01-01), pages 1034-1048, XP002336178, ISSN: 1355-8382, DOI: 10.1261/RNA.5103703

## Description

### FIELD OF THE INVENTION

The present invention relates to RNA interference.

### CROSS-REFERENCE TO RELATED APPLICATIONS

This application claims the benefit of U.S. Provisional Application No. 60/542,646, filed February 6, 2004, U.S. Provisional Application No. 60/543,640, filed February 10, 2004, and U.S. Provisional Application No. 60/572,270, filed May 18, 2004.

### BACKGROUND

RNA interference ("RNAi") is an important biological pathway that has practical applications in the fields of functional gene analysis, drug target validation, and therapeutics. Genetic and biochemical studies of this form of post-transcriptional gene silencing ("PTGS") have led to the discovery of an RNA Interfering Silencing Complex ("RISC"), which partners with short interfering RNAs ("siRNAs") to mediate sequence specific degradation of target transcripts.

Four major issues to consider when working with siRNAs include: (i) functionality; (ii) specificity; (iii) delivery methods; and (iv) stability. Functionality refers to the ability of a particular siRNA to silence the desired target. Methods for improving functionality include for example, the subject matter of U.S. Patent Application US 2007/0031844. Specificity refers to the ability of a particular siRNA to silence a desired target and only the desired target. Thus, specificity refers to minimizing off-target effects. Delivery methods are the means by which a user introduces a particular siRNA into a cell and may, for example, include using vectors and/or modifications of the siRNA itself Stabilization refers to the ability of an siRNA to resist degradation by enzymes and other harmful substances that exist in intra-cellular and extra-cellular environments, and is the subject of U.S. Patent Application 2004/0266707. For example, when naked siRNA molecules are introduced into blood, serum, or serum-containing media, they are not stable and are nearly immediately degraded, which reduces or eliminates their effectiveness.

Although some beneficial stabilization modifications have already been proposed, there is always a need to optimize further the stabilization of siRNA. Additionally, as the RNAi industry grows, users will become more and more aware of the need for controls useful for incorporation into experiments to ensure reliable data; controls that provide information concerning transfection efficiency are particularly critical. The present invention addresses these needs.
Amarzguioui et al. (Nucleic Acids Research vol. 31 no. 2 15 January 2003 pages 589-595) describes a study exploring the tolerance to mutations and chemical modifications in various parts of the two 21-nt strands of a siRNA targeting the blood clotting initiator Tissue Factor. Czauderna et al. (Nucleic Acids Research vol. 31 no. 11 1 June 2003 pages 2705-2716) describes a study investigating the structural requirements of chemically synthesized siRNAs to mediate efficient gene silencing in mammalian cells. Chiu and Rana (RNA vol. 9 2003 pages 1034-1048) describes a study in which various chemical modifications were created in siRNAs to determine the biochemical properties required for RNA interference. Elbashir et al. (Embo Journal vol. 20 no. 23 2001 pages 6877-6888) describes a study in which synthetic siRNAs were examined in *Drosophilia melanogaster* embryo lysate for their requirements regarding length, structure, chemical composition and sequence in order mediate efficient RNAi.

### SUMMARY OF THE INVENTION

The present invention is directed to stabilized RNAs, including siRNAs, that may be used as transfection controls or exaequo agents.

According to a first embodiment that is related to, but not part, of the present invention" there is provided an siRNA comprising:
a. a sense strand, wherein said sense strand comprises a first 5' terminal sense nucleotide and a second 5' terminal sense nucleotide, wherein said first 5' terminal sense nucleotide comprises a first 2'-O-alkyl sense modification and said second 5' terminal sense nucleotide comprises a second 2'-O-alkyl sense modification; and at least one 2'-O-alkyl pyrimidine modified sense nucleotide, wherein said at least one 2'-O-alkyl pyrimidine modified sense nucleotide is a nucleotide other than said first 5' terminal sense nucleotide or said second 5' terminal sense nucleotide; and
b. an antisense strand, wherein said antisense strand comprises at least one 2' halogen modified pyrimidine nucleotide; and a first 5' terminal antisense nucleotide, wherein said first 5' terminal antisense nucleotide is phosphorylated at its 5' carbon position.

The molecules of the first embodiment may be used to silence a target and/or as a control. These molecules may further comprise a label, such as a fluorescent label and/or a third 5' terminal sense nucleotide that comprises a third 2'-O-alkyl sense modification. Optionally, the 5' end of the sense strand comprises a 5' carbon blocking group that prevents phosphorylation of the first terminal sense nucleotide,

According to a second embodiment, the present invention provides an RNA duplex comprising:
(a) a sense strand, wherein said sense strand comprises (i) a first 5' terminal sense nucleotide and a second 5' terminal sense nucleotide, wherein said first 5' terminal sense nucleotide comprises a first 2'-O-alkyl sense modification and said second 5' terminal sense nucleotide comprises a second 2'-O-alkyl sense modification; (ii) at least one 2'-O-alkyl pyrimidine modified sense nucleotide, wherein said at least one 2'-O-alkyl pyrimidine modified sense nucleotide is a nucleotide other than said first 5' terminal sense nucleotide or said second 5' terminal sense nucleotide and such that each pyrimidine on said sense strand comprises a 2'-O-alkyl modification; and
(b) an antisense strand, wherein said antisense strand comprises (i) a first 5' terminal antisense nucleotide and a second 5' terminal antisense nucleotide, wherein said first 5' terminal antisense nucleotide comprises a first 2'-O-alkyl antisense modification and said second 5' terminal antisense nucleotide comprises a second 2'-O-alkyl antisense modification; and (ii) at least one 2'-O-alkyl pyrimidine modified antisense nucleotide, wherein said at least one 2'-O-alkyl modified antisense nucleotide is a nucleotide other than said first 5' terminal antisense nucleotide or said second 5' terminal antisense nucleotide and such that each pyrimidine on said antisense strand comprises a 2'-O-alkyl modification,
wherein the sense strand and antisense strand are capable of forming a duplex of between 16 and 50 base pairs; wherein all nucleotides within the duplex other than said first 5' terminal sense nucleotide, second 5' terminal sense nucleotide, each pyrimidine on said sense strand, first 5' terminal antisense nucleotides, second 5' terminal antisense nucleotides and each pyrimidine on said antisense strand are unmodified; and wherein the first 5' terminal antisense nucleotide is not phosphorylated.

Preferably, the molecules of the second embodiment also comprise a label that may for example be a fluorescent dye, and/or comprise a third 5' terminal sense nucleotide that comprises a third 2'-O-alkyl sense modification, and/or a third 5' terminal antisense nucleotide that comprises a third 2'-O-alkyl antisense modification. The 5' terminal sense and antisense nucleotides may contain a blocking group (e.g., an O-alkyl group such as, for example, a 2'-O-methyl group) that prevents cellular kinases from adding a phosphate group to the 5' carbon position of the first 5' terminal antisense nucleotide inside of a cell or inside of an organism. Preferably, the size of said exaequo agents is 16 to 30 base pairs.

Optionally, in the second embodiment, the sense strand comprises a third 5' terminal sense nucleotide comprising a third 2'-O-alkyl sense modification, and/or the antisense strand comprises a third 5' terminal antisense nucleotide comprising a third 2'-O-alkyl antisense modification.

A third embodiment includes another example of a control or exaequo agent, namely an RNA duplex comprising:
(a) a sense strand, wherein said sense strand comprises (i)a first 5' terminal sense nucleotide and a second 5' terminal sense nucleotide, wherein said first 5' terminal sense nucleotide comprises a first 2'-O-alkyl sense modification, and said second 5' terminal sense nucleotide comprises a second 2'-O- alkyl sense modification;
b) an antisense strand, wherein said antisense strand comprises (i) a first 5' terminal antisense nucleotide and a second 5' terminal antisense nucleotide, wherein said first 5' terminal antisense nucleotide comprises a first 2'-O-alkyl antisense modification, and said second 5' terminal antisense nucleotide comprises a second 2'-O-alkyl antisense modification;
wherein: neither the sense strand nor the antisense strand is phosphorylated at the 5' carbon position of their first 5' terminal nucleotides; the sense and/or antisense strand may comprise a blocking group at the 5' carbon position of the first 5' terminal nucleotide; the sense strand and antisense strand are capable of forming a duplex of 16 to 30 base pairs; and all nucleotides within the duplex other than said first 5' terminal sense nucleotide, second 5' terminal sense nucleotide, first 5' terminal antisense nucleotide and second 5' terminal antisense nucleotide are unmodified.

In a fourth embodiment related to the present invention, a control or exaequo agent containing the following modifications is provided: (i) a first and second, or first, second and third, 5' terminal sense nucleotides that each comprises 2' modifications, such as the 2' modifications described above, for example 2'-O-methyl modifications; (ii) a first and second, or first, second and third, 5' terminal antisense nucleotides that each comprises 2' modifications, such as the 2' modifications described above, for example 2'-O-methyl modifications; (iii) at least one 2'-O-alkyl pyrimidine modified sense nucleotide, wherein said at least one 2'-O-alkyl pyrimidine modified sense nucleotide is a nucleotide other than said first 5' terminal sense nucleotide, said second 5' terminal sense nucleotide, or said third 5' terminal sense nucleotide; (iv) at least one 2' halogen modified pyrimidine antisense nucleotide wherein said at least one 2' halogen modified nucleotide is a nucleotide other than said first, second, or third terminal antisense nucleotides and said halogen is preferably a fluorine atom; and (v) the 5' terminal antisense and sense nucleotides have not been phosphorylated at the 5' carbon position and may contain a blocking group (*e.g*., an O-alkyl group) that prevents cellular kinases from adding a phosphate group to the 5' terminal antisense and sense positions. The modifications can be applied to molecules of varying sizes. Preferably, the size of said exaequo agents ranges between 16 and 30 base pairs.

A fifth embodiment related to the present invention is an RNA duplex comprising.
a. a sense strand, wherein said sense strand comprises
   a first 5' terminal sense nucleotide, a second 5' terminal sense nucleotide, and a third 5' terminal sense nucleotide, wherein said first 5' terminal sense nucleotide comprises a first 2'-O-alkyl sense modification, said second 5' terminal sense nucleotide comprises a second 2'-O-alkyl sense modification, and said third 5' terminal sense nucleotide optionally comprises a third 2'-O-alkyl sense modification;
b. an antisense strand, wherein said antisense strand comprises
   a first 5' terminal antisense nucleotide, a second 5' terminal antisense nucleotide, and a third 5' terminal antisense nucleotide, wherein said first 5' terminal antisense nucleotide comprises a first 2'-O-alkyl antisense modification, said second 5' terminal antisense nucleotide comprises a second 2'-O-alkyl antisense modification, and said third 5' terminal antisense nucleotide optionally comprises a third 2'-O-alkyl antisense modification;
wherein neither the sense strand nor the antisense strand have been phosphorylated at the 5' carbon position of the first 5' terminal nucleotide and may comprise a blocking group at the 5' carbon position of the first 5' terminal nucleotide of the sense and/or antisense strand, and the sense strand and antisense strand are capable of forming a duplex of 16 to 30 base pairs.

A sixth embodiment related to the present invention is an RNA duplex comprising:
a. a sense strand, wherein said sense strand comprises
   a first 5' terminal sense nucleotide, a second 5' terminal sense nucleotide, and at least one 2'-O-alkyl pyrimidine modified sense nucleotide that is not the first or second 5' terminal sense nucleotide, wherein said first 5' terminal sense nucleotide comprises a first 2'-O-alkyl sense modification and said second 5' terminal sense nucleotide comprises a second 2'-O-alkyl sense modification; and
b. an antisense strand, wherein said antisense strand comprises
   a first 5' terminal antisense nucleotide, a second 5' terminal antisense nucleotide, and at least one 2' halogen modified pyrimidine antisense nucleotide that is not the first or second 5' terminal antisense nucleotide, wherein said first 5' terminal antisense nucleotide comprises a first 2'-O-alkyl antisense modification and said second 5' terminal antisense nucleotide comprises a second 2'-O-alkyl antisense modification;
wherein neither the sense strand nor the antisense strand have been phosphorylated at the 5' carbon position of the first 5' terminal nucleotide and may comprise a blocking group at the 5' carbon position of the first 5' terminal nucleotide of the sense and/or antisense strand, and the sense strand and antisense strand are capable of forming a duplex of l.d to 30 base pairs.

According to a seventh embodiment, the present invention provides an RNA duplex comprising:
a a sense strand, wherein said sense strand comprises
   a first 5' terminal sense nucleotide, a second 5' terminal sense nucleotide, a third 5' terminal nucleotide, and at least one 2'-O-alkyl pyrimidine modified sense nucleotide that is not the first, second, or third terminal sense nucleotide, wherein said first 5' terminal sense nucleotide comprises a first 2'-O-alkyl sense modification, said second 5' terminal sense nucleotide comprises a second 2'-O-alkyl sense modification, and said third 5' terminal sense nucleotide comprises a third 2'-O-akyl sense modification; and
b. an antisense strand, wherein said antisense strand comprises
   a first 5' terminal antisense nucleotide, a second 5' terminal antisense nucleotide, a third 5' terminal antisense nucleotide, and at least one 2' halogen modified pyrimidine antisense nucleotide that is not the first, or second, or third terminal antisense nucleotide, wherein said first 5' terminal antisense nucleotide comprises a first 2'-O-alkyl antisense modification, said second 5' terminal antisense nucleotide comprises a second 2'-O-alkyl antisense modification, and said third 5' terminal antisense nucleotide comprises a third 2'-O-alkyl antisense modification;
wherein neither the sense strand nor the antisense strand is phosphorylated at the 5' carbon position of their first 5' terminal nucleotide and the sense and/or antisense strand may comprise a blocking group at the 5' carbon position of the first 5' terminal nucleotide, and the sense strand and antisense strand are capable of forming a duplex of 16 to 30 base pairs.

An eighth embodiment related to the present invention is an RNA duplex comprising:
a. a sense strand, wherein said sense strand comprises
   a first 5' terminal sense nucleotide, a second 5' terminal sense nucleotide, and a third 5' terminal sense nucleotide, wherein said first 5' terminal sense nucleotide comprises a first 2'-O-methyl sense modification, said second 5' terminal sense nucleotide comprises a second 2'-O-methyl sense modification, and said third 5' terminal sense nucleotide optionally comprises a third 2'-O-methyl sense modification; and
b. an antisense strand, wherein said antisense strand comprises
   a first 5' terminal antisense nucleotide, a second 5' terminal, antisense nucleotide, and a third 5' terminal antisense nucleotide, wherein said first 5' terminal antisense nucleotide comprises a first 2'-O-methyl antisense modification, said second 5' terminal antisense nucleotide comprises a second 2'-O-methyl antisense modification, and said third 5' terminal antisense nucleotide optionally comprises a third 2'-O-methyl modification;
wherein neither the sense strand nor the antisense strand have been phosphorylated at the 5' carbon position of the first 5' terminal nucleotide and may comprise a blocking group at the 5' carbon position of the first 5' terminal nucleotide of the sense and/or antisense strand, and the sense strand and antisense strand are capable of forming a duplex of 16 to 30 base pairs.

A ninth embodiment related to the present invention is an RNA duplex comprising:
a. a sense strand, wherein said sense strand comprises
   a first 5' terminal sense nucleotide, a second 5' terminal sense nucleotide, and at least one 2'-O-methyl pyrimidine modified sense nucleotide that is not the first or second terminal sense nucleotide, wherein said first 5' terminal sense nucleotide comprises a first 2'-O-methyl sense modification and said second 5' terminal sense nucleotide comprises a second 2'-O-metliyl sense modification; and
b. an antisense strand, wherein said antisense strand comprises
   a first 5' terminal antisense nucleotide, a second 5' terminal antisense nucleotide, and at least one 2' fluorine modified pyrimidine antisense nucleotide that is not the first or second terminal antisense nucleotide, wherein said first 5' terminal antisense nucleotide comprises a first 2'-O-methyl antisense modification and said second 5' terminal antisense nucleotide comprises a second 2'-O-methyl antisense modification;
wherein neither the sense strand nor the antisense strand have been phosphorylated at the 5' carbon position of the first 5' terminal nucleotide and may comprise a blocking group at the 5' carbon position of the first 5' terminal nucleotide of the sense and/or antisense strand, and the sense strand and antisense strand are capable of forming a duplex of 16 to 30 base pairs.

A tenth embodiment related to the present invention is an RNA duplex comprising:
a. a sense strand, wherein said sense strand comprises
   a first 5' terminal sense nucleotide, a second 5' terminal sense nucleotide, a third 5' terminal sense nucleotide, and at least one 2'-O-methyl pyrimidine modified sense nucleotide that is not the first, or second, or third terminal sense nucleotide, wherein said first 5' terminal sense nucleotide comprises a first 2'-O-methyl sense modification, said second 5' terminal sense nucleotide comprises a second 2'-O-methyl sense modification, and said third terminal sense nucleotide comprises a third 2'-O methyl sense modification; and
b. an antisense strand, wherein said antisense strand comprises
   a first 5' terminal antisense nucleotide, a second 5' terminal antisense nucleotide, a third 5' terminal antisense nucleotide, and at least one 2' fluorine modified pyrimidine antisense nucleotide that is not the first, second, or third terminal antisense nucleotide, wherein said first 5' terminal antisense nucleotide comprises a first 2'-O-methyl antisense modification, said second 5' terminal antisense nucleotide comprises a second 2'-O-methyl antisense modification, and said third 5' terminal antisense nucleotide comprises a third 2'-O-methyl antisense modification;
wherein neither the sense strand nor the antisense strand have been phosphorylated at the 5' carbon position of the first 5' terminal nucleotide and may comprise a blocking group at the 5' carbon position of the first 5' terminal nucleotide of the sense and/or antisense strand, and the sense strand and antisense strand are capable of forming a duplex of 16 to 30 base pairs.

### BRIEF DESCRIPTION OF THE FIGURES

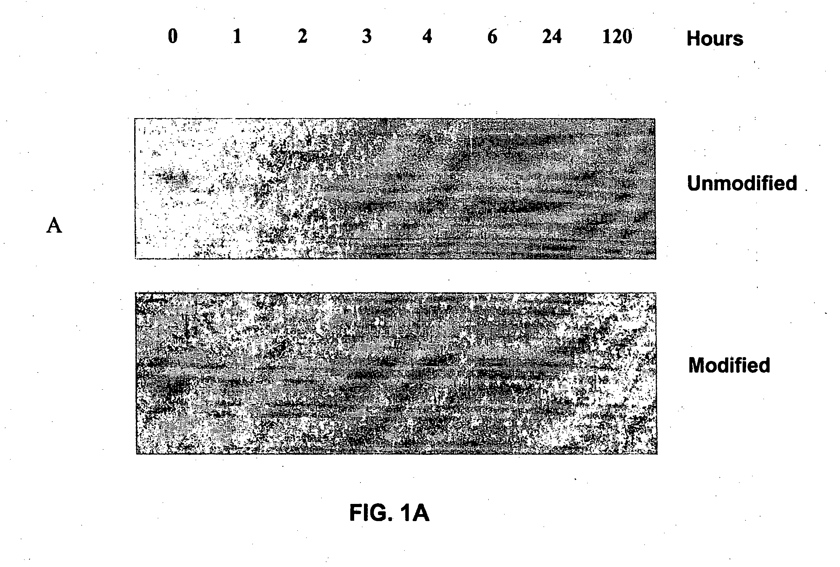

**Figure 1A** is a representation of results from a typical serum stability experiment that shows an ethidium bromide stained gel containing siRNA (unmodified, top, and "molecule 1 modifications" modified, bottom) that have been exposed to serum.

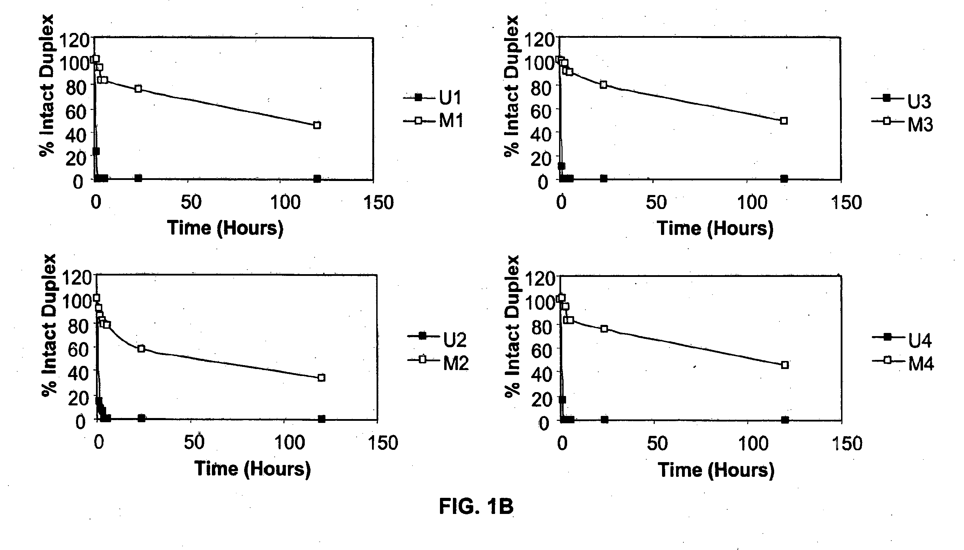

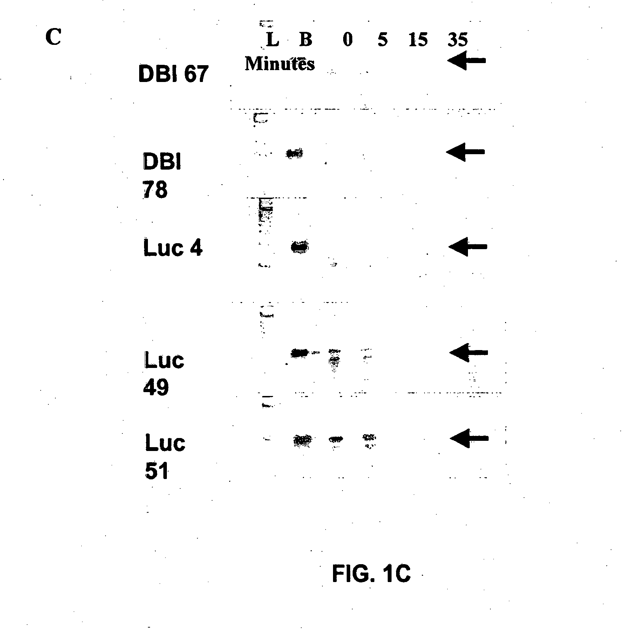

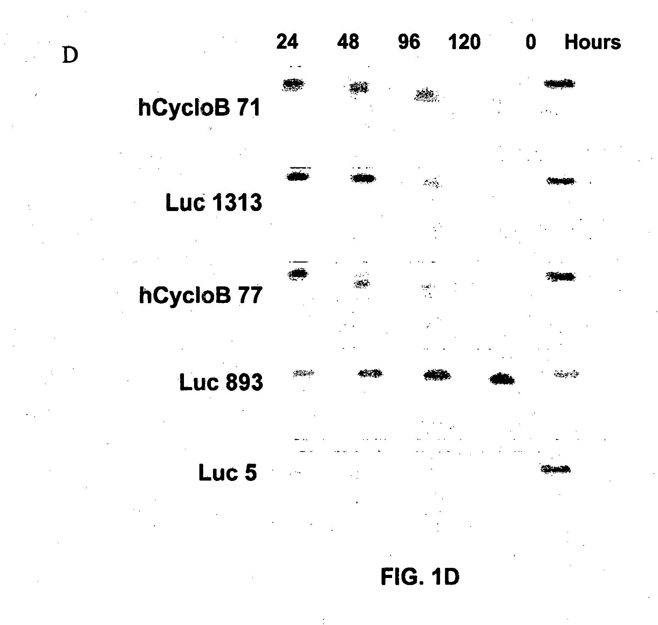

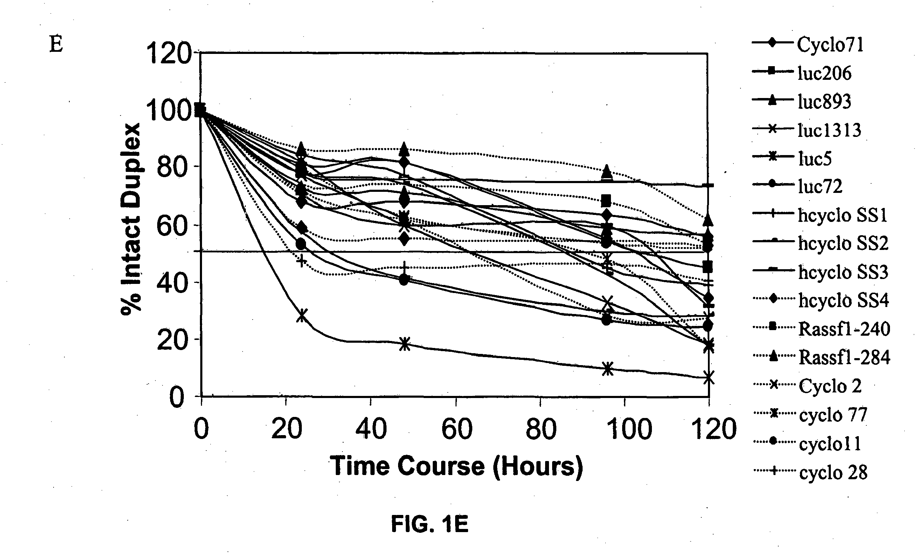

**Figures 1B** shows a set of line graphs that plot the relative stability of four human cyclophilin B siRNA in modified and unmodified formsol - 5'gaaagagcau cuacgguga (SEQ. ID NO. 1), U2 = 5'gaaaggauuug gcuacaaa (SEQ. ID NO. 2), U3 = 5'acagcaaauu ccaucgugu (SEQ. ID NO. 3), and U4 = 5'ggaaagacug uuccaaaaa, sense strand (SEQ. ID NO. 4). The X-axis represents time in hours. The Y-axis represents percentage of duplexes that remain intact. Black squares represent unmodified sequences. **Figure 1C** demonstrates the relative instability of five sequences in the presence of 100% human serum, **Figure 1D** is a gel showing the improved stability of 5 sequences (hcyclo B71, luc 1313, hcyclo B 77, luc 893, and luc 5). The small down shift observed in some sequences (bands) suggests cleavage of the 2 unmodified nucleotide 3' overhang. **Figure 1E** is a graph showing the stability of 16 different sequences (carrying molecule 1 modifications, see Examples) over the course of 120 hours.

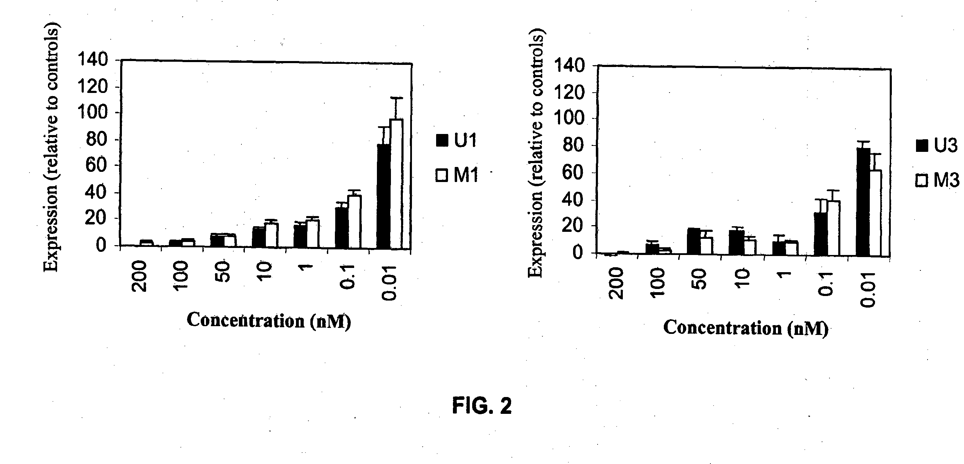

**Figure 2** compares the ability of siRNA (U1 and U3) bearing molecule 1 modifications (or unmodified) to silence a given target at varying concentrations. The Y-axis represents the level of expression relative to controls (unuansfeaed cells). The X-axis represents the concentration of siRNA during the transfection procedures. Black bars represent unmodified sequences. White bars represent modified sequences.

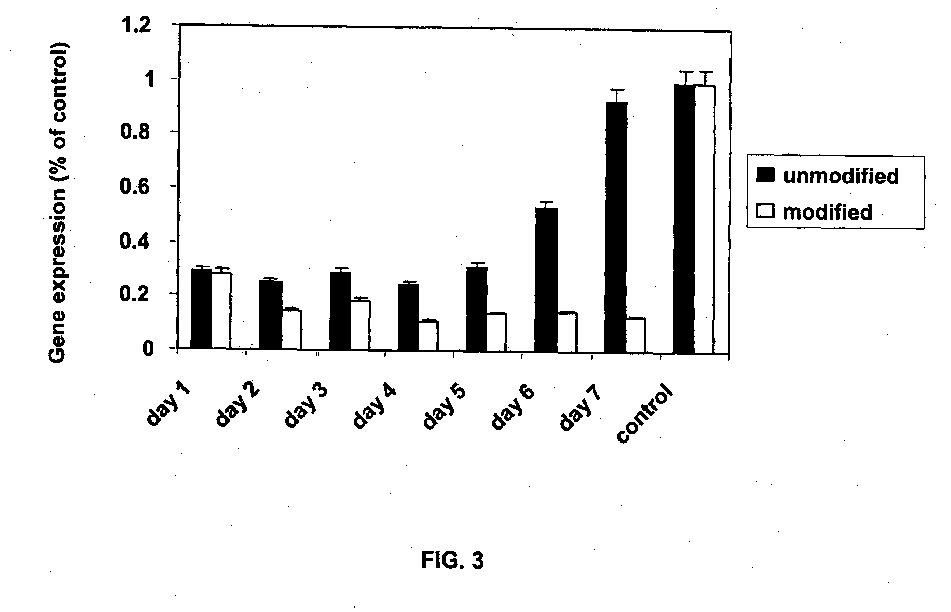

**Figure 3** assesses the level of silencing induced by siRNA carrying molecule 1 modifications (or unmodified) over the course of seven days. The Y-axis represents the level of gene expression relative to controls (untransfected cells). The X-axis represents the number of days after transfection. Black bars represent unmodified sequences. White bars represent modified sequences.

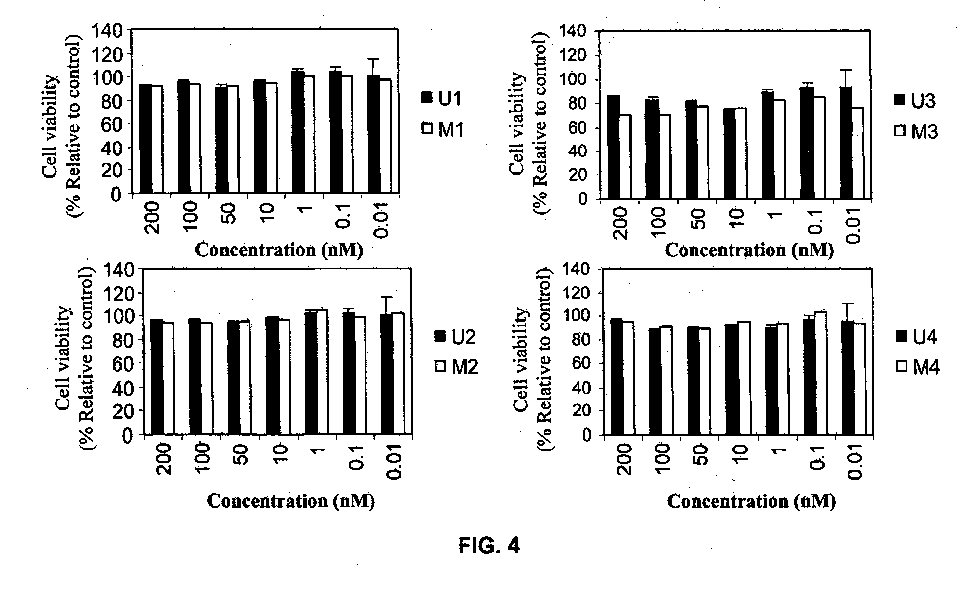

**Figure 4** compares the level of cell death induced by four separate modified (according to molecule 1 modifications) and unmodified siRNA transfected into cells at varying concentrations. The Y-axis represents the relative level of cell viability (as compared to untransfected cells). The X-axis represents the concentration of the siRNA during the transfection procedures. Cultures were tested 24-48 hours after transfection using an Alamar Blue assay. Black bars represent unmodified sequences. White bars represent modified sequences.

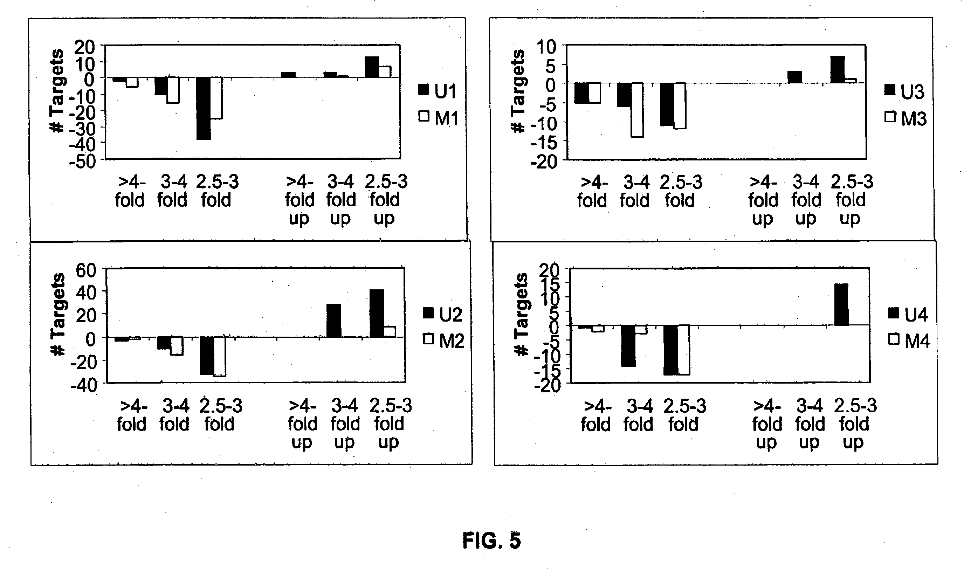

**Figure 5** compares the level of off-targeting by four separate human cyclophilin B siRNA in modified (according to molecule 1 modifications) and unmodified forms. Data are divided into six separate groups: number of targets that show decreased expression by more than four fold (4x), number of targets that show decreased expression by three to four fold (3-4x), number of targets that show decreased expression by 2.5-3 fold (2.5-3x), number of targets that show increased expression by greater than four fold (>4x), number of targets that show increased expression by three to four fold (3-4x), and number of targets that show increased expression by 2.5-3 fold (2.5-3x). Black bars represent unmodified sequences. White bars represent modified sequences.

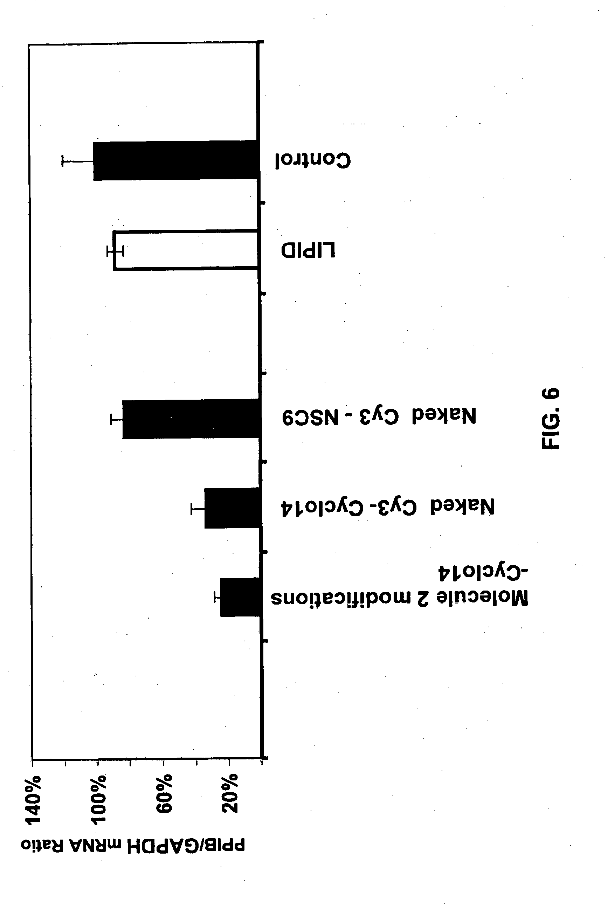

**Figure 6** is a histogram comparing the gene silencing ability of Cyclo 14 siRNA containing 1) molecule 2 modifications, 2) naked molecules, and 3) naked molecules with Cy3. The Y-axis depicts the level of human cyclophilin B expression relative to a control gene (GAPDH). "Lipid" represents control cells treated with the transfection reagent Lipofectamine 2000. "Control" represents cells that are untreated. NS9 is non-specific sequence #9 (5'- auuguaugcg aucgcagacu u-3', SEQ. ID NO. 55; 5' end of antisense phosphorylated; 3'uu overhang on both strands).

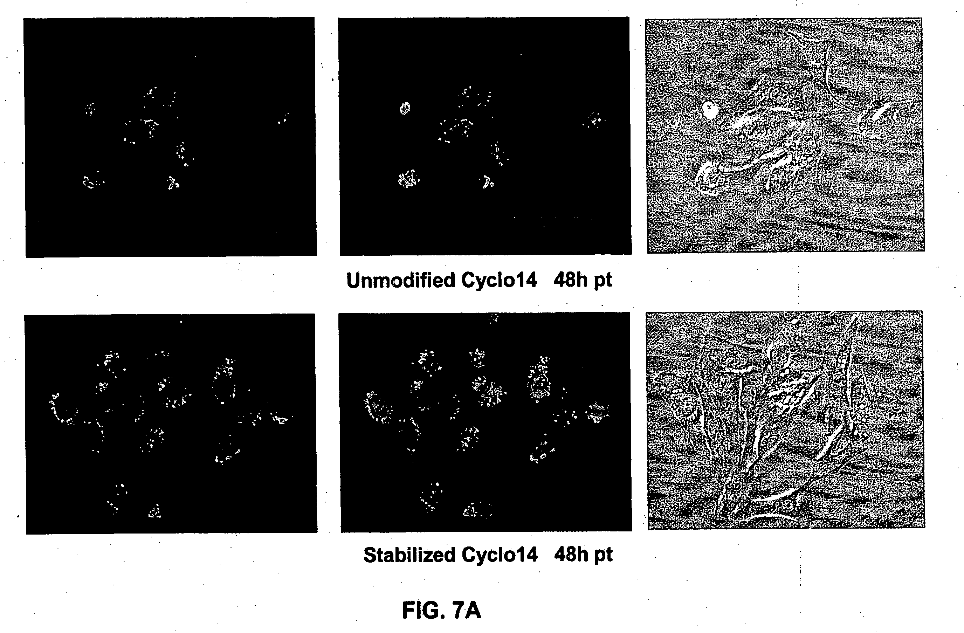

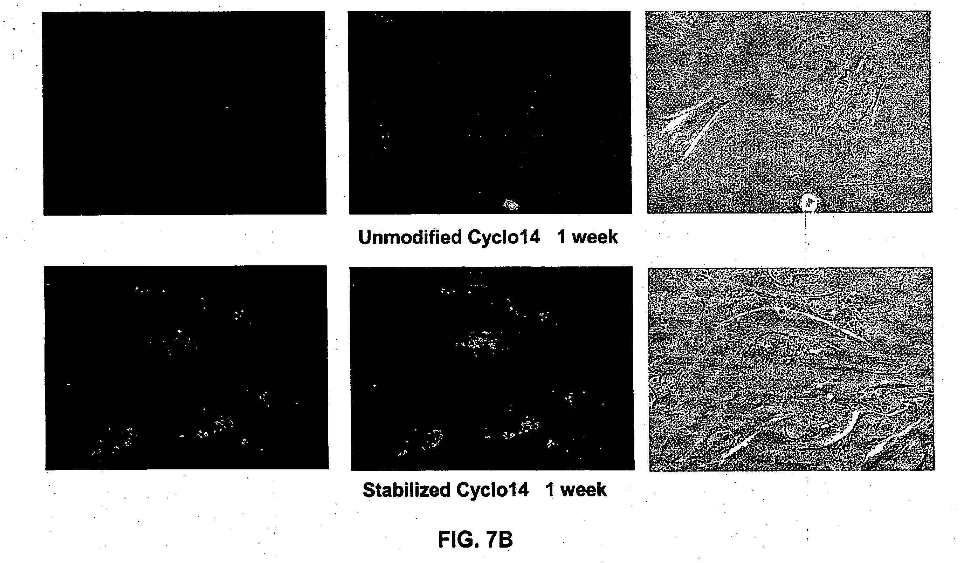

**Figure 7** shows the cellular distribution of cyclo 14 siRNA(Cy3 modified or modified with molecule 2 modifications) at 48 hours and 7 days. Slides on the left (top and bottom) show the staining pattern due to the Cy3 conjugate associated with each molecule. Slides in the middle (top and bottom) show equivalent fields and the position of the siRNA staining pattern is placed in perspective with the position of the nucleus (blue). Slides on the right (top and bottom, equivalent fields) are phase contrast images. Nuclear position was determined by staining cells with Hoechst 33342 stain.

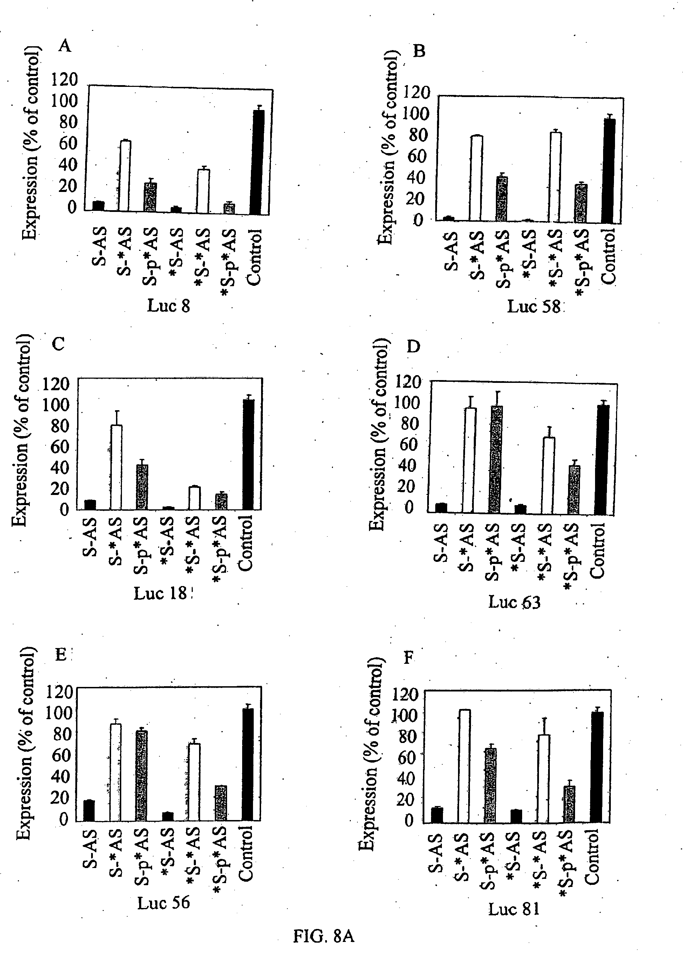

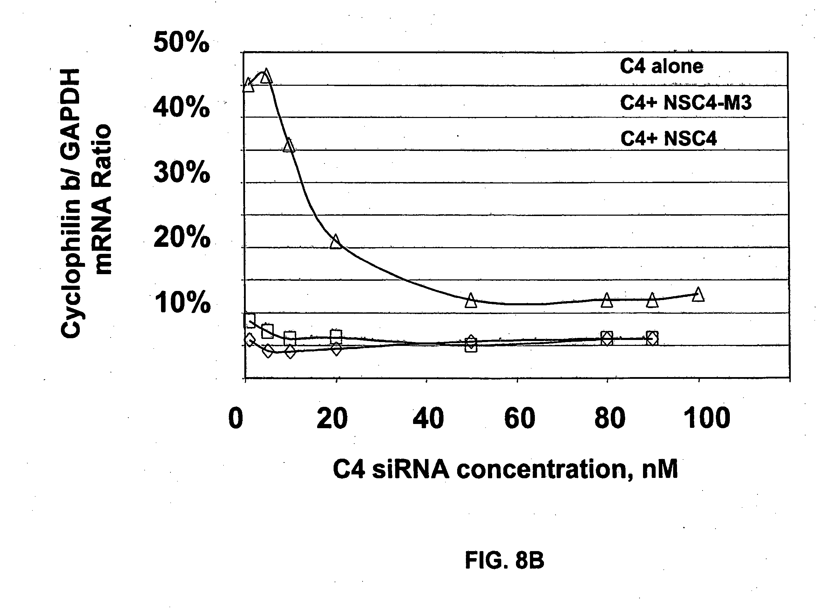

**Figure 8a** represents the silencing ability of 6 different siRNA targeting luciferase, when those molecules are unmodified or contain various modifications to the sense and/or antisense strand. **Figure 8b** is a competitive assay that shows how molecule 3 modifications on 17 bp duplexes alter the ability of an siRNA (19bp) to compete with unmodified cyclo 4 siRNA.

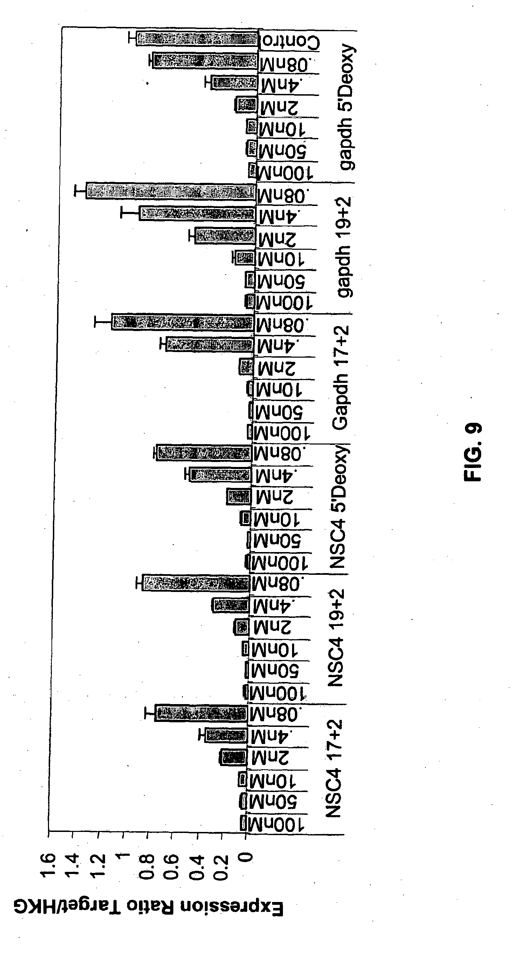

**Figure 9** is a competition assay comparing how NSC4 and GAPDH duplexes (17 bp and 19 bp, both modified with molecule 3 modifications and having 2 nucleotide overhangs on the 3' end of the molecules) or NSC4 and GAPDH duplexes (19 bp carrying a 5' deoxy modification on both the sense and antisense strands and having 2 nucleotide overhangs on the 3' end of the molecules) compete with unmodified cyclo 1 siRNA.

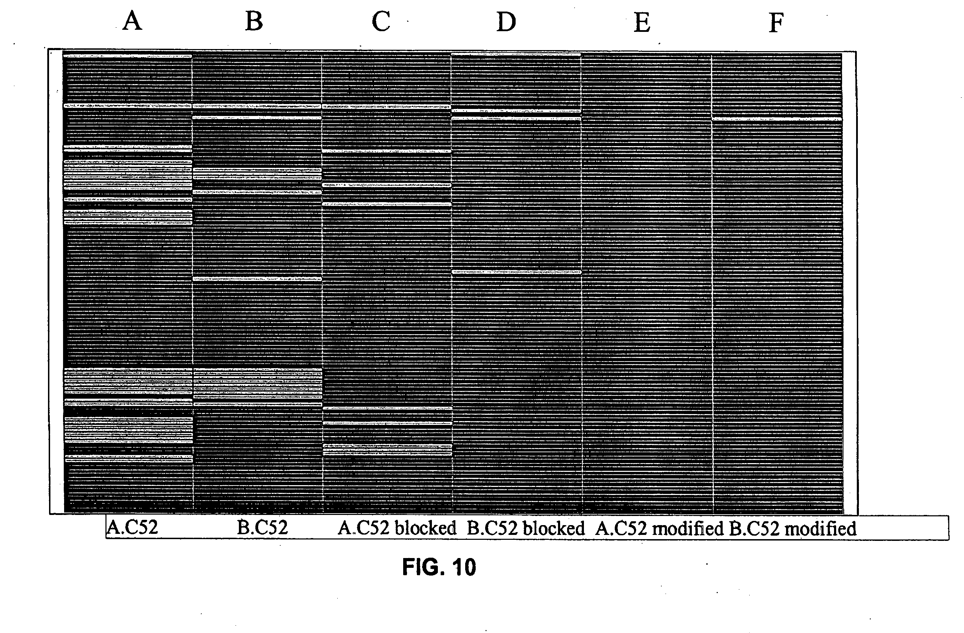

**Figure 10** shows a heat map comparing the off target signatures generated by (**A**) and (**B**) cyclo 52 (c52) siRNA, (**C**) and (**D**) cycle 52 siRNA containing 2'-O-methyl modifications on positions 1 and 2 of the sense and antisense strand, (**E**) and (**F**) cyclo 52 siRNA that have been reduced in length by 2 nucleotides (*i.e*., 17 mers) and contain 2'-O-methyl modifications on positions 1 and 2 of the sense and antisense strand.

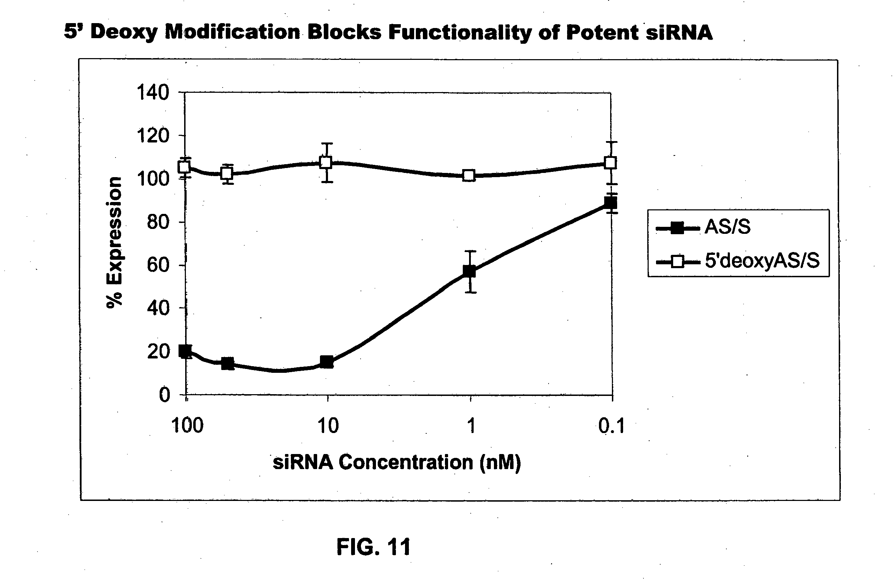

**Figure 11** shows the performance of GAPDH4 siRNA that are (1) unmodified, or (2) have 5'-deoxy modifications on the termini of the sense and antisense strands.

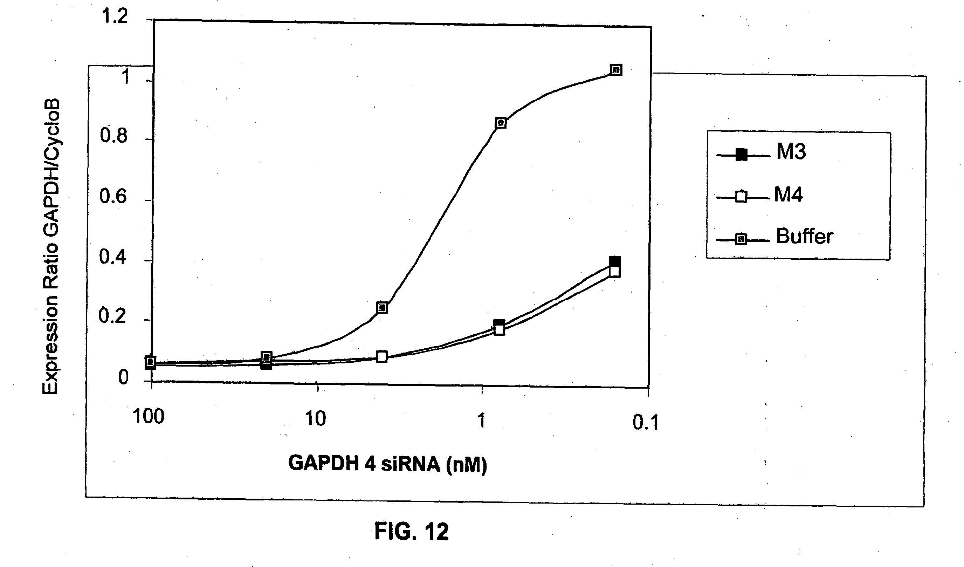

**Figure 12** shows a comparison in the ability of siRNA carrying molecule 3 modifications (M3) or molecule 4 modifications (M4), to compete with unmodified GAPDH4. "Buffer" represents reactions where no competitive molecule is present

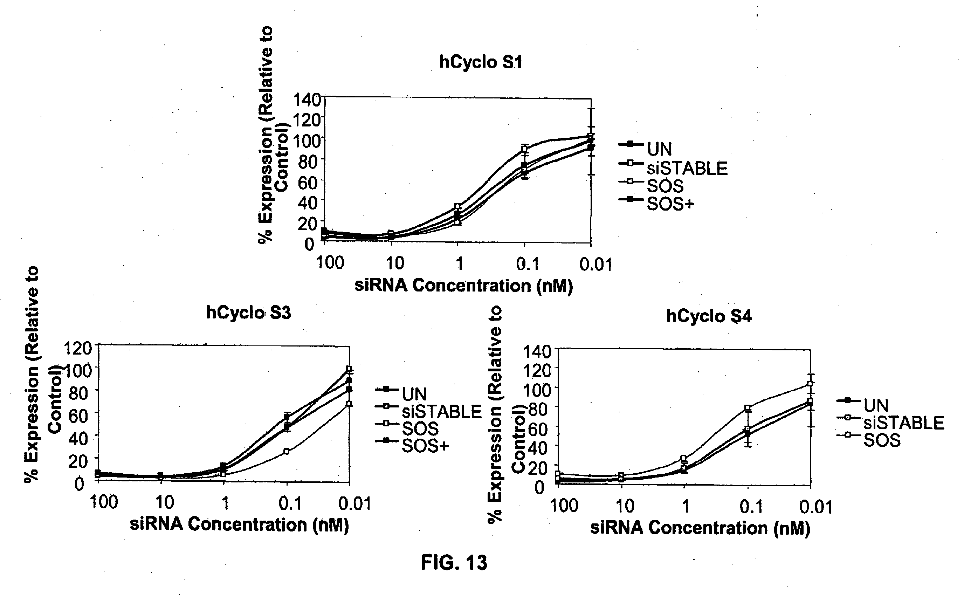

**Figure 13** shows the performance of cyclo 1, 3, and 4 in unmodified and modified forms at different concentrations.

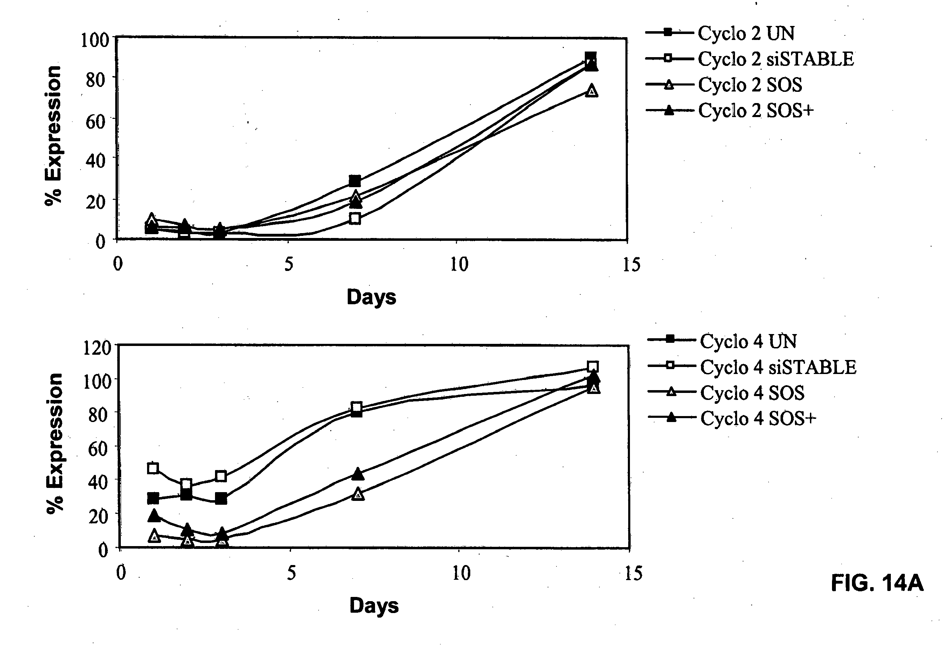

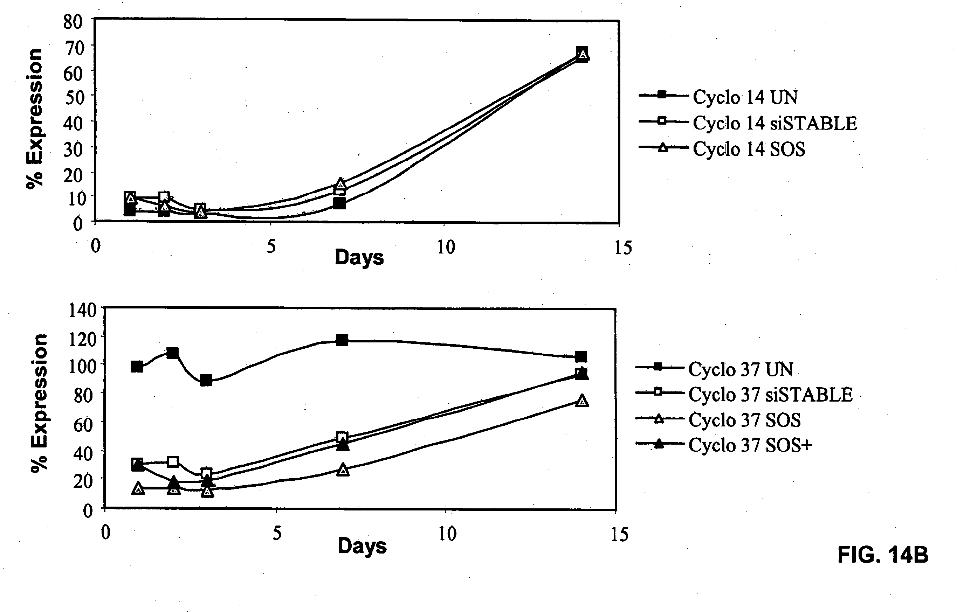

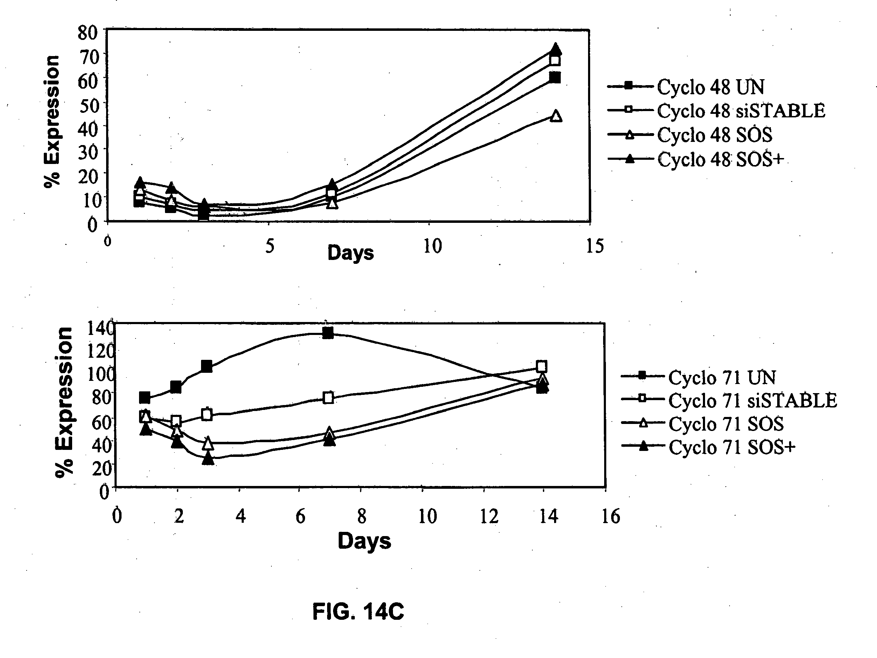

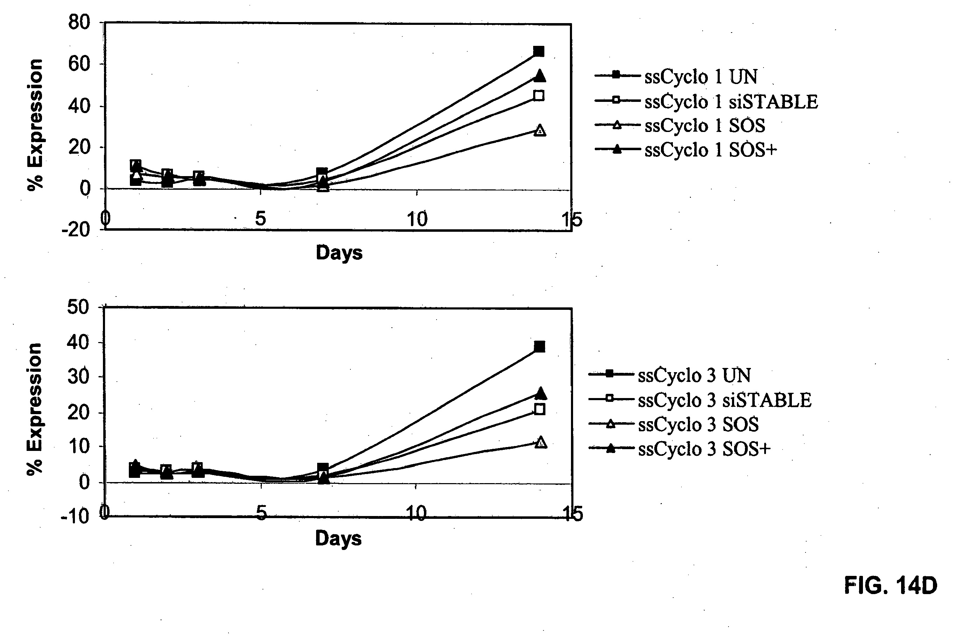

**Figure 14 a-d** shows a comparison of the longevity of silencing induced by unmodified and modified siRNA at a constant concentration.

### DETAILED DESCRIPTION

### Definitions

Unless stated otherwise, or implicit from context, the following terms and phrases include the meanings provided below. Unless explicitly stated otherwise, or apparent from context, the terms and phrases below do not exclude the meaning that the term or phrase has acquired in the art to which it pertains. The definitions are provided to aid in describing particular embodiments, and are not intended to limit the claimed invention, because the scope of the invention is limited only by the claims:

### Alkyl

The term "alkyl" refers to a hydrocarbyl moiety that can be saturated or unsaturated, and substituted or unsubstituted. It may comprise moieties that are linear, branched, cyclic and/or heterocyclic, and contain functional groups such as ethers, ketones, aldehydes, carboxylates, *etc*.

Exemplary alkyl groups include but are not limited to substituted and unsubstituted groups of methyl, ethyl, propyl, butyl, pentyl, hexyl, heptyl, octyl, nonyl, decyl, undecyl, dodecyl, tridecyl, tetradecyl, pentadecyl, hexadecyl, heptadecyl, octadecyl, nonadecyl, eicosyl and alkyl groups of higher number of carbons, as well as 2-methylpropyl, 2-methyl-4-ethylbutyl, 2,4-diethylpropyl, 3-propylbutyl, 2,8-dibutyldecyl, 6,6-dimethyloctyl, 6-propyl-6-butyloctyl, 2-methylbutyl, 2-methylpentyl, 3-methylpentyl, and 2-ethylhexyl. The term alkyl also encompasses alkenyl groups, such as vinyl, allyl, aralkyl and alkynyl groups.

Substitutions within an alkyl group can include any atom or group that can be tolerated in the alkyl moiety, including but not limited to halogens, sulfurs, thiols, thioethers, thioesters, amines (primary, secondary, or tertiary), amides, ethers, esters, alcohols and oxygen. The alkyl groups can by way of example also comprise modifications such as azo groups, keto groups, aldehyde groups, carboxyl groups, nitro, nitroso or nitrile groups, heterocycles such as imidazole, hydrazino or hydroxylamino groups, isocyanate or cyanate groups, and sulfur containing groups such as sulfoxide, sulfone, sulfide, and disulfide.

Further, alkyl groups may also contain hetero substitutions, which are substitutions of carbon atoms, by for example, nitragen, oxygen or sulfur. Heterocyclic substitutions refer to alkyl rings having one or more heteroatoms. Examples of heterocyclic moieties include but are not limited to morpholino, imidazole, and pyrrolidino. Preferably, the alkyl groups are not substituted. A preferred alkyl group is a methyl group, -CH₃.

### 2'-O-Alkyl Modified Nucleotide

The phrase "2'-O-alkyl modified nucleotide" refers to a nucleotide unit having a sugar moiety, for example a ribosyl moiety that is modified at the 2' position such that an oxygen atom is attached both to the carbon atom located at the 2' position of the sugar and to an alkyl group. A "2'-O-alkyl modified nucleotide" is modified at this position such that an oxygen atom is attached both to the carbon atom located at the 2' position of the sugar and to an alkyl group, *e.g*., 2'-O-methyl, 2'-O-ethyl, 2'-O-propyl, 2'-O-isopropyl, 2'-O-butyl, 2-O-isobutyl, 2'-O-ethyl-O-methyl (-OCH₂CH₂OCH₃), and 2'-O-ethyl-OH (-OCH₂CH₂OH). In various embodiments, the alkyl moiety consists essentially of carbons and hydrogens. A particularly preferred embodiment is one wherein the alkyl moiety is a methyl moiety.

### Antisense Strand

The phrase "antisense strand" as used herein, refers to a polynucleotide that is substantially or 100% complementary to a target nucleic acid of interest. An antisense strand may comprise a polynucleotide that is RNA, DNA or chimeric RNA/DNA. For example, an antisense strand may be complementary, in whole or in part, to a molecule of messenger RNA, an RNA sequence that is not mRNA (*e.g*., tRNA, rRNA and hnRNA) or a sequence of DNA that is either coding or non-coding. The phrase "antisense strand" includes the antisense region of both polynucleotides that are formed from two separate strands, as well as unimolecular polynucleotides that are capable of forming hairpin structures.

### 2' Carbon Modification

The phrase "2' carbon modification" refers to a nucleotide unit having a sugar moiety, for example a deoxyribosyl moiety that is modified at the 2' position. A "2' carbon sense modification" refers to a modification at the 2' carbon position of a nucleotide on the sense strand or within a sense region of polynucleotide. A "2' carbon antisense modification" refers to a modification at the 2' carbon position of a nucleotide on the antisense strand or within an antisense region of polynucleotide.

### Complementary

The term "complementary" refers to the ability of polynucleotides to form base pairs with one another. Base pairs are typically formed by hydrogen bonds between nucleotide units in antiparallel polynucleotide strands. Complementary polynucleotide strands can base pair in the Watson-Crick manner (*e.g*., a to t, a to u, c to g), or in any other manner that allows for the formation of stable duplexes.

Perfect complementarity or 100% complementarity refers to the situation in which each nucleotide unit of one polynucleotide strand can hydrogen bond with each nucleotide unit of a second polynucleotide strand. Less than perfect complementarity refers to the situation in which some, but not all, nucleotide units of two strands can hydrogen bond with each other. For example, for two 20-mers, if only two base pairs on each strand can hydrogen bond with each other, the polynucleotide strands exhibit 10% complementarity. In the same example, if 18 base pairs on each strand can hydrogen bond with each other, the polynucleotide strands exhibit 90% complementarity.

"Substantial complementarity" refers to polynucleotide strands exhibiting 90% or greater complementarity, excluding regions of the polynucleotide strands, such as overhangs, that are selected so as to be noncomplementary. ("Substantial similarity" refers to polynucleotide strands exhibiting 90% or greater similarity, excluding regions of the polynucleotide strands, such as overhangs, that are selected so as not to be similar.) Thus, for example, two polynucleotides of 29 nucleotide units each, wherein each comprises a di-dT (or di-dU) at the 3' terminus such that the duplex region spans 27 bases, and wherein 26 of the 27 bases of the duplex region on each strand are complementary, are substantially complementary since they are 96.3% complementary when excluding the overhangs.

### Downstream

A first region of a polynucleotide is considered to be downstream of a second region, if the 5' most portion of the first region is the closest portion of the first region to the 3' end of the second region.

### Duplex

The term "duplex" includes a region of complementarity between two regions of a single contiguous polynucleotide or between two regions of two or more polynucleotides that comprise separate strands. Thus, a contiguous polynucleotide that comprises a region or regions of self-complementarity comprises a "duplex.". A single contiguous polynucleotide includes a polynucleotide comprising a noncomplementary region, for example, a loop region; such a loop region can comprise a non-nucleotide element.

The phrase "duplex region" includes the region in two complementary or partially or substantially complementary polynucleotides that form base pairs with one another, either by Watson-Crick base pairing or any other manner that allows for a stabilized duplex between polynucleotide strands that are complementary or substantially complementary. For example, a polynucleotide strand having 21 nucleotide units can base pair with another polynucleotide of 21 nucleotide units, yet only 19 contiguous bases on each strand are complementary or substantially complementary, such that the "duplex region" has 19 base pairs. The remaining bases may, for example, exist as 5' and 3' overhangs. Further, within the duplex region, 100% complementarity is not required; substantial complementarity is allowable within a duplex region. Substantial complementarity refers to 90% or greater complementarity. For example, a mismatch in a duplex region consisting of 19 base pairs results in 94.7% complementarity, rendering the duplex region at least substantially complementary.

### Exaequo Agent

The phrase "exaequo agent" refers to a nucleic acid that is, from the perspective of its participation in the RNAi pathway, orability to compete with other nucleic acids for the ability to participate in the RNAi pathway, inert or semi-inert. Molecules can be used as an exaequo agent whereby said agents are used to equalize or to make level the total amount of nucleic acid in a solution.

### First 5' Terminal Antisense Nucleotide

The phrase "first 5' terminal antisense nucleotide" refers to the nucleotide of the antisense strand that is located at the 5' most position of that strand with respect to the bases of the antisense strand that have corresponding complementary bases on the sense strand. Thus, in a double stranded polynucleotide that is made of two separate strands, it refers to the 5' most base other than bases that are part of any 5' overhang on the antisense strand. When the first 5' terminal antisense nucleotide is part of a hairpin molecule, the term "terminal" refers to the 5' most relative position within the antisense region and thus is the 5' most nucleotide of the antisense region.

### First 5' Terminal Sense Nucleotide

The phrase "first 5' terminal sense nucleotide" is defined' in reference to the antisense nucleotide. In molecules comprising two separate strands, it refers to the nucleotide of the sense strand that is located at the 5' most position of that strand with respect to the bases of the sense strand that have corresponding complementary bases on the antisense strand. Thus, in a double stranded polynucleotide that is made of two separate strands, it is the 5' most base other than bases that are part of any 5' overhang on the sense strand. When the first 5' terminal sense nucleotide is part of a unimolecular polynucleotide that is capable of forming a hairpin molecule, the term "terminal" refers to the relative position within the sense region as measured by the distance from the base complementary to the first 5' terminal antisense nucleotide.

### Functional

siRNAs may be divided into five (5) groups (non-functional, semi-functional, functional, highly functional, and hyper-functional) based on the level or degree of silencing that they induce in cultured cell lines. As used herein, these definitions are based on a set of conditions where the siRNA is transfected into said cell line at a concentration of 100 nM and the level of silencing is tested at a time of roughly 24 hours after transfection, and not exceeding 72 hours after transfection. In this context, "non-functional siRNA" are defined as those siRNA that induce less than 50% (<50%) target silencing. "Semi-functional siRNA" induce 50-79% target silencing. "Functional siRNA" are molecules that induce 80-95% gene silencing. "Highly-functional siRNA" are molecules that induce greater than 95% gene silencing. "Hyperfunctional siRNA" are a special class of molecules. For purposes of this document, hyperfunctional siRNA are defined as those molecules that: (1) induce greater than 95% silencing of a specific target when they are transfected at subnanomolar concentrations (*i.e*., less than one nanomolar); and/or (2) induce functional (or better) levels of silencing for greater than 96 hours. These relative functionalities (though not intended to be absolutes) may be used to compare siRNAs to a particular target for applications such as functional genomics, target identification and therapeutics.

### Halogen

The term "halogen" refers to an atom of either fluorine, chlorine, bromine, iodine or astatine. The phrase "2' halogen modified nucleotide" refers to a nucleotide unit having a sugar moiety that is modified with a halogen at the 2' position, *i.e.*, attached directly to the 2' carbon position of the ribose or deoxyribose ring.

### 2' Halogen Modified Pyrimidine

The phrase "2' halogen modified pyrimidine" refers to a pyrimidine (*e.g*. cytosine or uracil) that contains a halogen group attached to the 2' carbon of the sugar of a nucleotide.

### Nucleotide

The term "nucleotide" includes a ribonucleotide or a deoxynbonucleotide or modified form thereof, as well as an analog thereof. Nucleotides include species that comprise purines, *e.g*., adenine, hypoxanthine, guanine, and their derivatives and analogs, as well as pyrimidines, e*.g.*, cytosine, uracil, thymine, and their derivatives and analogs.

Nucleotide analogs include nucleotides having modifications in the chemical structure of the base, sugar and/or phosphate, including, but not limited to, 5-position pyrimidine modifications, 8-position purine modifications, modifications at cytosine exocyclic amines, and substitution of 5-bromo-uracil; and 2'-position sugar modifications, including but not limited to, sugar-modified ribonucleotides in which the 2'-OH is replaced by a group such as an H, OR, R, halo, SH, SR, NH₂, NHR, NR₂, or CN, wherein R is an alkyl moiety as defined herein. Nucleotide analogs are also meant to include nucleotides with bases such as inosine, queuosine, xanthine, sugars such as 2'-methyl ribose, non-natural phosphodiester linkages such as methylphosphonates, phospharothioates and peptides.

Modified bases include nucleotide bases such as, for example, adenine, guanine, cytosine, thymine, uracil, xanthine, inosine, and queuosine that have been modified by the replacement or addition of one or more atoms or groups. Some examples of types of modifications that can comprise nucleotides that are modified with respect to the base moieties, include but are not limited to, alkylated, halogenated, thiolated, aminated, amidated, or acetylated bases, in various combinations. More specific modified bases include, for example, 5-propynyluridine, 5-propynylcytidine, 6-methyladenine, 6-methylguanine, N,N,-dimethyladenine, 2-propyladenine, 2-propylguanine, 2-aminoadenine, 1-methylinosine, 3-methyluridine, 5-methylcytidine, 5-methyluridine and other nucleotides having a modification at the 5 position, 5-(2-amino)propyl uridine, 5-halocytidine, 5-halouridine, 4-acetylcytidine, 1-methyladenosine, 2-methyladenosine, 3-methylcytidine, 6-methyluridine, 2-methylguanosine, 7-methylguanosine, 2,2-dimethylguanosine, 5-methylaminoethyluridine, 5-methyloxyuridine, deazanucleotides such as 7-deaza-adenosine, 6-azouridine, 6-azocytidine, 6-azothymidine, 5-methyl-2-thiouridine, other thio bases such as 2-thiouridine and 4-thiouridine and 2-thiocytidine, dihydrouridine, pseudouridine, queuosine, archaeosine, naphthyl and substituted naphthyl groups, any O- and N-alkylated purines and pyrimidines such as N6-methyladenosine, 5-methylcarbonylmethyluridine, uridine 5-oxyacetic acid, pyridine-4-one, pyridine-2-one, phenyl and modified phenyl groups such as aminophenol or 2,4,6-trimethoxy benzene, modified cytosines that act as G-clamp nucleotides, 8-substituted adenines and guanines, 5-substituted uracils and thymines, azapyrimidines, carboxyhydroxyalkyl nucleotides, carboxyalkylaminoalkyl nucleotides, and alkylcarbonylalkylated nucleotides. Modified nucleotides also include those nucleotides that are modified with respect to the sugar moiety, as well as nucleotides having sugars or analogs thereof that are not ribosyl. For example, the sugar moieties may be, or be based on, mannoses, arabinoses, glucopyranoses, galactopyranoses, 4'-thioribose, and other sugars, heterocycles, or carbocycles. The term nucleotide is also meant to include what are known in the art as universal bases. By way of example, universal bases include but are not limited to 3-nitropyrrole, 5-nitroindole, or nebularine.

Further, the term nucleotide also includes those species that have a detectable label, such as for example a radioactive or fluorescent moiety, or mass label attached to the nucleotide. Preferably, nucleotides are selected from adenine, guanine, uracil, thymidine, and cytosine.

### Nucleotide Unit

The phrase "nucleotide unit" refers to a single nucleotide residue and comprises a modified or unmodified nitrogenous base, a modified or unmodified sugar, and a modified or unmodified moiety that allows for linking of two nucleotides together or a conjugate that precludes further linkage.

### Off-Target

The term "off target" and the phrase "off-target effects" refer to any instance in which an siRNA or shRNA directed against a given target causes an unintended affect by interacting either directly or indirectly with another mRNA sequence, a DNA sequence or a cellular protein or other moiety. For example, an "off-target effect" may occur when there is a simultaneous degradation of other transcripts due to partial homology or complementarity between that other transcript and the sense and/or antisense strand of the siRNA or shRNA

### Overhang

The term "overhang" refers to terminal non-base pairing nucleotide(s) resulting from one strand extending beyond the terminus of the complementary strand to which the first strand forms a doubled stranded polynucleotide. One or both of two polynucleotides that are capable of forming a duplex through hydrogen bonding of base pairs may have a 5' and/or 3' end that extends beyond the 3' and/or 5' end of complementarity shared by the two polynucleotides. The single-stranded region extending beyond the 3' and/or 5' end of the duplex is referred to as an overhang. Overhangs are not included, or counted, when calculating complementarity, for example, in determining whether two strands are substantially complementary.

### Pharmaceutically Acceptable Carrier

The phrase "pharmaceutically acceptable carrier" includes compositions that facilitate the introduction of dsRNA, dsDNA, or dsRNA/DNA hybrids into a cell and includes but is not limited to solvents or dispersants, coatings, anti-infective agents, isotonic agents, and agents that mediate absorption time or release of the inventive polynucleotides and double stranded polynucleotides. The phrase "pharmaceutically acceptable" includes approval by a regulatory agency of a government, for example, the U.S. federal government, a non-U.S. government, or a U.S. state government, or inclusion in a listing in the U.S. Pharmacopeia or any other generally recognized pharmacopeia for use in animals, including in humans.

### Polynucleotide

The term "polynucleotide" refers to polymers of nucleotides, and includes but is not limited to DNA, RNA, DNA/RNA hybrids including polynucleotide chains of regularly and irregularly alternating deoxyribosyl moieties and ribosyl moieties (*i.e.,* wherein alternate nucleotide units have an -OH, then an -H, then an -OH, then an - H, and so on at the 2' position of a sugar moiety), and modifications of these kinds of polynucleotides wherein the attachment of various entities or moieties to the nucleotide units at any position are included. Unless otherwise specified, or clear from context, the term "polynucleotide" includes both single stranded and double stranded molecules.

### Polyribonacleotide

The term "polyribonucleotide" refers to a polynucleotide comprising two or more modified or unmodified ribonucleotides and/or their analogs.

### Ribonucleotide and Ribonucleic Acid

The term "ribonucleotide" and the phrase "ribonucleic acid" (RNA), refer to a modified or unmodified nucleotide or polynucleotide comprising at least one ribonucleotide unit. A ribonucleotide unit comprises an oxygen attached to the 2' position of a ribosyl moiety having a nitrogenous base attached in N-glycosidic linkage at the 1' position of a ribosyl moiety, and a moiety that either allows for linkage to another nucleotide or precludes linkage.

### RNA Interference and RNAi

The phrase "RNA interference" and the term "RNAi" are synonymous and refer to the process by which a polynucleotide or double stranded polynucleotide comprising at least one ribonucleotide unit exerts an effect on a biological process. The process includes but is not limited to gene silencing by degrading mRNA, interactions with tRNA, rRNA, hnRNA, cDNA and genomic DNA, as well as methylation of DNA with ancillary proteins.

### Second 5' Terminal Antisense Nucleotide

The phrase "second 5' terminal antisense nucleotide" refers to the nucleotide that is immediately adjacent to the first 5' terminal antisense nucleotide and attached to the 3' position of the first 5' terminal antisense nucleotide. Thus, it is the second most 5' nucleotide of the antisense strand within the set of nucleotide for which there are corresponding sense nucleotides.

### Second 5' Terminal Sense Nucleotide

The phrase "second 5' terminal sense nucleotide" refers to the nucleotide that is immediately adjacent to the first 5' terminal sense nucleotide and attached to the 3' position of the first 5' terminal sense nucleotide. Thus, it is the second most 5' nucleotide of the sense strand within the set of nucleotide for which there are corresponding antisense nucleotides.

### Sense Strand

The phrase "sense strand" refers to a polynucleotide that has the same nucleotide sequence, in whole or in part, as a target nucleic acid such as a messenger RNA or a sequence of DNA. The phrase "sense strand" includes the antisense region of both polynucleotide that are formed from two separate strands, as well as unimolecular polynucleotides that are capable of forming hairpin structures.

### siRNA or Short Interfering RNA

The term "siRNA" and the phrase "short interfering RNA" refer to a double stranded nucleic acid that is capable of performing RNAi and that is between 18 and 30 base pairs in length. Additionally, the term siRNA and the phrase "short interfering RNA" include nucleic acids that also contain moieties other than ribonucleotide moieties, including, but not limited to, modified nucleotides, modified internucleotide linkages, non-nucleotides, deoxynucleotides and analogs of the aforementioned nucleotides.

siRNAs can be duplexes, and can also comprise short hairpin RNAs, RNAs with loops as long as, for example, 4 to 23 or more nucleotides, RNAs with stem loop bulges, micro-RNAs, and short temporal RNAs. RNAs having loops or hairpin loops can include structures where the loops are connected to the stem by linkers such as flexible linkers. Flexible linkers can comprise a wide variety of chemical structures, as long as they are of sufficient length and materials to enable effective intramolecular hybridization of the stem elements. Typically, the length to be spanned is at least about 10-24 atoms.

When the siRNAs are hairpins, the sense strand and antisense strand are on a single polynucleotide, where the nucleotide units are contiguous, or that comprises a non-nucleotide region, such as, for example, a non-nucleotide loop.

### Stabilized

The term "stabilized" refers to the ability of the dsRNAs to resist degradation while maintaining functionality and can be measured in terms of its half-life in the presence of, for example, biological materials such as serum. The half-life of an siRNA in, for example, serum refers to the time taken for the 50% of the siRNA to be degraded.

### Substantial Complementarity

Substantial complementarity refers to polynucleotide strands exhibiting 90% or greater complementarity. Polynucleotide strands that are "capable of forming a duplex," such as, for example, sense and antisense strands, are at least substantially complementary.

### Trackabillty

The term "trackability" refers to the ability to follow the movement or localization of a molecule after said molecule has been *e.g.,* introduced into a cell. Molecules that are "trackable" are useful in monitoring the success or failure of *e,g.,* a cellular transfection procedure.

### Transfection

The term "transfection" includes a process by which agents are introduced into a cell. The list of agents that can be transfected is large and includes, but is not limited to, siRNA, sense and/or anti-sense sequences, DNA encoding one or more genes and organized into an expression plasmid, proteins, protein fragments, and more. Many methods for transfecting agents into a cell are known in the art, including, but not limited to, electroporation, calcium phosphate-based transfections, DEAE-dextran-based transfections, lipid-based transfections, molecular conjugate-based transfections (*e.g.,* polylysine-DNA conjugates), microinjection and others. Transfection may be forward or reverse. Reverse transfection is described in U.S. Provisional Patent Application Serial No. 60/630,320, filed November 22, 2004.

### PREFERRED EMBODIMENTS

The present invention will now be described in connection with preferred embodiments. These embodiments are presented in order to aid in an understanding of the present invention and are not intended, and should not be construed, to limit the invention in any way. All alternatives, modifications and equivalents that may become apparent to those of ordinary skill upon reading this disclosure are included within the spirit and scope of the present invention.

This disclosure is not a primer on compositions and methods for performing RNA interference. Basic concepts known to those of ordinary skill in this art have not been set forth in detail.

Throughout the disclosure, where a range of values is provided, it is understood that each intervening value, to the tenth of the unit of the lower limit unless the context clearly dictates otherwise, between the upper and lower limit of that range, and any other stated or intervening value in that stated range, is encompassed within the invention. The upper and lower limits of these smaller ranges may independently be included in the smaller ranges, and are also encompassed within the invention, subject to any specifically excluded limit in the stated range.

The first embodiment related to the present invention is an siRNA comprising a sense strand and an antisense strand. The sense strand has a first 5' terminal sense nucleotide and a second 5' terminal sense nucleotide, both of which have 2'carbon modifications, preferably 2'-O-akyl modifications. In some embodiments, the siRNA also comprises a label, which if present is preferably located on the first 5' terminal sense nucleotide. Further, at least one and preferably each of the pyrimidines (*e.g.,* cytosine or uracil) on the sense strand has a 2' carbon modifications, preferably 2'-O-alkyl modifications. These modifications are in addition to any modification of the first or second 5' terminal sense nucleotide, which may, depending on the sequence of the siRNA, be a pyrimidine. The molecules of the first embodiment may be used to silence a target and/or as a control. These molecules may further comprise a label, such as a fluorescent label and/or a third 5' terminal sense nucleotide that comprises a third 2'-O-alkyl sense modification. Optionally, the 5' end of the sense strand contains a 5' carbon blocking group that prevents phosphorylation of the first terminal sense nucleotide.

The antisense strand of this embodiment comprises at least one 2'-halogen modified pyrimidine, preferably, a fluoro modified pyrimidine, and a first 5' terminal antisense nucleotide that has been phosphorylated. Preferably all of the pyrimidines on the antisense strand have 2'-fluoro modifications.

Preferably, the double stranded polynucleotide comprises from 18 - 30 base pairs, more preferably from 19 - 25 base pairs, and most preferably from 19 - 23 base pairs, exclusive of overhangs or loop or stem structures when present in unimolecular polynucleotides that are capable of forming hairpins. Preferably, the sense strand and antisense strand are—exclusive of overhangs, loop or stem structures—at least 79% complementary, more preferably at least 90% complementary over the range of base pairs, and most preferably 100% complementary over this range. Similarly preferably, the antisense region is preferably at least 79%, more preferably at least 90%, and most preferably at least 100% complementary to the target region. Preferably, the polynucleotide is RNA, wherein at least a plurality of the nucleotides are ribonucleotides.

The double stranded polynucleotide may also contain overhangs at either the 5' or 3' end of either the sense strand or the antisense strand. However, in the case of polynucleotides comprising two separate strands preferably, if there are any overhangs, they are only on the 3' end of the sense strand and/or the antisense strand. Additionally, preferably any overhangs are six or fewer bases in length, more preferably two or fewer bases in length. Most preferably, there are either no overhangs, or overhangs of two bases on one or both of the sense strand and antisense strand at the 3' end. According to this embodiment it is preferable not to have overhangs on the 5' end of the antisense strand. Overhanging nucleotides are frequently removed by one or more intracellular enzymatic processes or events, which may leave an unphosphorylated 5'-nucleotide. Therefore, it is preferable not to have overhangs on the 5' end of the antisense strand.

Furthermore, the molecule may also contain alternative internucleotide linkages, such as stabilized linkages (*e.g.,* phosphorothioate linkages) between any or all of the nucleotides in the sense or antisense strand, or the overhangs of the sense or antisense strand. Preferably, the molecule contains a two nucleotide 3' overhang on the antisense strand, and phosphorothioate modifications exist between the two nucleotides of the overhang, as well as between the first nucleotide of the overhang and the adjacent, (upstream) nucleotide, which basepairs with the 5' most(1^{st}) nucleotide of the sense strand.

Furthermore, the molecule may also contain a single 2'-O-methyl modification on the second nucleotide (counting from the 5' end) of the antisense strand.

The phosphorylation of the first 5' terminal antisense nucleotide refers to the presence of one or more phosphate groups attached to the 5' carbon of the sugar moiety of the nucleotide. Preferably, there is only one phosphate group.

The 2'-O-alkyl modifications, regardless of on which bases they appear are preferably selected from the group consisting of 2'-O-methyl, 2'-O-ethyl, 2'-O-propyl, 2'-O-isopropyl, 2'-O-butyl, 2-O-isobutyl, 2'-O-ethyl-O-methyl (-CH₂CH₂OCH₃), and 2'-O-ethyl-OH (-OCH₂CH₂OH). Most preferably, the 2'-O-alkyl modification is a 2'-O-methyl moiety. Further, there is no requirement that the modification be the same on each of the first 5' terminal sense nucleotide and the second 5' terminal sense nucleotide. However, as a matter of practicality with respect to synthesizing the molecules of the present invention, it may be desirable to use the same modification throughout.

With respect to the 2'-O-alkyl pyrimidine modifications, at least one pyrimidine other than any pyrimidine that may exist within the first two 5' terminal sense nucleotides is modified with a 2'-O-alkyl group. Preferably, all pyrimidines on the sense strand within the duplex forming region are modified. Further, preferably, the 2'-O-alkyl modifications are 2'-O-methyl groups. As with the 2'-O-alkyl modifications, the same alkyl modification need not be used on each nucleotide that has a 2'-O-alkyl group. When overhangs, loops or stems are present, the pyrimidines of those regions may or may not contain 2'-O-alkyl groups. However, at least one of the 2'-O-alkyl groups is preferably in the sense region.

With respect to the 2' halogen modified nucleotides, the modification appears on at least one of the pyrimidines of the antisense strand and more preferably on all of the pyrimidines of the antisense strand. Further, preferably the halogen is fluorine. When overhangs, stems or loops are present, the pyrimidines of those regions may or may not contain halogen groups but preferably any stem or loop structure would not contain this modification, and the at least one halogen group is within the sense region. It should be noted that 2' halogen modifications, including 2' fluoro modifications might be used to increase the stability of the siRNA independent of the other modifications described herein. Thus, they may be used independently, as well as in connection with these other modifications in siRNA applications.

Other types of nucleotide modifications of the sense and/or antisense strands may be included if they do not greatly negate the benefits of the recited molecules, including stability and with respect to the first embodiment, functionality. For example, the use of pyrimidine modified nucleotides such a halogen, preferably fluorine, modified and additional 2'-O-alkyl modified pyrimidines provide a certain level of nuclease resistance.

In addition to chemical modifications, it is postulated that base pair mismatches or bulges can be added to the sense and/or antisense strands that alter the ability of these strands to participate in RISC-mediated association with targets that share less than 100% homology. Examples of such mismatches include (but are not limited to) purine-pyrimidine mismatches (*e.g.,* g-u, c-a) and purine-purine or pyrimidine-pyrimidine mismatches (*e.g.,* g-a, u-c). The introduction of these types of modification may be combined with the above-described modifications, and evaluated to determine whether they alter other attributes of functionality (*e.g.,* off-target effects) without detracting from the benefits of the recited molecules.

A preferred embodiment is an siRNA comprising: (a) a sense strand, wherein the first and second 5' terminal sense nucleotides each have a 2'-O-alkyl modification, and at least one pyrimidine nucleotide other than the first and second 5' terminal sense nucleotides also bears a 2'-O-alkyl modification; and (b) an antisense strand, wherein the antisense strand is phosphorylated at the first 5' nucleotide at the 5' carbon position and bears at least one 2' halogen modification, with the antisense strand having a 3' overhang of two nucleotides, wherein there is a modified internucleotide linkage between the last nucleotide of the duplex region of the antisense strand and the first overhang nucleotide, and a modified internucleotide linkage between the first overhang nucleotide and the second overhang nucleotide. In a particular embodiment, the 2'-O-alkyl modification is a 2'-O-methyl modification, all pyrimidine nucleotides of the sense strand comprise a 2'-O-methyl modification, the modified internucleotide linkages are each phosphorothioate linkages, and each pyrimidine nucleotide of the antisense strand comprises a 2'-fluorine modification; and the siRNA is blunt-ended at the 3' end of the sense strand and the 5' end of the antisense strand

A preferred embodiment is an siRNA comprising (a) a sense strand and an antisense strand, wherein the first and second 5' terminal sense nucleotides each have a 2'-O-alkyl modification, and at least one pyrimidine nucleotide other than the first and second 5' terminal sense nucleotides also bears a 2'-O-alkyl modification; and (b) an antisense strand or region, wherein the antisense strand is phosphorylated at the first 5' nucleotide at the 5' carbon position, has a 2'-O-alkyl modification at the second 5' terminal antisense nucleotide, and bears at least one 2' halogen modification on a nucleotide other than the second 5' terminal antisense nucleotide, with the antisense strand having a 3' overhang of two nucleotides, wherein the nucleotides comprise a modified internucleotide linkage between the last nucleotide of the duplex region of the antisense strand and the first overhang nucleotide, and a modified internucleotide linkage between the first overhang nucleotide and the second overhang nucleotide. In a particular embodiment, the 2'-O-alkyl modification is a 2'-O-methyl modification, all pyrimidine nucleotides of the sense strand comprise a 2'-O-methyl modification, the modified internucleotide linkages are each phosphorothioate linkages, and all pyrimidine nucleotides of the antisense strand, comprise a 2'-fluorine modification; and the siRNA is blunt-ended at the 3' end of the sense strand and the 5' end of the antisense strand.

In some applications, it may be desirable to use a label, for example, a fluorescent label. When the label is fluorescent, preferably, the fluorescent label is Cy3^{™}, Cy5^{™}, the Alexa dyes (Molecular Probes, Eugene, OR). These labels are preferably added to the 5' end of the sense strand, more preferably at the 5' terminal sense nucleotide. They may be used to enable users to visualize the distribution of the labeled siRNA within, *e.g.,* a transfected cell, and allow one to assess the success of any given transfection. The use of labeled nucleotides is well known to persons of ordinary skill, and labels other than fluorescent labels, *e.g.,* mass or radioactive labels, may be used in applications in which such types of labels would be advantageous.

The fluorescent modifications can be used to segregate transfected cells from untransfected cells. Specifically, a population of cells can be transfected with the molecules of the first embodiment and subsequently sorted by FACS to segregate transfected cells from untransfected cells. This enables one to obtain a purer population (rather than a mixed one), which in turn improves one's ability to identify clearly phenotypes that result from gene silencing.

The molecules of the above-described embodiments have a variety of uses including, for example, being used as transfection control reagents or stable silencing reagents. When these molecules contain labels, transfection of these molecules into cells allows the user to visualize and to determine what fraction of the cells have been successfully transfected. In addition, these modifications do not appreciably alter siRNA function; thus, the molecules of this embodiment can simultaneously be transfection controls and silencing reagents. Further, because the above-described modifications do not place limitations on the sequences that may be used, they may be used in diverse siRNA and RNAi applications and are not target sequence dependent.

Thus, molecules of the above-described embodiment, with their unique set of modifications, provide stability and "trackability" without altering functionality. They can also be used to isolate a pure population of cells that have been transfected. If 2'-O-alkyl groups are added to the first two or first three nucleotides of the antisense strand, the additional benefit of reducing off-target effects can be realized. Further if 2'-O-alkyl groups are added to the first two or three nucleotides of the sense strand, they can have the additional benefit of reducing sense strand off-target effects.

According to a second embodiment, the present invention provides an siRNA comprising a sense strand and an antisense strand. The sense strand is defined according to the same parameters as the sense strand for the first embodiment, including the 2'-O-alkyl modifications of the first and second 5' terminal sense nucleotides and at the at least one 2'-O-alkyl pyrimidine modification. However, instead of the above-described modifications to the antisense strand, the antisense strand of this embodiment comprises first and second 5' terminal antisense nucleotides, each of which have 2'-O-alkyl modifications. Further, there is at least one 2'-O-alkyl modified pyrimidine on the antisense strand other than the modification that may be present on the first and second 5' terminal antisense nucleotides. Still further, there are no 2' F modifications and there is no phosphorylation of the first 5' terminal antisense nucleotide. The absence of phosphorylation renders the molecule of limited functionality as compared to corresponding functional polynucleotides. Optionally, the 5' end of the sense and/or antisense strand contains a 5' carbon blocking group that prevents phosphorylation of the first terminal sense nucleotide and/or the first terminal antisense nucleotide.

The other preferred parameters with respect to size, overhangs and other modifications are the same as for the first embodiment. However, because these compositions are not used for silencing of genes, they could be 16 - 50 base pairs in length, though are preferably 16-30 base pairs in length.

The molecules of the second embodiment may, in addition to being a transfection control, also act as a potential "filler" or exaequo agent, which is particularly useful in dosage experiments or as a negative control in microarray studies. Many experiments test the effects of a given siRNA at different concentrations (*e.g.,* 100 nM, 50 nM, 25 nM, and 1 nM). Optimally, when transfection experiments are performed, one wants to have a constant concentration of "total siRNA" to avoid any anomalies that result from transfection of different levels of nucleic acids. Unfortunately, addition of any unmodified control siRNA (e.g., a non-specific control) has the potential downside of competing with the siRNA under study for interaction with RISC. Molecules of this embodiment alleviate this problem. The addition of 2'-O-methyl modifications on positions 1 and 2 of both the sense and antisense strands in the absence of a phosphorylated 5' terminal antisense nucleotide, limits the ability of this molecule to interact with RISC. Thus, this molecule can be transfected, but it cannot compete with other siRNA for RISC. As was the case with the molecules of the first embodiment, the addition of 2'-O-methyl groups on the pyrimidines (Cs and Us) minimizes the possibility of nuclease digestion.

The molecules of this embodiment, unlike other "non-specific" sequences or controls, interact poorly with RISC. Thus, these molecules should generate only limited off-targeting side effects, and have the ability of being trackable without competing for sites on RISC.

A third embodiment includes another example of a control or exaequo agent containing only the following 2' carbon modifications: (i) first and second 5' terminal sense nucleotides that each comprises 2' modifications, such as the 2' modifications described above, for example 2'-O-methyl modifications; and (ii) first and second 5' terminal antisense nucleotides that each comprises 2'modifications, such as the 2' modifications described above, for example, 2'-O-methyl modifications, and the 5' terminal sense and antisense nucleotide, has not been phosphorylated and the 5' terminal sense and/or antisense nucleotide may contain a blocking group (*e.g.,* an O-alkyl group such as, for example, a 2'-O-methyl group) that prevents cellular kinases from adding a phosphate group to the 5' carbon position of the first 5' terminal antisense nucleotide inside of a cell or inside of an organism. The modifications can be applied to molecules of varying sizes. The size of said exaequo agents is 16 to 30 base pairs.

a fourth embodiment related to the present invention is a control or exaequo agent containing the following modifications: (i) a first and second, or first, second and third, 5' terminal sense nucleotides that each comprises 2' modifications, such as the 2' modifications described above, for example 2'-O-methyl modifications; (ii) a first and second, or first, second and third, 5' terminal antisense nucleotides that each comprises 2' modifications, such as the 2'modifications described above, for example, 2'-O-ethyl modifications; (iii) at least one 2'-O-alkyl pyrimidine modified sense nucleotide, wherein said at least one 2'-O-alkyl pyrimidine modified sense nucleotide is a nucleotide other than any pyrimidine that may be present as said first 5' terminal sense nucleotide, said second 5' terminal sense nucleotide, or said third 5' terminal sense nucleotide; (iv) at least one 2' halogen modified pyrimidine antisense nucleotide, wherein said at least one 2' halogen modified nucleotide is a nucleotide other than said first, second, or third terminal antisense nucleotides and said halogen is preferably a fluorine atom; and (v) the 5' terminal antisense and sense nucleotides have not been phosphorylated at the 5' carbon position and the 5' terminal sense and/or antisense nucleotide may contain a blocking group (*e.g.,* an O-alkyl group) that prevents cellular kinases from adding a phosphate group to that position. The following modifications can be applied to molecules of varying sizes. Preferably, the size of said exaequo agents ranges between 16 and 30 base pairs.

The above-described embodiments may apply to siRNA that do not have contiguous sense and antisense strands (*i.e.,* two separate strands), as well as to unimolecular polynucleotides that are capable of forming hairpins (shRNA). The term "siRNA" includes both types of RNA. For example, the hairpin may comprise a loop structure, which preferably comprises from four to ten bases, and a sense region, wherein the sense region and antisense regions are independently 19-23 base pairs in length and substantially complementary to each other. Preferable sequences of the loop structure include, for example, 5'-uucg (SEQ. ID NO. 5), 5'-uuuguguag (SEQ. ID NO. 6), and 5'-cuuccuguca (SEQ. ID NO.7).

The hairpin is preferably constructed with the loop region downstream of the antisense region. This construction is desirable particularly with respect to the first embodiment, because it is easier to phosphorylate the terminal antisense nucleotide. Thus, when designing the unimolecular polynucleotide, it is preferable that there are no overhangs upstream of the 5' terminal antisense nucleotide.

When designing a unimolecular polynucleotide, specifically a left-handed unimolecular structure (*i.e.,* 5'-AS-Loop-S) according to the present invention, preferably, the first 5' terminal sense nucleotide is defined as the nucleotide that is the 18^{th}, 19^{th} or 20^{th} base of the sense region counting from the base that is complementary to the first 5' terminal antisense nucleotide (*i.e.* counting from the 3' end of the sense region). The first 5' terminal sense nucleotide is defined in this manner because when unimolecular polynucleotides that are capable of forming hairpins enter a cell, typically, Dicer will process hairpin polynucleotides that contain lengthier duplex regions, into molecules that comprise two separate strands (siRNA) of approximately 18 -20 base pairs, and it is desirable for these molecules to have the sense strand modifications associated with the end of this processed molecule. Most preferably, the first 5' terminal sense nucleotide is defined as the nucleotide that is the 19^{th} base of the sense region from the 3' end of the sense region. Further, preferably, the polynucleotide is capable of forming a left-handed hairpin.

The shRNA can further comprise a stem region, wherein the stem region comprises one or more nucleotides or modified nucleotides immediately adjacent to the 5' end and the 3' end of the loop structure, and wherein the one or more nucleotides or modified nucleotides of the stem region are or are not target-specific. Preferably, the entire length of the unimolecular polynucleotide contains fewer than 100 bases, more preferably fewer than 85 bases.

The unimolecular polynucleotides of the present invention may ultimately be processed by cellular machinery such that they are converted into two separate strands. Alternatively, the molecules may bypass one or more steps in the RNAi pathway (*e.g.,* Dicer processing) and enter RISC as unimolecular hairpin molecules. Further, these unimolecular polynucleotides may be introduced into the cell with less than all modifications, and modified in the cell itself through the use of natural processes or processing molecules that have been introduced (*e.g.,* with respect to the first embodiment, phosphorylation in the cell by native kinases). However, preferably the polynucleotide is introduced with all modifications already present (Similarly, when the siRNA comprises two separate strands, preferably those strands contain all modifications when introduced into the cell with all modifications, though the antisense strand could *e.g.,* be modified after introduction.)

Although the above-described embodiments are directed to increased stability, it is important to note that these and other types of modifications may also affect other parameters, such as specificity (*e.g.,* (1) 2' carbon modifications (preferably-O-methyl modifications) of the first and second (or first, second and third) 5' terminal sense nucleotides in connection with a phosphorylated 5' terminal antisense nucleotide; (2) 2' carbon modifications (preferably-O-methyl modifications) of the first and second (on first, second, and third) 5' terminal sense nucleotides, and a phosphorylated 5' terminal antisense nucleotide, in connection with 2' carbon modifications (preferably-O-methyl modifications) of the first and second (or first, second and third) 5' terminal antisense nucleotides). Further, these stability and functionality modifications may be combined (*e.g.,* 2' carbon modifications (preferably-O-methyl modifications) of the first and second (or first, second and third) 5' terminal sense and antisense nucleotides in conjunction with an additional 2' carbon modifications (preferably-O-methyl modifications) of at least one sense pyrimidine, preferably all sense pyrimidine(s) in addition to any pyrimidine located at the first two (or three) sense nucleotides that have 2' carbon modifications (preferably-O-methyl modifications), at least one, preferably all, 2'-F modifications of antisense pyrimidine(s) other than any pyrimidines that may be present as the first two or three nucleotides that have 2' carbon modifications (preferably-O-methyl modifications) and phosphorylation of the 5' terminal antisense nucleotide. Further, the above described modifications of the present invention may be combined with siRNA that contain sequences that were selected at random, or according to rationale design as described in, for example, U.S. Patent Application US 2007/0031844.

Additionally stabilization modifications that are addressed to the phosphate backbone may be included in the polynucleotides for some applications of the present invention. For example, at least one phosphorothioate and/or methylphosphonate may be substituted for the phosphate group at some or all 3' positions of any or all pyrimidines in the sense and/or antisense strands of the oligonucleotide backbone, as well as in any overhangs, loop structures or stem structures that may be present. Phosphorothioate (and methylphosphonate) analogues arise from modification of the phosphate groups in the oligonucleotide backbone. In the phosphorothioate, the phosphate O⁻ is replaced by a sulfur atom. In methylphosphonates, the oxygen is replaced with a methyl group. In one embodiment the phosphorothioate modification or methylphosphonate is located at the 3' positions of all antisense strand nucleotides that also contains 2' fluoro (or other halogen) modified nucleotides. Additionally, phosphorothioate 3' modifications may be used instead of and independent of 2' fluoro modifications to increase stability of an siRNA molecule. These modifications may be used in combination with the other modifications disclosed herein, or independent of those modifications in siRNA applications.

Nucleases typically use both the oxygen groups on the phosphate moiety and the 2'OH position of the ribose ring to mediate attack on RNA. Substitution of a sulfur group for one of the oxygens eliminates the ability of the phosphate to participate in this reaction, thus limiting the sensitivity of this site to nuclease digestion. However, it should be noted that phosphorothioates are typically toxic, thus, they would be beneficial primarily when any toxic effects are negated, which it is postulated might be accomplished by limiting the use of this modification to *e.g.,* every other nucleotide, every third nucleotide, or every fourth nucleotide.

The molecules of the second embodiment are of limited functionality, but as noted above, may be used in negative control studies, and as exaequo agents. When using exaequo agents or controls, it may be desirable to modify the 5' carbon position of the 5' end of the sense and/or the antisense strand with a blocking group. The blocking group may for example be an alkyl group or any other group that prevents phosphorylation of the 5' carbon position of the nucleotide. Phosphorylation may occur in a cell due to the activity of kinases that are present in cells. Exemplary blocking groups include but are not limited to methyl, O-methyl, and amine groups.

According to any of the embodiments, the RNA duplexes may further comprise a label. The label can be attached, for example, to the first 5' terminal sense nucleotide. The label can be any label known in the art, for example, a fluorescent label. In one aspect, all pyrimidines of the sense strands of the fifth through tenth embodiments can comprise a 2'-O-alkyl modification. In another aspect, all pyrimidines on the antisense strand of the sixth seventh, ninth, and tenth embodiments, except for any pyrimidines that may be present as the first 5' terminal antisense nucleotide and the second 5' terminal antisense nucleotide of the sixth and ninth embodiments, and except for any pyrimidines that may be present as the first, second, and third 5' antisense nucleotides of the seventh and tenth embodiments, can comprise a 2'-fluoro modification. Any of the embodiments can comprise at least one phosphorothioate or methylphosphonate internucleotide linkage.

Any of the embodiments, with the exception of the first embodiment, can be used in a method of monitoring gene silencing comprising using a control reagent, wherein said control reagent is an RNA duplex, by introducing a duplex into a cell that is expressing or is capable of expressing a target gene

The control or exaequo agents described above can further comprise a label. The label is preferably attached to the first 5' terminal sense nucleotide of the control or exaequo agent. Preferably, the label is a fluorescent dye.

The present invention may be used advantageously with diverse cell types, including but not limited to primary cells, germ cell lines and somatic cells. The cells may be stem cells or differentiated cells. For example, the cell types may be embryonic cells, oocytes, sperm cells, adipocytes, fibroblasts, myocytes, cardiomyocytes, endothelium, neurons, glia, blood cells, megakaryocytes, lymphocytes, macrophages, neutrophils, eosinophils, basophils, mast cells, leukocytes, granulocytes, keratinocytes, chondrocytes, osteoblasts, osteoclasts, hepatocytes and cells of the endocrine or exocrine glands.

The present invention is applicable for use for employing RNA interference (and/or using as a control) directed against a broad range of genes, including but not limited to the 45,000 genes of a human genome, such as those implicated in diseases such as diabetes, Alzheimer's and cancer, as well as all genes in the genomes of the humans, mice, hamsters, chimpanzees, goats, sheep, horses, camels, pigs, dogs, cats, nematodes *(e.g.*, *C. elegans),* flies (*e.g.*, *D. melanogaster*), and other vertebrates and invertebrates.

The polynucleotides of the present invention may be administered to a cell by any method that is now known or that comes to be known and that from reading this disclosure, one skilled in the art would conclude would be useful with the present invention. For example, the polynucleotides may be passively delivered to cells.

Passive uptake of modified polynucleotides can be modulated, for example, by the presence of a conjugate such as a polyethylene glycol moiety or a cholesterol moiety at the 5' terminal of the sense strand and/or, in appropriate circumstances, a pharmaceutically acceptable carrier.

Other methods for delivery include, but are not limited to, transfection techniques employing DEAE-Dextran, calcium phosphate, cationic lipids/liposomes, microinjection, electroporation, immunoporation, and coupling of the polynucleotides to specific conjugates or ligands such as antibodies, antigens, or receptors.

Further, the method of assessing the level of gene silencing is not limited. Thus, the silencing ability of any given siRNA can be studied by one of any number of art tested procedures including but not limited to Northern analysis, Western Analysis, RT PCR, expression profiling, and others.

Any of the siRNA, or RNA duplexes, described herein can be used in a method of performing RNA interference, comprising introducing the RNA duplex into a cell in the presence or absence of an RNA duplex that is capable of silencing a target gene.

Further, the RNAs of the present invention may be used in a diverse set of applications, including but not limited to basic research, drug discovery and development, diagnostics and therapeutics. For example, the present invention may be used to validate whether a gene product is a target for drug discovery or development. In this application, the mRNA that corresponds to a target nucleic acid sequence of interest is identified for targeted degradation. Inventive polynucleotides that are specific for targeting the particular gene are introduced into a cell or organism, preferably in double stranded form. The cell or organism is maintained under conditions allowing for the degradation of the targeted mRNA, resulting in decreased activity or expression of the gene. The extent of any decreased expression or activity of the gene is then measured, along with the effect of such decreased expression or activity, and a determination is made that if expression or activity is decreased, then the nucleic acid sequence of interest is an agent for drug discovery or development. In this manner, phenotypically desirable effects can be associated with RNA interference of particular target nucleic acids of interest, and in appropriate cases toxicity and pharmacokinetic studies can be undertaken and therapeutic preparations developed.

The present invention may also be used in RNA interference applications that induce transient or permanent states of disease or disorder in an organism by, for example, attenuating the activity of a target nucleic acid of interest believed to be a cause or factor in the disease or disorder of interest. Increased activity of the target nucleic acid of interest may render the disease or disorder worse, or tend to ameliorate or to cure the disease or disorder of interest, as the case may be. Likewise, decreased activity of the target nucleic acid of interest may cause the disease or disorder, render it worse, or tend to ameliorate or cure it, as the case may be. Target nucleic acids of interest can comprise genomic or chromosomal nucleic acids or extrachromosomal nucleic acids, such as viral nucleic acids.

Further, the present invention may be used in RNA interference applications that determine the function of a target nucleic acid or target nucleic acid sequence of interest. For example, knockdown experiments that reduce or eliminate the activity of a certain target nucleic acid of interest. This can be achieved by performing RNA interference with one or more siRNAs targeting a particular target nucleic acid of interest. Observing the effects of such a knockdown can lead to inferences as to the function of the target nucleic acid of interest. RNA interference can also be used to examine the effects of polymorphisms, such as biallelic polymorphisms, by attenuating the activity of a target nucleic acid of interest having one or the other allele, and observing the effect on the organism or system studied. Therapeutically, one allele or the other, or both, may be selectively silenced using RNA interference where selective allele silencing is desirable.

Still further, the present invention may be used in RNA interference applications, such as diagnostics, prophylactics, and therapeutics including use of the composition in the manufacture of a medicament in animals, preferably mammals, more preferably humans in the treatment of diseases, or over or under expense of a target. Preferably, the disease or disorder is one that arises from the malfunction of one or more proteins, the disease or disorder of which is related to the expression of the gene product of the one or more proteins. For example, it is widely recognized that certain cancers of the human breast are related to the malfunction of a protein expressed from a gene commonly known as the "bcl-2" gene. A medicament can be manufactured in accordance with the compositions and teachings of the present invention, employing one or more siRNAs directed against the bcl-2 gene, and optionally combined with a pharmaceutically acceptable carrier, diluent and/or adjuvant, which medicament can be used for the treatment of breast cancer. Applicants have established the utility of the methods and compositions in cellular models. Methods of delivery of polynucleotides to cells within animals, including humans, are well known in the art. Any delivery vehicle now known in the art, or that comes to be known, and has utility for introducing polynucleotides to animals, including humans, is expected to be useful in the manufacture of a medicament in accordance with the present invention, so long as the delivery vehicle is not incompatible with any modifications that may be present a composition made according to the present invention. A delivery vehicle that is not compatible with a composition made according to the present invention is one that reduces the efficacy of the composition by greater than 95% as measured against efficacy in cell culture.

Animal models exist for many, many disorders, including, for example, cancers, diseases of the vascular system, inborn errors or metabolism, and the like. It is within ordinary skill in the art to administer nucleic acids to animals in dosing regimens to arrive at an optimal dosing regimen for particular disease or disorder in an animal such as a mammal, for example, a mouse, rat or non-human primate. Once efficacy is established in the mammal by routine experimentation by one of ordinary skill, dosing regimens for the commencement of human trials can be arrived at based on data arrived at in such studies.

Dosages of medicaments manufactured in accordance with the present invention may vary from micrograms per kilogram to hundreds of milligrams per kilogram of a subject. As is known in the art, dosage will vary according to the mass of the mammal receiving the dose, the nature of the mammal receiving the dose, the severity of the disease or disorder, and the stability of the medicament in the serum of the subject, among other factors well known to persons of ordinary skill in the art.

For these applications, an organism suspected of having a disease or disorder that is amenable to modulation by manipulation of a particular target nucleic acid of interest is treated by administering siRNA. Results of the siRNA treatment may be ameliorative, palliative, prophylactic, and/or diagnostic of a particular disease or disorder. Preferably, the siRNA is administered in a pharmaceutically acceptable manner with a pharmaceutically acceptable carrier or diluent.

Therapeutic applications of the present invention can be performed with a variety of therapeutic compositions and methods of administration. Pharmaceutically acceptable carriers and diluents are known to persons skilled in the art. Methods of administration to cells and organisms are also known to persons skilled in the art. Dosing regimens, for example, are known to depend on the severity and degree of responsiveness of the disease or disorder to be treated, with a course of treatment spanning from days to months, or until the desired effect on the disorder or disease state is achieved. Chronic administration of siRNAs may be required for lasting desired effects with some diseases or disorders. Suitable dosing regimens can be determined by, for example, administering varying amounts of one or more siRNAs in a pharmaceutically acceptable carrier or diluent, by a pharmaceutically acceptable delivery route, and amount of drug accumulated in the body of the recipient organism can be determined at various times following administration. Similarly, the desired effect (for example, degree of suppression of expression of a gene product or gene activity) can be measured at various times following administration of the siRNA, and this data can be correlated with other pharmacokinetic data, such as body or organ accumulation. Those of ordinary skill can determine optimum dosages, dosing regimens, and the like. Those of ordinary skill may employ EC₅₀ data from *in vivo* and *in vitro* animal models as guides for human studies.

Further, the polynucleotides can be administered in a cream or ointment topically, an oral preparation such as a capsule or tablet or suspension or solution, and the like. The route of administration may be intravenous, intramuscular, dermal, subdermal, cutaneous, subcutaneous, intranasal, oral, rectal, by eye drops, by tissue implantation of a device that releases the siRNA at an advantageous location, such as near an organ or tissue or cell type harboring a target nucleic acid of interest.

The polynucleotides of the present invention may be synthesized by any method that is now known or that comes to be known and that from reading this disclosure a person of ordinary skill in the art would appreciate would be useful to synthesize the molecules of the present invention. siRNA duplexes containing the specified modifications maybe chemically synthesized using compositions of matter and methods described in Scaringe, S.A. (2000) "Advanced 5'-silyl-2'-orthoester approach to RNA oligonucleotide synthesis," Methods Enzymol. 317, 3-18; Scaringe, S.A. (2001) "RNA oligonucleotide synthesis via 5'-silyl-2'-orthoester chemistry," Methods 23, 206-217; Scaringe, S. and Caruthers, M.H. (1999) U.S. Patent No. 5,889,136; Scaringe, S. and Caruthers, M.H. (1999) U.S. Patent No. 6,008,400; Scaringe, S. (2000) U.S. Patent No. 6111086; Scaringe, S. (2003) U.S. Patent No. 6,590,093. The synthesis method utilizes nucleoside base-protected 5'-O-silyl-2'-O-orthoester-3'-O-phosphoramidites to assemble the desired unmodified siRNA sequence on a solid support in the 3' to 5' direction. Briefly, synthesis of the required phosphoramidites begins from standard base-protected ribonucleosides (uridine, N⁴-acetylcytidine, N²-isobutyrylguanosine and N⁶-isobutyryladenosine). Introduction of the 5'-O-silyl and 2'-O-orthoester protecting groups, as well as the reactive 3'-O-phosphoramidite moiety is then accomplished in five steps, including:

Simultaneous transient blocking of the 5'- and 3'-hydroxyl groups of the nucleoside sugar with Markiewicz reagent (1,3-dichloro-1,1,3,3,-tetraisopropyldisiloxane [TIPS-Cl₂]) in pyridine solution {Markiewicz, W.T. (1979) "Tetraisopropyldisiloxane-1,3-diyl, a Group for Simultaneous Protection of 3'- and 5'-Hydroxy Functions of Nucleosides," J. Chem. Research(S), 24-25}, followed by chromatographic purification;

Regiospecific conversion of the 2'-hydroxyl of the TIPS-nucleoside sugar to the bis(acetoxyethyl)orthoester [ACE derivative] using tris(acetoxyethyl)-orthoformate in dichloromethane with pyridinium p-toluenesulfonate as catalyst, followed by chromatographic purification;

Liberation of the 5'- and 3'-hydroxyl groups of the nucleoside sugar by specific removal of the TIPS-protecting group using hydrogen fluoride and N,N,N"N'-tetramethylethylene diamine in acetonitrile, followed chromatographic purification;

Protection of the 5'-hydroxyl as a 5'-O-silyl ether using benzhydroxy-bis(trimethylsilyloxy)silyl chloride [BzH-Cl] in dichloromethane, followed by chromatographic purification; and

Conversion to the 3'-O-phosphoramidite derivative using *bis*(N,N-diisopropylamino)methoxyphosphine and 5-ethylthio-1H-tetrazole in dichloromethane/acetonitrile, followed by chromatographic purification.

The phosphoramidite derivatives are typically thick, colorless to pale yellow syrups. For compatibility with automated RNA synthesis instrumentation, each of the products is dissolved in a pre-determined volume of anhydrous acetonitrile, and this solution is aliquoted into the appropriate number of serum vials to yield a 1.0-mmole quantity of phosphoramidite in each vial. The vials are then placed in a suitable vacuum desiccator and the solvent removed under high vacuum overnight. The atmosphere is then replaced with dry argon, the vials are capped with rubber septa, and the packaged phosphoramidites are stored at -20°C until needed. Each phosphoramidite is dissolved in sufficient anhydrous acetonitrile to give the desired concentration prior to installation on the synthesis instrument.

The synthesis of the desired oligoribonucleotide is carried out using automated synthesis instrumentation. It begins with the 3'-terminal nucleoside covalently bound via its 3'-hydroxyl to a solid beaded polystyrene support through a cleavable linkage. The appropriate quantity of support for the desired synthesis scale is measured into a reaction cartridge, which is then affixed to synthesis instrument. The bound nucleoside is protected with a 5'-O-dimethoxytrityl moiety, which is removed with anhydrous acid (3% [v/v] dichloroacetic acid in dichloromethane) in order to free the 5'-hydroxyl for chain assembly.

Subsequent nucleosides in the sequence to be assembled are sequentially added to the growing chain on the solid support using a four-step cycle, consisting of the following general reactions:

Coupling: the appropriate phosphoramidite is activated with 5-ethylthio-1H-tetrazole and allowed to react with the free 5'-hydroxyl of the support bound nucleoside or oligonucleotide. Optimization of the concentrations and molar excesses of these two reagents, as well as of the reaction time, results in coupling yields generally in excess of 98% per cycle.

Oxidation: the internucleotide linkage formed in the coupling step leaves the phosphorous atom in its P(III) [phosphite] oxidation state. The biologically relevant oxidation state is P(V) [phosphate]. The phosphorous is therefore oxidized from P(III) to P(V) using a solution of tert-butylhydroperoxide in toluene.

Capping: the small quantity of residual un-reacted 5'-hydroxyl groups must be blocked from participation in subsequent coupling cycles in order to prevent the formation of deletion-containing sequences. This is accomplished by treating the support with a large excess of acetic anhydride and 1-methylimidazole in acetonitrile, which efficiently blocks residual 5'-hydroxyl groups as acetate esters.

De-silylation: the silyl-protected 5'-hydroxyl must be deprotected prior to the next coupling reaction. This is accomplished through treatment with triethylamine trihydrogen fluoride in N,N-dimethylformamide, which rapidly and specifically liberates the 5'-hydroxyl without concomitant removal of other protecting groups (2'-O-ACE, N-acyl base-protecting groups, or phosphate methyl).

It should be noted that in between the above four reaction steps are several washes with acetonitrile, which are employed to remove the excess of reagents and solvents prior to the next reaction step. The above cycle is repeated the necessary number of times until the unmodified portion of the oligoribonucleotide has been assembled. The above synthesis method is only exemplary and should not be construed as limited the means by which the molecules may be made. Any method that is now known or that comes to be known for synthesizing siRNA and that from reading this disclosure one skilled in the art would conclude would be useful in connection with the present invention may be employed.

The siRNA duplexes of certain embodiments of this invention include two modified nucleosides (*e.g*., 2'-O-methyl derivatives) at the 5'-end of each strand. The 5'-O-silyl-2'-O-methyl-3'-O-phosphoramidite derivatives required for the introduction of these modified nucleosides are prepared using procedures similar to those described previously (steps 4 and 5 above), starting from base-protected 2'-O-methyl nucleosides [2'-O-methyl-uridine, 2'-O-methyl- N⁴-acetylcytidine, 2'-O-methyl-N²-isobutyrylguanosine and 2'-O-methyl-N⁶-isobutyryladenosine). The absence of the 2'-hydroxyl in these modified nucleosides eliminates the need for ACE protection of these compounds. As such, introduction of the 5'-O-silyl and the reactive 3'-O-phosphoramidite moiety is accomplished in two steps, including:

Protection of the 5'-hydroxyl as a 5'-O-silyl ether using benzhydroxy-bis(trimethylsilyloxy)silyl chloride [BzH-Cl] in N,N-dimethylformamide, followed by chromatographic purification; and

Conversion to the 3'-O-phosphoramidite derivative using *bis*(N,N-diisopropylamino)methoxyphosphine and 5-ethylthio-1H-tetrazole in dichloromethane/acetonitrile, followed by chromatographic purification.

Post-purification packaging of the phosphoramidites is carried out using the procedures described previously for the standard nucleoside phosphoramidites. Similarly, the incorporation of the two 5'-O-silyl-2'-O-methyl nucleosides via their phosphoramidite derivatives is accomplished by twice applying the same four-step cycle described previously for the standard nucleoside phosphoramidites.

The siRNA duplexes of certain embodiments include a phosphate moiety at the 5'-end of the antisense strand. This phosphate is introduced chemically as the final coupling to the antisense sequence. The required phosphoramidite derivative (*bis*(cyanoethyl)-N,N-diisopropylamino phosphoramidite) is synthesized as follows in brief: phosphorous trichloride is treated one equivalent of N,N-diisopropylamine in anhydrous tetrahydrofuran in the presence of excess triethylamine. Then, two equivalents of 3-hydroxypropionitrile are added and allowed to react completely. Finally, the product is purified by chromatography. Post-purification packaging of the phosphoramidite is carried out using the procedures described previously for the standard nucleoside phosphoramidites. Similarly, the incorporation of the phosphoramidite at the 5'-end of the antisense strand is accomplished by applying the same four-step cycle described previously for the standard nucleoside phosphoramidites.

The modified, protected oligoribonucleotide remains linked to the solid support at the finish of chain assembly. A two-step rapid cleavage/deprotection procedure is used to remove the phosphate methyl protecting groups, cleave the oligoribonucleotide from the solid support; and remove the N-acyl base-protecting groups. It should be noted that this procedure also removes the cyanoethyl protecting groups from the 5'-phosphate on the antisense strand. Additionally, the procedure removes the acetyl functionalities from the ACE orthoester, converting the 2'-O-ACE protecting group into the bis(2-hydroxyethyl)orthoester. This new orthoester is significantly more labile to mild acid as well as more hydrophilic than the parent ACE group. The two-step procedure is briefly as follows:

The support-bound oligoribonucleotide is treated with a solution of disodium 2-carbamoyl-2-cyanoethylene-1,1-dithiolate trihydrate in N,N-dimethylformamide. This reagent rapidly and efficiently removes the methyl protecting groups from the internucleotide phosphate linkages without cleaving the oligoribonucleotide from the solid support. The support is then washed with water to remove excess dithiolate.

The oligoribonucleotide is cleaved from the solid support with 40% (w/v) aqueous methylamine at room temperature. The methylamine solution containing the crude oligoribonucleotide is then heated to 55°C to remove the protecting groups from the nucleoside bases. The crude orthoester-protected oligoribonucleotide is obtained following solvent removal *in vacuo.*

Removal of the 2'-orthoesters is the final step in the synthesis process. This is accomplished by treating the crude oligoribonucleotide with an aqueous solution of acetic acid and N,N,N',N'-tetramethyl ethylene diamine, pH 3.8, at 55°C for 35 minutes. The completely deprotected oligoribonucleotide is then desalted by ethanol precipitation and isolated by centrifugation.

In addition, incorporation of fluorescent labels at the 5'-terminus of a polynucleotide is a common and well-understood manipulation for those skilled in the art. In general, there are two methods that are employed to accomplish this incorporation, and the necessary materials are available from several commercial sources (*e.g*., Glen Research Inc., Sterling, Virginia, USA; Molecular Probes Inc., Eugene, Oregon, USA; TriLink BioTechnologies Inc., San Diego, California, USA; and others). The first method utilizes a fluorescent molecule that has been derivatized with a phosphoramidite moiety similar to the phosphoramidite derivatives of the nucleosides described previously. In such case, the fluorescent dye is appended to the support-bound polynucleotide in the final cycle of chain assembly. The fluorophore-modified polynucleotide is then cleaved from the solid support and deprotected using the standard procedures described above. This method has been termed "direct labeling." Alternatively, the second method utilizes a linker molecule derivatized with a phosphoramidite moiety that contains a protected reactive functional group (*e.g*., amino, sulfhydryl, carbonyl, carboxyl, and others). This linker molecule is appended to the support-bound polynucleotide in the final cycle of chain assembly. The linker-modified polynucleotide is then cleaved from the solid support and deprotected using the standard procedures described above. The functional group on the linker is deprotected either during the standard deprotection procedure, or by utilizing a subsequent group-specific treatment. The crude linker-modified polynucleotide is then reacted with an appropriate fluorophore derivative that will result in formation of a covalent bond between a site on the fluorophore and the functional group of the linker. This method has been termed "indirect labeling."

In developing the present invention, two or more different modifications were added to a duplex to increase stability. Applicants appreciate that other modifications and combinations may be discovered in the future that assist in improving stability. Additionally, the modifications of the present invention could be combined with modifications that are desired for other purposes. For example, in some instances, one modification could stabilize the molecule against one particular set of conditions (*e.g*., one type of nuclease) while a second modification could stabilize the molecule against a second set of conditions (*e.g*., a different family of nucleases). Alternatively, two separate modifications could act additively or synergistically to stabilize a molecule towards a certain set of conditions. In still other instances, one modification could stabilize the molecule, but have detrimental consequences on other desirable properties, *e.g*., the potency or toxicity of the siRNA. In cases such as these, additional modifications could be added that restore these aspects of functionality of the molecule.

A variety of approaches can be used to identify both the type of molecule and the key position(s) needed to enhance stability. In one non-limiting example, a modification-function walk is performed. In this procedure, a single type of modification is added to one or more nucleotides across the sense and/or antisense strand. Subsequently, modified and unmodified molecules are tested for (1) functionality and (2) stability, by one of several methods. Thus, for example, 2'-O-Me groups can be added to positions 1 and 2, 3 and 4, 5 and 6, 7 and 8, 9 and 10, 11 and 12, 13 and 14, 15 and 16, 17 and 18, or 18 and 19 of either the sense and/or antisense strand and tested for functionality (*e.g*., by measuring the ability of these molecules to silence specific targets) and stabilize the molecule against actions by *e.g*. nucleases. If key positions are identified that enhance stability, but result in a loss of duplex functionality, then a second round of modification walks, whereby additional chemical groups (*e.g*., 5' phosphate on the 5' end of the antisense strand), mismatches, or bulges that are suspected to increase duplex functionality can be added to molecules that already contain the modification that enhance stability.

Having described the invention with a degree of particularity, examples will now be provided. These examples are not intended to and should not be construed to limit the scope of the claims in any way. Although the invention may be more readily understood through reference to the following examples, they are provided by way of illustration and are not intended to limit the present invention unless specified.

### EXAMPLES - All for reference only (not part of present invention)

For the purposes of these examples, the phrase "molecule 1 modifications" refers to molecules that contain 2'-O-methyl modifications on positions 1 and 2 of the sense strand, 2'-O-methyl modifications on all Cs and Us of the sense strand, 2'-fluoro ("F or FI," *i.e*., 2'-fluorine) modification of all Cs and Us of the antisense strand, and a phosphate modification on the 5' terminus of the antisense strand. In addition, these molecules can comprise a 3' overhang on one or both strands. The phrase "molecule 2 modifications" refers to molecules that contain 2'-O-methyl modifications on positions 1 and 2 of the sense strand, 2'-O-methyl modifications on all Cs and Us of the sense strand, a Cy3 label on the 5' end of the sense strand, 2' F modifications on all Cs and Us of the antisense strand, and a phosphate modification on the 5' terminus of the antisense strand. In addition, these molecules can comprise a 3' overhang on one or both strands. The phrase "molecule 3 modifications" refers to siRNA that comprise 2'-O-methyl modifications on positions 1 and 2 of the sense strand, and 2'-O-methyl modifications on positions 1 and 2 of the antisense strand. In addition, siRNA containing molecule 3 modifications can also contain stabilizing modifications including 2'-O-methyl modifications on the Cs and Us of the sense and antisense strand, 2' F modifications on the Cs and Us of the sense and antisense strand, or any combination of the above. In addition, these molecules can comprise a 3' overhang on one or both strands. The phrase "molecule 4 modifications" refers to siRNA that contain a 5' deoxy modification on the 5' end of the sense and/or antisense strand. In addition, siRNA containing molecule 3 modifications can also contain stabilizing modifications including 2'-O-methyl modifications on the Cs and Us of the sense and antisense strand, 2' F modifications on the Cs and Us of the sense and antisense strand, or any combination of the above. In addition, these molecules can comprise a 3' overhang on one or both strands. The phrase "molecule 5 modifications" refers to siRNA that contain the following design: 2'-O-methyl modification of positions 1 and 2 of the sense strand, 2'-O-methy modification of all Cs and Us of the sense strand, 2' F modification of all Cs and Us of the antisense strand, a phosphate group on the 5' carbon of the first (5' most) nucleotide of the antisense strand, a 2 basepair 3' overhang on the antisense strands, and stabilized phosphorothioate internucleotide linkages between the two nucleotides of the 3' overhang, and the first nucleotide of the overhang and the adjacent nucleotide that is complementary to the first nucleotide (5'-most) of the sense strand The phrase "molecule 6 modifications" refers to molecules that contain molecule 5 modifications plus an additional 2'-O-methyl group on the second 5' terminal nucleotide of the antisense strand.

### EXAMPLE 1

### GENERAL SYNTHESIS OF 5'-DEOXYNUCLEOSIDE PHOSPHORAMIDITES

For synthesis of 5'deoxy modified molecules, the following procedures were followed:

Intermediates that are commonly used in the synthesis of standard 5'-silyl phosphoramidites (Scaringe, S.A., Kitchen, D., Kaiser, R., Marshall, W.M. (2004), "Preparation of 5'-Silyl-2'-Orthoester Ribonucleosides for Use in Oligoribonucleotide Synthesis," in: Current Protocols in Nucleic Acid Chemistry, edited by Beaucage, S.L., vol. 1. pp. 2.10.11-15. New York: John Wiley & Sons, Inc.) are utilized as starting materials in the synthesis of 5'-deoxynucleoside phosphoramidites. In order to, convert selectively the 5'-hydroxyl position of the nucleoside, the 5'-benzhydryloxy-bis(trimethylsilyloxy)silyl [BZH]-protected nucleosides **I** are further protected at the 3'-hydroxyl position with t-butyldimethylsilyl [TBDMS] chloride. The 5'-BZH group is then selectively removed in the presence of the 3'-TBDMS group with a mixture of hydrofluoric acid and N,N,N',N'-tetramethylethylene diamine to give intermediate **II**. The 5'-hydroxyl group is converted into the 5'-iodide **III** by the use of methyltriphenoxyphosphonium iodide (Verheyden, J. P. H., Moffat, J. G. (1970), "Halo Sugar Nucleosides. I. Iodination of the Primary Hydroxyl Groups of Nucleosides with Methyltriphenoxyphosphonium Iodide," J. Org. Chem. 35, 2319-2326). Hydrogenation of the 5'-iodide in the presence of catalystic palladium on carbon and subsequent 3'-desilyation with tetrabutylammonium fluoride furnishes the 5'-deoxy-derivative **IV**. Phosphitylation of the 3'-hydroxyl with methyl tetraisopropylphosphorodiamidite and 5-ethylthio-1*H-*tetrazole produces the desired 5'-deoxyphosphoramidite **V**.

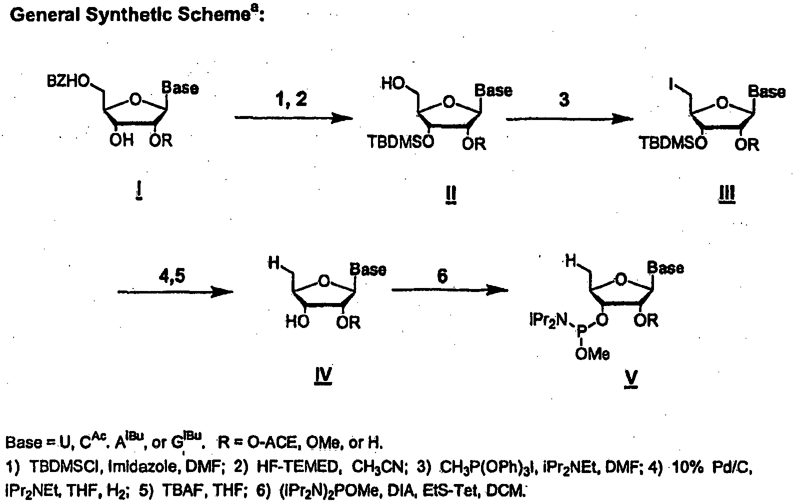

### EXAMPLE II

### SYNTHESIS METHODS FOR MAKING 5'-DEOXY OLIGOMERS

Making 2'-*O-bis*(2-acetoxyethoxy)methyl- 3'-*O-t*-butydimethylsilyluridine (**2**):

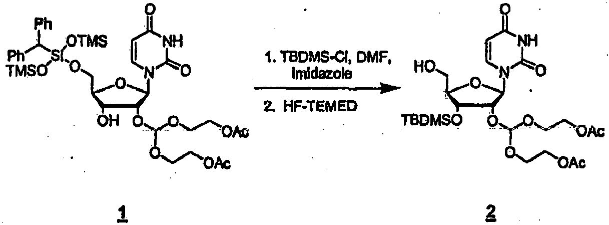

2'-*O*-bis(2-acetoxyethoxy)methyl-5'-*O*-[benzhydryloxy-bis(trimethylsilyloxy)silyl]- uridine, **1**, (Scaringe, S.A., Kitchen, D., Kaiser, R., Marshall, W.M. (2004), "Preparation of 5'-Silyl-2'-Orthoester Ribonucleosides for Use in Oligoribonucleotide Synthesis," in: Current Protocols in Nucleic Acid Chemistry, edited by Beaucage, S.L., vol. 1. pp. 2.10.11-15. New York: John Wiley & Sons, Inc.), (7.3 g, 8.6 mmol), and imidazole (1.8 g, 25.8 mmol) are dissolved in N,N-dimethylformamide [DMF] (40 mL), t-butyldimethylsilyl chloride [TBDMS-Cl] (1.9 g, 12.9 mmol) is added. The solution is stirred for 24 hours at room temperature. The reaction mixture is then diluted with water (100 mL) and extracted with ether (3 x 100 mL). The combined organic extracts are washed with water then saturated aqueous sodium chloride. The solution is dried with anhydrous sodium sulfate and evaporated to produce a gummy residue.

A solution of N,N,N',N'-tetramethylethylene diamine [TEMED] (3.9 mL, 25.8 mmol) in acetonitrile (86 mL) is cooled to 0 ºC and 48% hydrofluoric acid (0.33 mL, 9.0 mmol) is added dropwise. This solution is allowed to stir for 5 minutes and is then added to the crude product from above at room temperature. The reaction is stirred for 1 hour and the solution is then evaporated to dryness. The crude product is purified by flash chromatography (200 mL silica gel; 60:20:20 (v:v:v) hexanes:ethyl acetate:acetone containing 0.1% (v/v) TEMED to 40:40:20 (v:v:v) hexanes:ethyl acetate:acetone containing 0.1% (v/v) TEMED) to afford **2** as a white foam (1.8 g, 36%).

Making 2'-*O*-*bis*(2-acetoxyethoxy)methyl-3'-O-t-butydimethylsilyl-5'-iodouridine (3):

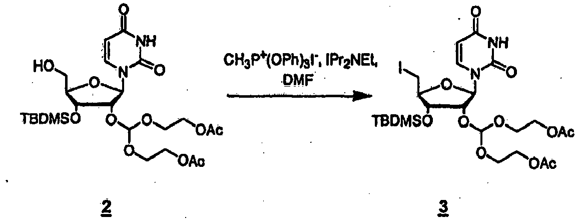

Methyltriphenoxyphosphonium iodide (2.1 g, 4.7 mmol) is added to a stirred solution of compound **2** (1.8 g, 3.1 mmol) in DMF (21 mL), and N,N-diisopropylethylamine [DIEA] (1.4 mL, 7.8 mmol) (Verheyden, J. P. H., Moffat, J. G. (1970), "Halo Sugar Nucleosides. I. Iodination of the Primary Hydroxyl Groups of Nucleosides with Methyltriphenoxyphosphonium Iodide," J. Org. Chem. 35, 2319-2326). After 1 hour the reaction is stopped by the addition of methanol and evaporated to dryness. The resulting brown paste is dissolved in dichloromethane (100 mL) and washed with saturated aqueous sodium thiosulfate followed by water. The combined aqueous phases are extracted with dichloromethane. The combined organic layers are dried with anhydrous sodium sulfate and evaporated to dryness. The crude product is purified by flash chromatography (150 mL silica gel; 25:75 (v:v) ethyl acetate:hexanes containing 0.1% (v:v) triethylamine [TEA] to 50:50(v:v) ethyl acetate:hexanes containing 0.1% (v:v) TEA) to give **3** (1.7 g, 81%).

Making 2'-*O*-bi(2-acetoxyethoxy)methyl-5'- deoxyuridine (**4**):

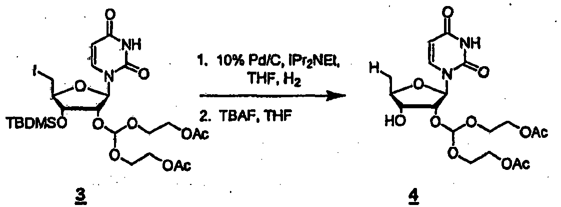

Compound **3** (1.7 g, 2.5 mmol) is dissolved in a solution of tetrahydrofuran [THF] (50 mL) and DIEA (0.9 mL, 5.0 mmol). Pd on carbon (10% (w/w); 0.7 g) is added, and the mixture is stirred under hydrogen at atmospheric pressure for 16 hours at room temperature. The suspension is filtered through a pad of Celite in a glass-fritted funnel. The solid is washed well with ethyl acetate then methanol, and the filtrate is evaporated to dryness. The resulting powder is dissolved in THF (50 mL) and tetrabutylammonium fluoride hydrate [TBAF] (1.2 g, 4.6 mmol) is added. After 5 hours the solution is evaporated to dryness and the crude product is purified by flash chromatography (100 mL silica gel; 50:50 (v:v) ethyl acetate:hexanes containing 0.1 % (v:v) TEA to ethyl acetate containing 0.1% (v:v) TEA) to give **4** (0.9 g, 79%).

2'-*O-bis*(2-acetoxyethoxy)methyl- 5'- deoxyuridine - 3'-methyl-*N,N*,-diisopropylamino phosphoramidite (**5**):

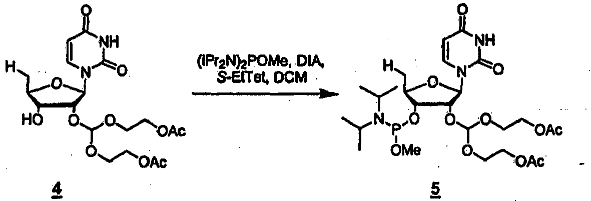

Methyl tetraisopropylphosphorodiamidite (0.8 g, 3.0 mmol) is dissolved in dichloromethane [DCM] (10 mL) and a solution of 5-ethylthio-1*H*-tetrazole in acetonitrile [S-EtTet] (0.45 M; 2.2 mL, 1.0 mmol) is added. Diisopropylamine [DIA] (0.3 mL; 2.0 mmol) is then added and this solution is stirred for 5 minutes at room temperature. In a separate flask compound **4** (0.9 g, 2.0 mmol) and DIA (0.3 mL, 2.0 mmol) are dissolved in DCM (10 mL). The phosphitylation solution is poured into the nucleoside solution and the reaction is stirred at room temperature. After 16 hours the reaction is quenched with absolute ethanol (5 mL) and evaporated to dryness. The resulting paste is purified by flash chromatography (50 mL silica gel; 95:5 (v:v) hexanes:dichloromethane containing 1% (v:v) TEA to 70:30 (v:v) hexanes:acetone containing 0.1% (v:v) TEA) to yield compound **5** as a colorless oil (1.1 g, 95%). ³¹P NMR (CD₃CN) δ 151.65, 151.03 ppm.

### 3'-O-acetyl-2'-O-methyluridine (7):

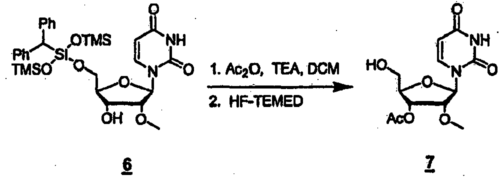

A solution of 2'-*O*-methyl-5'-*O*-[benzhydryloxy-*bis*(trimethylsilyloxy)silyl]uridine, **6**, (Scaringe, S.A., Kitchen, D., Kaiser, R., Marshall, W.M. (2004), "Preparation of 5'-Silyl-2'-Orthoester Ribonucleosides for Use in Oligoribonucleotide Synthesis," in: Current Protocols in Nucleic Acid Chemistry, edited by Beaucage, S.L., vol. 1. pp. 2.10.11-15. New York: John Wiley & Sons, Inc.), (10.2 g, 15.7 mmol), and TEA (21.8 mL g, 157 mmol) in dichloromethane [DCM] (100 mL) is treated with acetic anhydride [Ac₂O] (7.4 mL, 78.7 mmol) for 16 hours at room temperature. The reaction mixture is diluted with DCM (400 mL) and washed two times with saturated aqueous sodium bicarbonate and once with saturated aqueous sodium chloride. The DCM solution is dried with anhydrous sodium sulfate and evaporated to obtain a paste that is purified by flash chromatography (300 mL silica gel; 90:10 (v:v) hexanes:acetone to 80:20 (v:v) hexanes:acetone) to give 3'-*O-*acetyl-2'-*O*-methyl- 5'-*O*-[benzhydryloxy-bis(trimethylsilyloxy)silyl] uridine (8.5 g).

A solution of N,N,N,N'-tetramethylethylene diamine [TEMED] (7.4 mL, 49.1 mmol) in acetonitrile (55 mL) is cooled to 0 °C and 48% hydrofluoric acid (0.59 mL, 16.3 mmol) is added dropwise. This solution is allowed to stir for 5 minutes and is then added to the crude product from above at room temperature. The reaction is stirred for 30 minutes and the solution is then evaporated to dryness. The crude product is purified by flash chromatography (250 mL silica gel; 15:85 (v:v) hexanes:ethyl acetate containing 0.1% (v/v) TEMED to 98:2 (v:v) ethyl acetate:methanol containing 0.1% (v/v) TEMED) to afford **7** (3.2 g, 98%).

### 3'-O-acetyl-2'-O-methyl-5'-iodouridine (8):

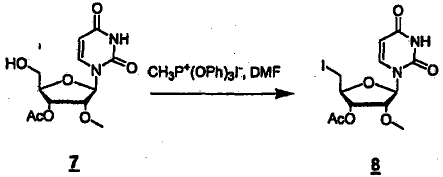

Methyltriphenoxyphosphonium iodide (4.4 g, 9.7 mmol) is added to a stirred solution of compound **7** (2.0 g, 6.5 mmol) in DMF (33 mL) (Verheyden, J. P. H., Moffat, J. G. (1970), "Halo Sugar Nucleosides. I. Iodination of the Primary Hydroxyl Groups of Nucleosides with Methyltriphenoxyphosphonium Iodide," J. Org. Chem. 35, 2319-2326). After 1.25 hours the reaction is stopped by the addition of methanol and evaporated to dryness. The resulting brown paste is dissolved in DCM (100 mL) and washed with saturated aqueous sodium thiosulfate followed by water. The combined aqueous phases are extracted with dichloromethane. The combined organic layers are dried with anhydrous sodium sulfate and evaporated to dryness. The crude material was purified by flash chromatography (100 mL silica gel; 25:75 (v:v) ethyl acetate:hexanes to 50:50(v:v) ethyl acetate:hexanes to give **8** (2.4 g, 91%).

### 2'-O-methyl-5'-deoxyuridine (9):

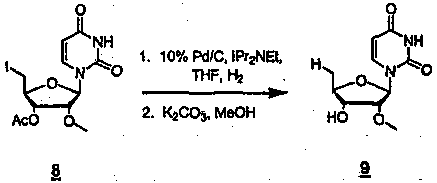

Compound **8** (2.4 g, 5.9 mmol) is dissolved in a solution of THF (24 mL) and DIEA (0.84 mL, 4.8 mmol). Pd on carbon (10% (w/w); 1.0 g) is added, and the mixture is stirred under hydrogen at atmospheric pressure for 16 hours at room temperature. The suspension is filtered through a pad of Celite in a glass-fritted funnel. The solid is washed well with ethyl acetate then methanol, and the filtrate is evaporated to dryness. The resulting powder is dissolved in methanol (50 mL) and anhydrous potassium carbonate (2.0 g, 14.5 mmol) is added. After 2 hours the reaction is neutralized with solid ammonium chloride. The mixture is evaporated to dryness. The crude product is dissolved in DCM (200 mL) and the solution washed with water. The aqueous phase is extracted with 10:90 (v:v) 2-propanol:DCM. The organic extracts are combined, dried over anhydrous sodium sulfate, and evaporated to dryness to give **9** (0.8 g, 53%) that was used without further purification. Phases were passed over Na₂SO₄ and concentrated to dryness to give **9** (0.75 g, 52.5%) that was used without further purification.

### 2'-O-methyl-5'- deoxyuridine - 3'-methyl-N,N,-diisopropylamino phosphoramidite (10):

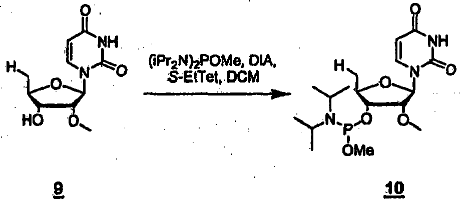

Methyl tetraisopropylphosphorodiamidite(1.2 g, 4.7 mmol) is dissolved in dichloromethane [DCM] (10 mL) and a solution of 5-ethylthio-1*H*-tetrazole in acetonitrile [S-EtTet] (0.45 M; 3.4 mL, 1.6 mmol) is added. Diisopropylamine [DIA] (0.40 mL, 3.1 mmol) is then added and this solution is stirred for 5 minutes at room temperature. In a separate flask compound **9** (0.8 g, 3.1 mmol) and DIA (0.40 mL, 3.1 mmol) are dissolved in DCM (30 mL). The phosphitylation solution is poured into the nucleoside solution and the reaction is stirred at room temperature. After 16 hours the reaction is quenched with absolute ethanol (5 mL) and evaporated to dryness. The resulting paste is purified by flash chromatography (50 mL silica gel; 95:5 (v:v) hexanes:dichloromethane containing 1% (v:v) TEA to 70:30 (v:v) hexanes:acetone containing 0.1% (v:v) TEA) to yield compound **10** as a white powder (1.1 g, 87%). ³¹P NMR (CD₃CN) δ 151.48, 151.14.

### 3'-O-acetyl-5'-O-[benzhydryloxy-bis(trimethylsilyloxy)silyl]-2'-deoxyuridine (11):

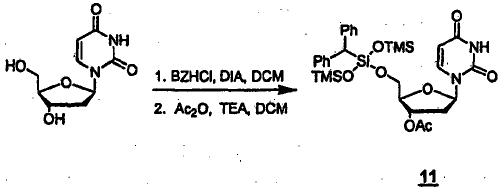

2'-Deoxyuridine (10.0 g, 44.0 mmol) is dissolved DMF (100 mL) and diluted with DCM (100 mL), and DIA (6.2 mL, 44.0 mmol) is added. The stirred solution is cooled in a ice/water bath. In a separate flask, benzhydryloxy-bis(trimethylsilyloxy)silyl chloride [BZHCI] (28.0 g, 66.0 mmol) is dissolved in DCM (56 mL). DIA (11.0 mL, 79.2 mmol) is added dropwise to the solution of silyl chloride over 1 minute. The silylation solution is then added dropwise to the cold deoxyuridine solution and the reaction is kept cold until TLC analysis shows complete consumption of starting material. The reaction is quenched with methanol and the solution is evaporated to dryness. The resulting thick paste is dissolved in DCM (400 mL) and washed with saturated aqueous sodium chloride. The aqueous phase is extracted with DCM and the combined organic layers are dried over anhydrous sodium sulfate. The solution is evaporated to dryness to give predominantly 5'-*O-*[benzhydryloxy-*bis*(trimethylsilyloxy)silyl]-2'-deoxyuridine plus a small amount of 3',5'-*bis*-silylated by-product. The crude material is dissolved in a solution of DCM (240 mL) and TEA (61.0 mL, 440 mmol). N,N-dimethylaminopyridine [DMAP) (1.1 g, 8.8 mmol) and acetic anhydride (20.0 mL, 220 mmol) are added, and the reaction is stirred for 16 hours at room temperature. The solution is then evaporated to dryness and the crude product is purified by flash chromatography (400 mL silica gel; 10:90 (v:v) acetone:hexanes containing 0.1% (v:v) TEA) to give two fractions of material. The first fraction contains 9.3 g of material that is acetylated at both the 3'-hydroxyl and the *O⁴* position of the uracil ring, as well as some 3',5'-bis-silylated 2'-deoxyuridine. The second fraction contains pure **11** (15.5 g, 53%).

### 3'-O-acetyl-2'-deoxyuridine (12):

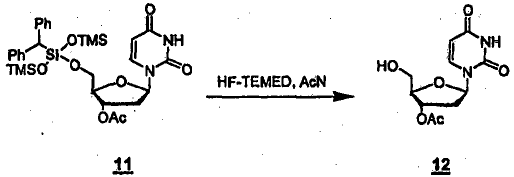

To a solution of TEMED (17.7 mL, 118 mmol) in acetonitrile (50 mL) at 0 ºC is dropwise added 48% hydrofluoric acid (3.0 mL, 82.2 mmol). This solution is allowed to stir for 5 minutes and is then added to **11** (15.5 g, 23.3 mmoles) at room temperature. The reaction is stirred for 2 hours and evaporated to dryness. The crude material is purified by flash chromatography (500 mL silica gel; 30:70 (v:v) hexanes:ethyl acetate containing 0.1% (v:v) TEMED to 10:90 (v:v) methanol:ethyl acetate containing 0.1 % (v:v) TEMED) to give **12** as a colorless oil (5.5 g, 84%).

### 3'-O-acetyl-5'-iodo-2'-deoxyuridine (13):

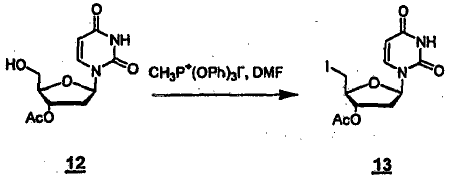

Methyltriphenoxyphosphonium iodide (13.7 g, 30.3 mmol) is added to a stirred solution of **12** (5.5 g, 20.2 mmol) in DMF (100mL) (Verheyden, J. P. H., Moffat, J. G. (1970), "Halo Sugar Nucleosides. I. Iodination of the Primary Hydroxyl Groups of Nucleosides with Methyltriphenoxyphosphonium iodide," J. Org. Chem. 35, 2319-2326). After 2 hours the reaction is stopped by the addition of methanol and evaporated to dryness. The resulting brown paste is dissolved in DCM (250 mL) and washed with saturated aqueous sodium thiosulfate followed by water. The combined aqueous phases are extracted with dichloromethane. The combined organic layers are dried with anhydrous sodium sulfate and evaporated to dryness. The crude material was purified by flash chromatography (200 mL silica gel; 10:90 (v:v) acetone:hexanes to 35:65(v:v) acetone:hexanes to give **13** (2.1 g, 27%).

### 2', 5'-Dideoxyuridine (14):

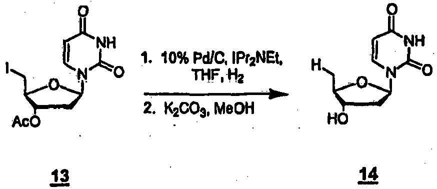

Compound **13** (2.1 g, 5.5 mmol) is dissolved in a solution of THF (110 mL) and DIEA (1.9 mL, 11.0 mmol). Pd on carbon (10% (w/w); 1.1 g) is added, and the mixture is stirred under-hydrogen at atmospheric pressure for 16 hours at room temperature. The suspension is filtered through a pad of Celite in a glass-fritted funnel. The solid is washed well with ethyl acetate then methanol, and the filtrate is evaporated to dryness. The resulting powder is dissolved in methanol (50 mL) and anhydrous potassium carbonate (2.0 g, 14.5 mmol) is added. After 3 hours the reaction is neutralized with solid ammonium chloride and filtered. After evaporation the crude product is purified by column chromatography (100 mL silica gel; ethyl acetate to 10:90 (v:v) methanol:ethyl acetate) to leave **14** a white powder (1.0 g, 87%).

### 2',5'-dideoxyuridine-3'-methyl-N,N,-diisopropylamino phosphoramidite (15):

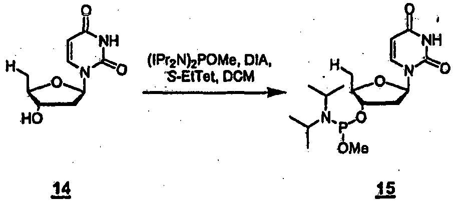

Methyl tetraisopropylphosphorodiamidite (1.9 g, 7.1 mmol) is dissolved in dichloromethane [DCM] (10 mL) and a solution of 5-ethylthio-1*H*-tetrazole in acetonitrile [S-EtTet] (0.45 M; 5.2 mL, 2.4 mmol) is added. DIA (0.66 mL, 4.7 mmol) is then added and this solution is stirred for 5 minutes at room temperature. In a separate flask compound **14** (1.0 g, 4.7 mmol) and DIA (0.66 mL, 4.7 mmol) are dissolved in DCM (30 mL). The phosphitylation solution is poured into the nucleoside solution and the reaction is stirred at room temperature. After 4 hours the reaction is quenched with absolute ethanol (5 mL) and evaporated to dryness. The resulting paste is purified by flash chromatography (50 mL silica gel; 95:5 (v:v) hexanes:dichloromethane containing 1% (v:v) TEA to 70:30 (v:v) hexanes:acetone containing 0.1% (v:v) TEA) to yield compound **15** as a white powder (0.6 g, 34%).

### 3'-O-acetyl-O⁴-acetyl-2',5'-dideoxyuridine (16):

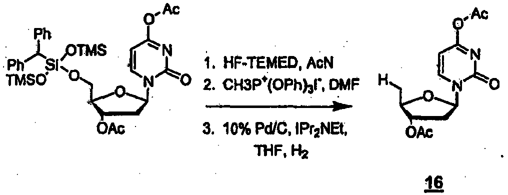

The fraction from synthesis of **11** (9.3 g, 13.3 mmol) that contains *bis-*acetylated material as well as 3',5'-*bis*-silylated-2'-deoxyuridine is desilylated using the procedure set forth above where conversion of **12** to **13** is described. The resulting product is then transformed into **16** using the procedures set forth above where the conversion of **13** to **14** and the conversion of **14** to **15** is described.

### 5-(hexyn-1-ol)-2',5'-dideoxyuridine (17):

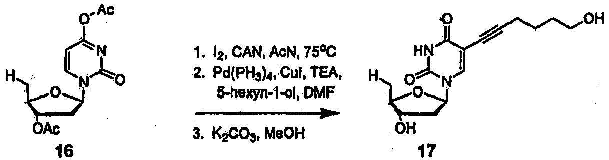

To a solution of **16** (0.9 g, 3.2 mmol) in acetonitrile is added iodine (0.5 g, 1.9 mmol) and ceric ammonium nitrate (0.9 g, 1.6 mmol) (Asakura, J., Robins, M. J. (1990), "Cerium (IV)-Mediated Halogenation at C-5 of Uracil Derivatives," J. Org. Chem., 55, 4928-4933). The mixture is heated to reflux for 1 hour, then cooled to room temperature. The solution is evaporated to dryness and the resulting solids are partitioned between ethyl acetate and saturated aqueous sodium chloride. The layers are separated and the aqueous phase is extracted twice more with ethyl acetate. The combined ethyl acetate extracts are washed with saturated aqueous sodium thiosulfate, dried with anhydrous sodium sulfate and evaporated. The resulting foam is purified by flash chromatography (100 mL silica gel; 20:80 (v:v) ethyl acetate:hexanes to 40:60 (v:v) ethyl acetate:hexanes) to give a mixture of mono- and di-acetylated 5-iodo-2',5'-dideoxyuridine (1.2 g).

The above product (1.2 g, ~2.8 mmol) is dissolved in DMF (30 mL). *Tetrakis*(triphenylphosphine)palladium(0) (0.3 g, 0.3 mmol), copper(I) iodide (0.1 g, 0.6 mmol), TEA (0.78 mL, 5.6 mmol) and 5-hexyn-1-ol (0.8 g, 8.4 mmol) are added and the solution is stirred for 18 hours at room temperature. The reaction mixture is diluted with ethyl acetate (200 mL) and washed with saturated aqueous sodium bicarbonate followed by saturated aqueous sodium chloride. The solution is dried over anhydrous sodium sulfate and evaporated to dryness. The resulting material is purified by flash chromatography (100 mL silica gel; 25:75 (v:v) ethyl acetate:hexanes to ethyl acetate) to give a complex mixture of products (1.4 g).

The crude material from the above reaction is treated with anhydrous potassium carbonate (0.8 g, 5.4 mmol) in methanol (50 mL) for 4 hours at room temperature. The reaction is carefully quenched with glacial acetic acid (1 mL) and evaporated to dryness. The resulting oil is purified by flash chromatography (50 mL silica gel; 50:50 (v:v) ethyl acetate:hexanes to 5:95 (v:v) methanol:ethyl acetate) to give **17** (0.4 g, 45% based on starting **16**) as a colorless oil containing a small impurity.

### 5-[hexyn-1-(benzhydryloxy-bis(trimethylsilyloxy)silyl)]- 2',5'-dideoxyuridine (18):

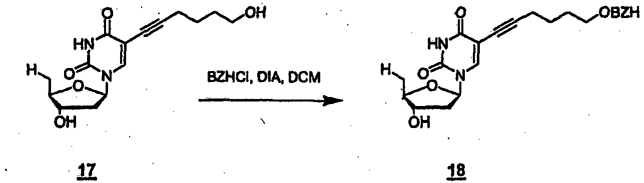

Compound **17** (0.4 g, 1.4 mmol) is dissolved in DMF (1 niL) and the solution is diluted with DCM (9 mL). DIA (0.2 mL, 1.4 mmol) is added and the reaction mixture is cooled to 0 ºC. In a separate flask, BZHCl (1.2 g, 2.8 mmol) is dissolved in DCM (3 mL). DIA (0.5 mL, 3.4 mmol) is added dropwise to the solution of silyl chloride over 1 minute. The silylation solution is added slowly to the cold nucleoside solution and the reaction is kept cold until TLC analysis shows complete consumption of starting material. The reaction is quenched with methanol and the solution is evaporated to dryness and the crude product is purified by flash chromatography (70 mL silica gel; 20:80 (v:v) acetone:hexanes containing 0.1% (v:v) TEA to 20:60:20 (v:v:v) acetone:hexanes:ethyl acetate) to give **18** (0.3 g, 33%) as a colorless oil.

### 5-[hexyn-1-(benzhydryloxy-bis(trimethylsilyloxy)siyl)]-2',5'-dideoxyuridine-3'-methyl-N,N,-diisopropylamino phosphoramidite (19):

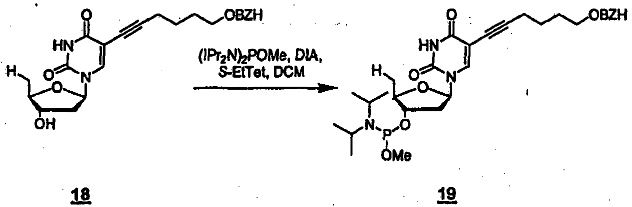

Methyl tetraisopropylphosphorodiamidite (0.2 g, 0.7 mmol) is dissolved in DCM (2 mL) and a solution of 5-ethylthio-1*H*-tetrazole in acetonitrile {S-EtTet] (0.45 M; 0.5 mL, 0.2 mmol) is added. DIA (0.07 mL, 0.5 mmol) is then added and this solution is stirred for 5 minutes at room temperature. In a separate flask compound **18** (0.3 g, 0.5 mmol) and DIA (0.07 mL, 0.5 mmol) are dissolved in DCM (3 mL). The phosphitylation solution is added to the nucleoside solution and the reaction is stirred at room temperature. After 16 hours the reaction is quenched with absolute ethanol (1 mL) and evaporated to dryness. The resulting oil is purified by flash chromatography (40 mL silica gel; 95:5 (v:v) hexanes:dichloromethane containing 1% (v:v) TEA to 80:20 (v:v) hexanes:acetone containing 0.1 % (v:v) TEA) to yield compound **19** as a white powder (0.4 g, 98%).

### 2'-O-bis(2-acetoxyethoxy)methyl-3'-O-t-butydimethylsilyl-N²-isobutyrylguanosine (21):

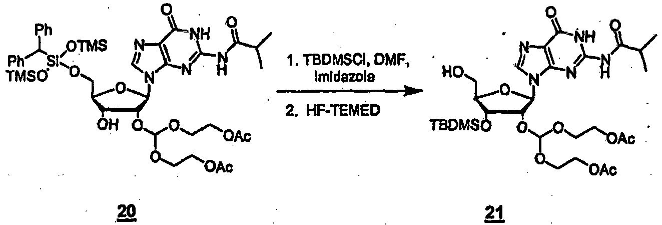

2'-*O*-bis(2-acetoxyethoxy)methyl-5'-*O*-[benzhydryloxy-*bis*(trimethylsilyloxy)silyl]-*N*²-isobutyrylguanosine, **20**, (Scaringe, S.A., Kitchen, D., Kaiser, R., Marshall, W.M. (2004), "Preparation of 5'-Silyl-2'-Orthoester Ribonucleosides for Use in Oligoribonucleotide Synthesis," in: Current Protocols in Nucleic Acid Chemistry, edited by Beaucage, S.L., vol. 1. pp. 2.10.11-15. New York: John Wiley & Sons, Inc.), (9.7 g, 10.1 mmol) and imidazole (2.1 g, 30.3 mmol) are dissolved in DMF (35 mL), and TBDMSC1 (2.3 g, 15.2 mmol) is added. The solution is stirred for 24 hours at room temperature and is then diluted with water (100 mL). The aqueous solution is extracted with ether (3 x 100mL) and the combined organic layers are washed with water then saturated aqueous sodium chloride. The combined organic layers are dried with anhydrous sodium sulfate and evaporated to dryness to give a white foam.

To a solution of TEMED (4.6 mL, 30.3 mmol) in acetonitrile (100 mL) at 0 ºC is dropwise added 48% hydrofluoric acid (0.4 mL, 11.1 mmol). This solution is allowed to stir for 5 minutes and is then added to the product of the previous reaction at room temperature. The reaction is stirred for 1 hour and evaporated to dryness. The crude material is purified by flash chromatography (300 mL silica gel; 20:80 (v:v) hexanes:ethyl acetate containing 0.1% (v:v) TEMED to 2:98 (v:v) methanol:ethyl acetate containing 0.1% (v:v) TEMED) to give **21** as a white foam (5.5 g, 80%).

### 2'-O-bis(2-acetoxyethoxy)methyl-3'-O-t-butydimethylsilyl-5'-iodo-N²-isobutyrylguanosine (22):

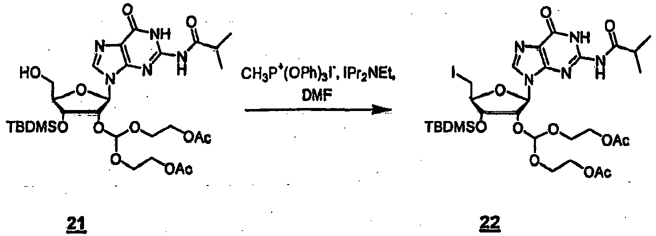

Methyltriphenoxyphosphonium iodide (5.4 g, 12.0 mmol) is added to a stirred solution of **21** (5.5 g, 8.0 mmol) in DMF (40 mL) and DIEA (3.5 mL, 20.0 mmol) (Verheyden, J. P. H., Moffat, J. G. (1970), "Halo Sugar Nucleosides. L Iodination of the Primary Hydroxyl Groups of Nucleosides with Methyltriphenoxyphosphonium Iodide," J. Org. Chem. 35, 2319-2326). After 1 hour the reaction is stopped by the addition of methanol and evaporated to dryness. The resulting brown paste is dissolved in DCM (250 mL) and washed with saturated aqueous sodium thiosulfate followed by water. The combined aqueous phases are extracted with dichloromethane. The combined organic layers are dried with anhydrous sodium sulfate and evaporated to dryness. The crude material was purified by flash chromatography (400 mL silica gel; 25:75 (v:v) ethyl acetate:DCM containing 0.1 % (v:v) TEA to 40:60 (v:v) ethyl acetate:DCM containing 0.1 % (v:v) TEA) to give **22** (3.1 g, 49%).

### 2'-O-bis(2-acetoxyethoxy)methyl-5'-deoxy-N²-isobutyrylguanosine (23):

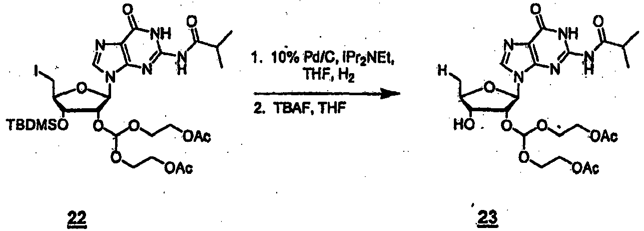

Compound **22** (3.1 g, 4.0 mmol) is dissolved in a solution of THF (80 mL) and DIEA (1.4 mL, 7.9 mmol). Pd on carbon (10% (w/w); 1.3 g) is added, and the mixture is stirred under hydrogen at atmospheric pressure for 5 hours at room temperature. The suspension is filtered through a pad of Celite in a glass-fritted. funnel. The solid is washed well with ethyl acetate then methanol, and the filtrate is evaporated to dryness. The resulting powder is dissolved in THF (40 mL) and TBAF (2.1 g, 7.9 mmol) is added. After 24 hours the solution is evaporated to dryness and the crude product is purified by flash chromatography (200 mL silica gel; 1:99 (v:v) methanol:DCM containing 0.1% (v:v) TEA to 5:95 (v:v) methanol:DCM containing 0.1% (v:v) TEA) to give **23** (1.8 g, 83%).

### 2'-O-bis(2-acetoxyethoxy)methyl-5'-deoxy-N²-isobutyrylguanosine 3'-methyl-N,N,-diisopropylamino phosphoramidite (24):

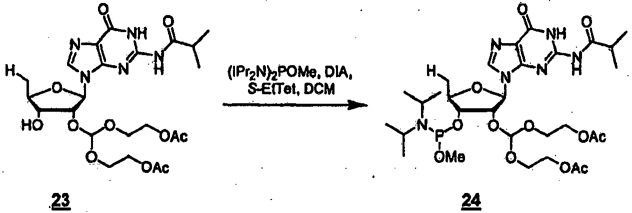

Methyl tetraisopropylphosphorodiamidite (1.6 g, 5.9 mmol) is dissolved in DCM (10 mL) and a solution of 5-ethylthio-1*H*-tetrazole in acetonitrile [S-EtTet] (0.45 M; 4.4 mL, 2.0 mmol) is added. DIA (0.55 mL, 4.0 mmol) is then added and this solution is stirred for 5 minutes at room temperature. In a separate flask compound **23** (1.8 g, 4.0 mmol) and DIA (0.55 mL, 4.0 mmol) are dissolved in DCM (30 mL). The phosphitylation solution is added to the nucleoside solution and the reaction is stirred at room temperature. After 14 hours the reaction is quenched with absolute ethanol (5 mL) and evaporated to dryness. The resulting paste is purified by flash chromatography (120 mL silica gel; 95:5 (v:v) hexanes:dichloromethane containing 1 % (v:v) TEA to 60:40 (v:v) hexanes:acetone containing 0.1% (v:v) TEA) to yield compound **24** as a colorless oil (1.8 g, 64%).

### 2'-O-methyl-3'-O-t-butydimethylsilyl-N²-isobutyrylguanosine (26):

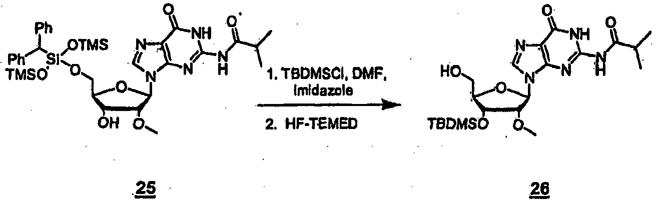

A solution of 2'-*O*-methyl-5'-*O*-[benzbydryloxy-bis(trimethylsilyloxy)silyl]-*N*²-isobutyrylguanosine, **25**, (Scaringe, S.A., Kitchen, D., Kaiser, R., Marshall, W.M. (2004), "Preparation of 5'-Silyl-2'-Orthoester Ribonucleosides for Use in Oligoribonucleotide Synthesis," in: Current Protocols in Nucleic Acid Chemistry, edited by Beaucage, S.L., vol. 1. pp. 2.10.11-15. New York: John Wiley & Sons, Inc.), (6.4 g, 8.5 mmol) and imidazole (1.7 g, 25.5 mmol) in DMF (25 mL) and TBDMSC1 (1.9 g, 12.8 mmol) is added. The solution is stirred for 24 hours at room temperature and is then evaporated to dryness. The resulting paste is dissolved in DCM (100 mL) and washed with saturated aqueous sodium chloride. The aqueous layer is then extracted with DCM. The combined organic layers are dried with anhydrous sodium sulfate and evaporated to dryness to give a white foam.

To a solution of TEMED (3.8 mL, 25.5 mmol) in acetonitrile (85 mL) at 0 ºC is dropwise added 48% hydrofluoric acid (0.4 mL, 11.1 mmol). This solution is allowed to stir for 5 minutes and is then added to the product of the previous reaction at room temperature. The reaction is stirred for 30 minutes and evaporated to dryness. The crude material is purified by flash chromatography (200 mL silica gel; ethyl acetate containing 0.1% (v:v) TEMED to 2:98 (v:v) methanol:ethyl acetate containing 0.1% (v:v) TEMED) to give **26** as a white foam (3.3 g, 81%).

### 2'-O-methyl-3'-O-t-butydimethylsilyl-5'-iodo-N²-isobutyrylguanosine (27):

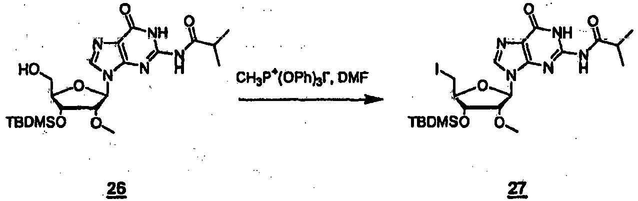

Methyltriphenoxyphosphonium iodide (4.7 g, 10.3 mmol) is added to a stirred solution of compound **26** (3.3 g, 6.9 mmol) in DMF (34 mL) (Verheyden, J. P. H., Moffat, J. G. (1970), "Halo Sugar Nucleosides. I. Iodination of the Primary Hydroxyl Groups of Nucleosides with Methyltriphenoxyphosphonium Iodide," J. Org. Chem. 35, 2319-2326). After 1 hour the reaction is stopped by the addition of methanol and evaporated to dryness. The resulting brown paste is dissolved in DCM (250 mL) and washed with saturated aqueous sodium thiosulfate followed by water. The combined aqueous phases are extracted with dichloromethane. The combined organic layers are dried with anhydrous sodium sulfate and evaporated to dryness. The crude material was purified by flash chromatography (150 mL silica gel; 50:50 (v:v) ethyl acetate:hexanes to 75:25 (v:v) ethyl acetate:hexanes) to give **27** (3.0 g, 75%).

### 2'-O-methyl-5'-deoxy-N²-isobutyrylguanosine (28):

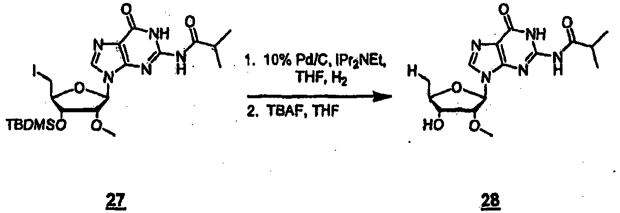

Compound **27** (3.0 g, 5.1 mmol) is dissolved in a solution of THF (102 mL) and DIEA (1.8 mL, 10.2 mmol). Pd on carbon (10% (w/w); 1.2 g) is added, and the mixture is stirred under hydrogen at atmospheric pressure for 16 hours at room temperature. The suspension is filtered through a pad of Celite in a glass-fritted funnel. The solid is washed well with ethyl acetate then methanol, and the filtrate is evaporated to dryness. The resulting powder is dissolved in THF (51 mL) and TBAF (2.0 g, 7.7 mmol) is added. After 24 hours the solution is evaporated to dryness and the crude product is purified by flash chromatography (200 mL silica gel; ethyl acetate to 6:94 (v:v) methanol:ethyl acetate) to give **28** (2.2 g) contaminated with residual TBAF salts.

### 2'-O-methyl-5'-deoxy-N²-isobutyrylguanosine 3'-methyl-N,N,-diisopropylamino phosphoramidite (29):

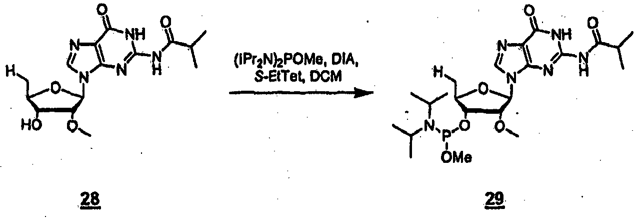

Methyl tetraisopropylphosphorodiamidite (2.0 g, 7.7 mmol) is dissolved in DCM (10 mL) and a solution of 5-ethylthio-1H-tetrazole in acetonitrile [S-EtTet] (0.45 M; 5.8 mL, 2.6 mmol) is added. DIA (0.71 mL, 5.1 mmol) is then added and this solution is stirred for 5 minutes at room temperature. In a separate flask compound 28 (2.2 g, 5.1 mmol) and DTA (0.71 mL, 5.1 mmol) are dissolved in DCM (40 mL). The phosphitylation solution is added to the nucleoside solution and the reaction is stirred at room temperature. After 14 hours the reaction is quenched with absolute ethanol (5 mL) and evaporated to dryness. The resulting paste is purified by flash chromatography (120 mL silica gel; 95:5 (v:v) hexanes:dichloromethane containing 1% (v:v) TEA to 60:40 (v:v) hexanes:acetone containing 0.1% (v:v) TEA) to yield compound **29** as a colorless oil (1.6 g, 62%).

### 2'-O-bis(2-acetoxyethoxy)methyl- 3'-O-t-butydimethylsilyl-N⁶-isobutyryladenosine (31):

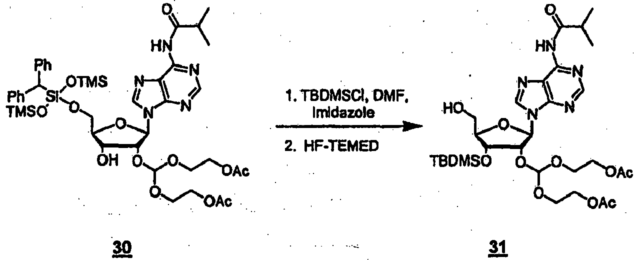

2'-*O*-bis(2-acetoxyethoxy)methyl-5'-*O*-(benzhydryloxy-*bis*(timethylsilyloxy)silyl]-*N*⁶-isobutyryladenosine, **30**, (Scaringe, S.A., Kitchen, D., Kaiser, R., Marshall, W.M. (2004), "Preparation of 5'-Silyl-2'-Orthoester Ribonucleosides for Use in Oligoribonucleotide Synthesis," in: Current Protocols in Nucleic Acid Chemistry, edited by Beaucage, S.L., vol. 1. pp. 2.10.11-15. New York: John Wiley & Sons, Inc.), (8.7 g, 9.2 mmol) and imidazole (1.9 g, 27.6 mmol) are dissolved in DMF (31 mL), and TBDMSCl (2.1 g, 13.8 mmol) is added. The solution is stirred for 24 hours at room temperature and is then is then diluted with water (100 mL). The aqueous solution is extracted with ether (3 x 200mL) and the combined organic layers are washed with water then saturated aqueous sodium chloride. The organic extract is dried with anhydrous sodium sulfate and evaporated to dryness to give a white foam.

To a solution of TEMED (4.6 mL, 31.2 mmol) in acetonitrile (100 mL) at 0 ºC is dropwise added 48% hydrofluoric acid (0.3 mL,11.4 mmol). This solution is allowed to stir for 5 minutes and is then added to the product of the previous reaction at room temperature. The reaction is stirred for 1 hour and evaporated to dryness. The crude material is purified by flash chromatography (300 mL silica gel; 50:50 (v:v) ethyl acetate:hexanes containing 0.1% (v:v) TEMED to 80:20 (v:v) ethyl acetate:hexanes containing 0.1% (v:v) TEMED) to give **31** as a white foam (5.5 g, 90%).

### 2'-O-bis(2-acetoxyethoxy)methyl- 3'-O-t-butydimethylsilyl-5'-iodo-N⁶-isobutyryladenosine (32):

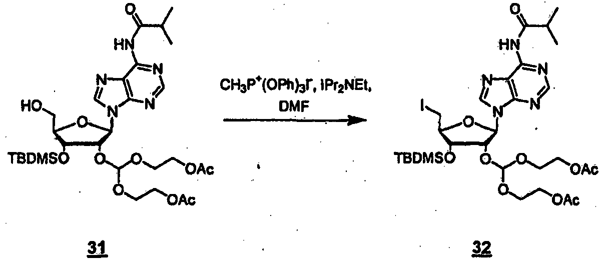

Methyltriphenoxyphosphonium iodide (4.68 g, 10.4 mmol) is added to a stirred solution of compound **31** (5.5 g, 8.3 mmol) in DMF (40 mL) and DIEA (3.6 m, 20.7 mmol) (Verheyden, J. P. H., Moffat, J. G. (1970), "Halo Sugar Nucleosides. I. Iodination of the Primary Hydroxyl Groups of Nucleosides with Methyltriphenoxyphosphonium Iodide," J. Org. Chem. 35, 2319-2326). After 1.5 hours the reaction is stopped by the addition of methanol and evaporated to dryness. The resulting brown paste is dissolved in DCM (250 mL) and washed with saturated aqueous sodium thiosulfate followed by water. The combined aqueous phases are extracted with dichloromethane. The combined organic layers are dried with anhydrous sodium sulfate and evaporated to dryness. The crude material was purified by flash chromatography (300 mL silica gel; 25:75 (v:v) ethyl acetate:DCM containing 0.1 % (v:v) TEA to 75:25 (v:v) ethyl acetate:DCM containing 0.1% (v:v) TEA) to give **32** (4.1 g, 62%).

### 2'-O-bis(2-acetoxyethoxy)methyl-3'-deoxy-N⁶-isobutyryladenosine (33):

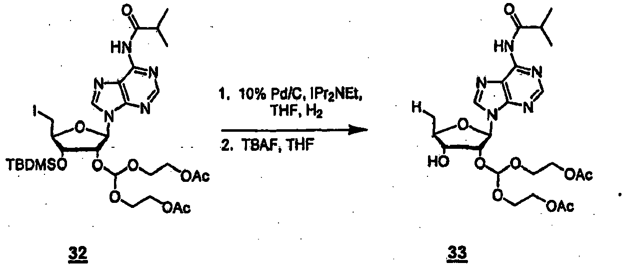

Compound **32** (4.1 g, 5.2 mmol) is dissolved in a solution of THF (100 mL) and DIEA (1.8 mL, 10.2 mmol). Pd on carbon (10% (w/w); 1.6 g) is added, and the mixture is stirred under hydrogen at atmospheric pressure for 16 hours at room temperature. The suspension is filtered through a pad of Celite in a glass-fritted funnel. The solid is washed well with ethyl acetate then methanol, and the filtrate is evaporated to dryness. The resulting powder is dissolved in THF (26 mL) and TBAF (2.7 g, 10.3 mmol) is added. After 24 hours the solution is evaporated to dryness and the crude product is purified by flash chromatography (100 mL silica gel; 50:50 (v:v) ethyl acetate:hexanes containing 0.1% (v:v) TEA to 3:97 (v:v) methanol:ethyl acetate containing 0.1% (v:v) TEA) to give **33** (2.9 g) contaminated with residual TBAF salts.

### 2'-O-bis(2-acetoxyethoxy)methyl-5'-deoxy-N⁶-isobutyryladenosine 3'-methyl-N,N,-diisopropylamino phosphoramidite (34):

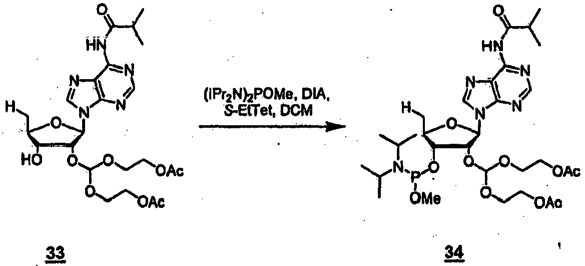

Methyl tetraisopropylphosphorodiamidite (1.7 g, 6.4 mmol) is dissolved in DCM (10 mL) and a solution of 5-ethylthio-1*H*-tetrazole in acetonitrile [S-EtTet] (0.45 M; 5.7 mL, 2.6 mmol) is added. DIA (0.72 mL, 5.2 mmol) is then added and this solution is stirred for 5 minutes at room temperature. In a separate flask compound **23** (2.9 g, 5.2 mmol) and DIA (0.72 mL, 5.2 mmol) are dissolved in DCM (10 mL). The phosphitylation solution is added to the nucleoside solution and the reaction is stirred at room temperature. After 14 hours the reaction is quenched with absolute ethanol (5 mL) and evaporated to dryness. The resulting paste is purified by flash chromatography (50 mL silica gel; 95:5 (v:v) hexanes:dichloromethane containing 1% (v:v) TEA to 60:40 (v:v) hexanes:acetone containing 0.1% (v:v) TEA) to yield compound **34** as a colorless oil (3.0 g, 82%).

### 2'-O-bis(2-acetoxyethoxy)methyl- 3'-O-t-butydimethylsilyl-N⁴-acetylcytidine (36):

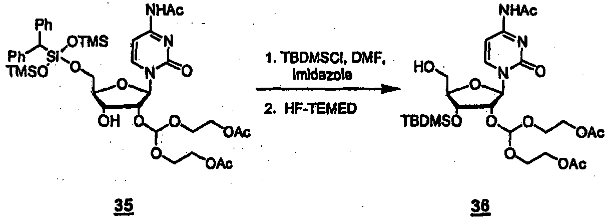

2'-*O*-*bis*(2-acetoxyethoxy)methyl-5-*O*-[benzhydryloxy-*bis*(trimethylsilyloxy)silyl]-*N*⁴-acetylcytidine, **35**, (Scaringe, S.A., Kitchen, D., Kaiser, R., Marshall, W.M. (2004), "Preparation of 5'-Silyl-2'-Orthoester Ribonucleosides for Use in Oligoribonucleotide Synthesis," in: Current Protocols in Nucleic Acid Chemistry, edited by Beaucage, S.L., vol. 1. pp. 2.10.11-15. New York: John Wiley & Sons, Inc.), (10.4 g, 11.3 mmol) and imidazole (2.3 g, 33.9 mmol) are dissolved in DMF (31 mL), and TBDMSCl (2.1 g, 13.8 mmol) is added. The solution is stirred for 24 hours at room temperature and is then is then diluted with water (100 mL). The aqueous solution is extracted with ether (3 x 200mL) and the combined organic layers are washed with water then saturated aqueous sodium chloride. The organic extract is dried with anhydrous sodium sulfate and evaporated to dryness to give a white foam.

To a solution of TEMED (5.4 mL, 36.3 mmol) in acetonitrile (120 mL) at 0 ºC is dropwise added 48% hydrofluoric acid (0.4 mL, 13.2 mmol). This solution is allowed to stir for 5 minutes and is then added to the product of the previous reaction at room temperature. The reaction is stirred for 1 hour and evaporated to dryness. The crude material is purified by flash chromatography (300 mL silica gel; 50:50 (v:v) ethyl acetate:hexanes containing 0.1% (v:v) TEMED to 80:20 (v:v) ethyl acetate:hexanes containing 0.1 % (v:v) TEMED) to give **36** as a white foam (6.6 g, 90%).

### 2'-O-bis(2-acetoxyethoxy)methyl- 3'-O-t-butydimethylsilyl-5'-iodo-N⁴-acetylcytidine (37):

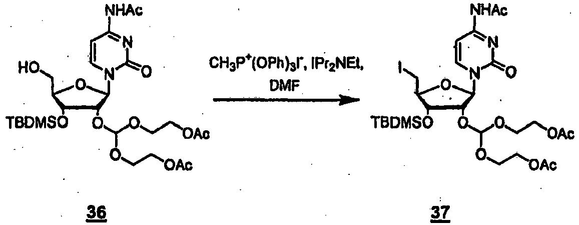

Methyltriphenoxyphosphonium iodide (5.8 g, 12.7mmol) is added to a stirred solution of compound **37** (6.6 g, 10.2 mmol) in DMF (40 mL) and DIEA (4.4 mL, 25.4 mmol) (Verheyden, J. P. H., Moffat, J. G. (1970), "Halo Sugar Nucleosides. I. Iodination of the Primary Hydroxyl Groups of Nucleosides with Methyltriphenoxyphosphonium Iodide," J. Org. Chem. 35, 2319-2326). After 1.5 hours the reaction is stopped by the addition of methanol and evaporated to dryness. The resulting brown paste is dissolved in DCM (250 mL) and washed with saturated aqueous sodium thiosulfate followed by water. The combined aqueous phases are extracted with dichloromethane. The combined organic layers are dried with anhydrous sodium sulfate and evaporated to dryness. The crude material was purified by flash chromatography (300 mL silica gel; 50:50 (v:v) ethyl acetate:hexanes containing 0.1% (v:v) TEA to ethyl acetate containing 0.1% (v:v) TEA) to give **37** (6.8 g, 88%).

### 2'-O-bis(2-acetoxyethoxy)methyl-5'-deoxy-N⁴acetylcytidine (38):

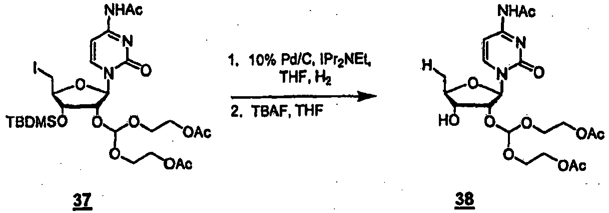

Compound **37** (6.8 g, 9.0 mmol) is dissolved in a solution of THF (200 mL) and DIEA (3.9 mL, 22.6 mmol). Pd on carbon (10% (w/w); 2.7 g) is added, and the mixture is stirred under hydrogen at atmospheric pressure for 16 hours at room temperature. The suspension is filtered through a pad of Celite in a glass-fritted funnel. The solid is washed well with ethyl acetate then methanol, and the filtrate is evaporated to dryness. The resulting powder is dissolved in THF (35 mL) and TBAF (3.7 g, 14.0 mmol) is added. After 24 hours the solution is evaporated to dryness and the crude product is purified by flash chromatography (100 mL silica gel; 75:25 (v:v) ethyl acetate:hexanes containing 0.1 % (v:v) TEA to 3:97 (v:v) methanol:ethyl acetate containing 0.1% (v:v) TEA) to give **38** (3.7 g, 77%).

### 2'-O-bis(2-acetoxyethoxy)methyl-5'-deoxy-N⁴-acetyloykidine-3'-methyl-N,N,-diisopropylamino phosphoramidite (39):

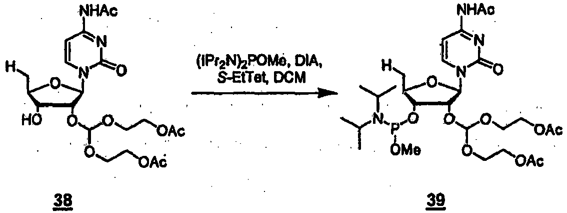

Methyl tetraisopropylphosphorodiamidite (2.7 g, 10.4 mmol) is dissolved in DCM (15 mL) and a solution of 5-ethylthio-1*H-*tetrazole in acetonitrile [S-EtTet] (0.45 M; 7.7 mL, 3.5 mmol) is added. DIA (0.97 mL, 6.9 mmol) is then added and this solution is stirred for 5 minutes at room temperature. In a separate flask compound **38** (3.7 g, 6.9 mmol) and DIA (0.97 mL, 6.9 mmol) are dissolved in DCM (15 mL). The phosphitylation solution is added to the nucleoside solution and the reaction is stirred at room temperature. After 14 hours the reaction is quenched with absolute ethanol (5 mL) and evaporated to dryness. The resulting paste is purified by flash chromatography (100 mL silica gel; 95:5 (v:v) hexanes:dichloromethane containing 1% (v:v) TEA to 70:30 (v:v) hexanes:acetone containing 0.1% (v:v) TEA) to yield compound **39** as a colorless oil (3.4 g, 72%).

### EXAMPLE III

### MOLECULE 1 MODIFICATIONS AND STABILITY

To assess the effects of molecule 1 modifications on siRNA stability, four unique siRNA were synthesized in modified and Unmodified forms. Subsequently, these molecules were incubated in 100% serum at 37°C for varying periods of time and then analyzed by PAGE to assess the intactness of the duplexes. Visualization of sequences was accomplished by ethidium bromide staining.

The results of these experiments are illustrated in **Figures la** and **b** and show that duplexes carrying molecule 1 modifications are drastically more stable than unmodified equivalents. Unmodified molecules typically exhibit 50% degradation or greater within two minutes of being exposed to serum at room temperature. In contrast, the half-life for sequences carrying molecule 1 modifications typically ran between 125 and 135 hours. Thus, molecule 1 modifications significantly enhanced stability by approximately 500-fold.

A further test of the value of these modifications to stabilize siRNA was performed. In **Figure 1c**, five additional siRNA including DBI67, DBI78, Luc4, Luc49, and Luc 51 were first examined for stability in 100% human serum. The sequences associated with these studies are listed below.

| **TABLE I** | | |
|---|---|---|
| **SEQUENCES FOR TESTING MOLECULE I MODIFICATIONS ON STABILITY** | | |
| **SEQUENCE NAME** | **SEQUENCE (SENSE, 5'**→**3')** | **SEQ. ID NO.** |
| DBI 67 | gcuuacauca acaaaguag | 8 |
| DBI 78 | caaaguagaa gagcuaaag | 9 |
| LUC4 | agagagaucc ucauaaagg | 10 |
| LUC49 | guaacaaccg cgaaaaagu | 11 |
| LUC51 | ucaaguaaca accgcgaaa | 12 |

As was observed previously, the half-life of siRNA in the presence of serum is generally less than ten minutes. Addition of the molecule 1 modification pattern to siRNA dramatically enhanced the stability of these (and additional) duplexes. (see table below for list of additional sequences).

| **TABLE II** | | |
|---|---|---|
| **ADDITIONAL SEQUENCES FOR TESTING MOLECULE 1 MODIFICATIONS** | | |
| **SEQUENCE NAME** | **SEQUENCE (SENSE, 5'→3')** | **SEQ. ID NO.** |
| CYCLO 71 | aaagagcauc uacggugag | 13 |
| LUC 5 | ucagagagau ccucauaaa | 14 |
| LUC72 | aaagacgaug acggaaaaa | 15 |
| CYCLO2 | uccaaaaaca guggauaau | 16 |
| CYCLO 77 | cggugagcgc uuccccgau | 17 |
| CYCLO 11 | uuuuguggcc uuagcuaca | 18 |
| CYCLO 28 | ggcuacaaaa acagcaaau | 19 |
| RASSF1A-284 | uuugcggucg ccgucguug | 20 |
| RASSF1A-240 | aggggacgaa ggagggaag | 21 |
| FLUC893 | gcacucugau ugacaaaua | 22 |
| FLUC1313 | ugaagucucu gauuaagua | 23 |
| FLUC206 | gauaugggcu gaauacaaa | 24 |
| HCYCLO SS1 | gaaagagcau cuacgguga | 25 |
| HCYCLO SS2 | gaaaggauuu ggcuacaaa | 26 |
| HCYCLO SS2 | acagcaaauu ccaucgugu | 27 |
| HCYCLO SS4 | ggaaagacug uuccaaaaa | 28 |

As shown in **Figures 1d** and **1e**, half life typically increased to greater than 20 hours, and frequently was greater than 80 hours. These studies again support the conclusions that addition of molecule 1 modification patterns to siRNA greatly enhances duplex stability.

### EXAMPLE IV

### MOLECULE 1 MODIFICATIONS AND siRNA SILENCING POTENCY

To assess the effects of molecule 1 modifications on siRNA potency, two unique siRNA directed against human Cyclophilin B (U1 and U3) were synthesized in modified and unmodified forms using 2'-O-ACE chemistry and tested for functionality in a whole cell assay. Briefly, modified and umnodified siRNA were transfected (Lipofectamine 2000) into HeLa cells (10,000 cells/well, 96 well plate) at concentrations between 0.01-200 nM and cultured for 24-48 hours. Subsequently, the level of expression of the intended target was assessed using a branched DNA assay (Genospectra, Fremont, CA).

Results of these experiments are illustrated in **Figure 2** and show that duplexes carrying molecule 1 modifications perform comparably with unmodified siRNA at all concentrations tested. Thus, molecule 1 modifications do not appear to alter the potency of siRNA.

### ExAMPLE V

### MOLECULE 1 MODIFICATIONS AND SILENCING LONGEVITY

To determine the effects of molecule 1 modifications on siRNA silencing longevity, siRNA directed against the human cyclophilia B gene were synthesized in the modified and unmodified forms and transfected into HeLa cells (100nM) as previously described. Subsequently, the level of silencing was monitored over the course of 7 days using a branched DNA assay.

An example of the results of these experiments are presented in **Figure 3** and demonstrate that while the level of silencing induced by unmodified molecules depreciates from roughly 70% to 0% over the course of the seven day period, modified duplexes induce >80% functionality throughout the course of the experiment. Thus, siRNA modified with molecule 1 modifications enhance the longevity of silencing induced by these duplexes.

### EXAMPLE VI

### MOLECULE 1 MODIFICATIONS AND TOXICITY

To determine the effects of molecule 1 modifications on siRNA toxicity, 4 siRNA (U1-U4) directed against human cyclophilin B were synthesized in the modified and unmodified forms and transfected into HeLa cells at concentrations that ranged between 0.01-200nM. At t = 48 hours after transfection, Alamar Blue assays were performed to assess the level of cell death within the population. A side-by-side comparison between the modified and unmodified duplexes showed no difference in the level of cell death induced at any of the concentrations tested **(****Figure 4****)**.

### EXAMPLE VII

### MOLECULE 1 MODIFICATIONS AND OFF-TARGET EFFECTS

To assess the effects of molecule 1 modifications on off-target effects, four separate siRNA targeting human Cyclophilin B (U1-U4) were synthesized in both the modified and unmodified forms and transfected into HeLa cells at 100nM concentrations. Subsequently, total RNA from untransfected, transfected (unmodified), and transfected (modified) cells was purified (Qiagen) and labeled with Agilent's Low RNA Input Linear Amp Kit. This material was then hybridized to an Agilent Human 1A (V2) Oligo Microarray containing over 21,000 probes.

A summary of these off-target studies are shown in **Figure 5** and illustrates that modified and unmodified siRNA perform similarly in terms of the numbers of off-targeted genes. Specifically, when off-targeted genes were segregated based on the level of induction or repression compared to wild type gene expression, modified siRNA performed similarly to their unmodified counterparts.

### EXAMPLE VII

### MOLECULE 2 MODIFICATIONS AND SILENCING POTENTIAL

To test the functionality of siRNA containing molecule 2 modifications, Cycle 14 (5'ggccuuagcu acaggagag, sense strand SEQ. ID NO. 29), an siRNA directed against human cyclophilin B, was synthesized with molecule 2 modifications and tested for the ability to silence the intended target. Briefly, Cyclo 14 was synthesized with the appropriate modifications using 2'-O-ACE chemistry. Subsequently, T482 HeLa cells (10,000 cells per well, 96 well plates) were plated, cultured overnight, and transfected (Lipofectamine 2000) with the appropriate duplex at 100nM concentrations. Three days after transfection, the level of mRNA silencing was assessed using a branched DNA assay (Genospectra, Fremont, CA).

Results of these studies are presented in **Figure 6****.** Unmodified Cyclo 14 duplexes typically induce 80-95% silencing. Cyclo 14 duplexes modified with the Cy3 label alone induced roughly 70-80% silencing, and Cyclo14 siRNA carrying molecule 2 modifications induced 80% or better silencing. As similar modification of non-specific sequences induced little or no silencing, addition of molecule 2 modifications has little or no effect on duplex functionality.

### EXAMPLE IX

### THE EFFECTS OF MOLECULE 2 MODIFICATIONS ON TRACKABILITY

To test the usefulness of molecule 2 modifications as a means of assessing transfection efficiencies, Cyclo 14 siRNA were prepared with the aforementioned modifications and visualized by fluorescence microscopy. Specifically, Cyclo14 was synthesized with the appropriate modifications using 2'-O-ACE chemistry. Concomitantly, the same duplexes were synthesized with the Cy3 label (5' sense strand) and a 5' phosphate on the antisense 5' terminus, but without the 2'-O-methyl modifications on positions 1 and 2 of the sense strand, 2'-0-methyl modifications on all Cs and Us of the sense strand, 2'-Fluoro (FI) modifications on all Cs and Us of the antisense strand. Subsequently, T482 HeLa cells (10,000 cells per well, 96 well plates) were plated, cultured overnight, and transfected (Lipofectamine 2000) with the appropriate duplex at 100 nM concentrations. At varying times after transfection, cultures were incubated with Hoechst 33342 (2µg/ml, 20 minutes. 37°C) and then visualized on a Leica DMIL fluorescence microscope using Dapi and Rhodamine filters.

Results of these manipulations indicate a strong Cy3 nuclear and perinuclear stain. A fluorescence micrograph **(****Figure 7a****)** of HeLa cells transfected with Cyclo 14 siRNA carrying molecule 2 modifications shows perinuclear and nuclear stain. Thus, siRNA carrying molecule 2 modifications provide an excellent means of assessing the intracellular position of any given siRNA and can also be used to assess the success of transfection.

### EXAMPLE X

### EFFECTS OF MOLECULE 2 MODIFICATIONS ON STABILITY AND NUCLEAR ACCESS

To test the whether molecule 2 modifications enhanced the intracellular stability of duplexes, Cyclo 14 siRNA that were prepared as described in Example IX were transfected into HeLa cells and observed over the course of seven days **(****Figure 7a****,** **b****).** Addition of the aforementioned modifications significantly enhanced siRNA stability over duplexes modified with Cy3 alone. While both samples exhibit strong staining patterns on Day 2 (48 hours), the Cy3-Cyclo14 transfected cells lose the majority of their stain by day 7. In contrast, cells containing Cyclo14 siRNA carrying molecule 2 modifications retain a strong pattern of staining on day 7. Moreover, unlike Cy3-labeled cyclo 14 duplexes, siRNA carrying molecule 2 modifications also promote nuclear access to the duplex (observable on both day 2 and day 7).

### EXAMPLE XI

### SIRNA CONTAINING THE MOLECULE 3 MODIFICATION PATTERN AS TRANSFECTlON CONTROL REAGENTS AND ENHANCERS OF TARGET SIRNA DELIVERY

Several sets of experiments were performed to test whether siRNA containing molecule 3 modifications could act as bulking/exaequo/non-competitive reagents that fail to enter RNAi. In the first experiment, luciferase targeting siRNA (Luc 18, 63, 56, 81, 8, and 58) containing 2'-O-methyl modifications on positions 1 and 2 of (1) the sense strand, (2) the antisense strand, or (3) both the sense and antisense strand (molecule 3 modifications) were tested for the ability to silence. The sequences of these siRNA are shown below.

| **TABLE III** | | |
|---|---|---|
| **SEQUENCES FOR TESTING MOLECULE 3 MODIFICATIONS** | | |
| **SEQUENCE NAME** | **SEQUENCE (SENSE, 5'**→**3')** | **SEQ. ID NO.** |
| Luc 8 | gaaaaaucag agagauccu | 30 |
| Luc 18 | uaccggaaaa cucgacgca | 31 |
| Luc 56 | acgucgccag ucaaguaac | 32 |
| Luc 58 | gauuacgucg ccagucaag | 33 |
| Luc 63 | agagaucgug gauuacguc | 34 |
| Luc 81 | uguuguuuug gagcacgga | 35 |

The results **(****Figure 8a****)** show that addition of paired 2'-O-methyl groups to positions 1 and 2 of the antisense strand dramatically reduces the ability of this siRNA to silence the target gene. The authors reasoned that if addition of paired 2'-O-methyl groups to positions 1 and 2 of the antisense strand eliminated the ability of this strand to participate in RNAi, then addition of these modifications to both strands would eliminate the ability of either strand to enter RISC. Surprisingly, this was not the case. siRNA containing paired 2'-O-methyl groups on both strands exhibited greater activity than siRNA containing 2'-O-methyl groups on the antisense strand alone. Moreover, the researchers further found that addition of a phosphate group to the 5' end of antisense strand of siRNA containing paired 2'-O-methyl groups on positions 1 and 2 of both strands, further enhanced the molecules activity. Thus, from these studies, it was decided that in order to create an efficient exaequo/ bulking/ non-competitive reagent, phosphate groups could not be present on the molecule. Furthermore, it was decided that addition of 2'-O-methyl groups to positions 1 and 2 of both strands was insufficient.

In the next step in development of an efficient exaequo reagent, the inventors tested whether combination of the 2'-O-methyl modifications with a shorter duplex could reduce the functionality of the molecule. To test this, a competition study was designed whereby the ability of cyclo4 siRNA to silence its intended target was tested in the presence of either (1) Non-specific sequence #4 (NSC4, 19bp, sense strand = 5'- uagcgacuaa acacaucaau u SEQ. ID NO 36), or (2) a 17 mer of NSC4 containing molecule 3 modifications (NSC4-M3-Cy3, sense strand = 5' gcgacuaaac acaucaauu SEQ. ID NO 37). To accomplish this varying amounts of cycle 4 (0.1-100M) were transfected into HeLa cells along with a sufficient amount of the modified (17 bp) or unmodified (19 bp) NSC4 duplex such that the total amount of RNA in the transfection was 100nM. Subsequently, the amount of target (human cyclophilin B) knockdown was measured and compared with the knockdown of a control housekeeping gene (GAPDH) using a BDNA assay.

The results of these experiments are shown in **Figure 8b** and show that in the absence of any competitor siRNA, the cyclo 4 siRNA is an adept silencing reagent at all of the concentrations tested. In the presence of unmodified (19bp)NSC4, the silencing curve for cyclo 4 is dramatically altered, reflecting the fact that (19bp) NSC4 competes with cyclo4 siRNA for access to RISC. Interestingly, when NSC4 is shortened by 2 basepairs and modified to carry molecule 3 modifications, the silencing curve for cyclo 4 is similar to that which is generated when there is no competitive siRNA. This result identifies the 17bp duplex containing the molecule 3 modifications as an excellent exaequo/bulldng/non-competitive reagent that fails to compete with functional siRNAs.

To further test the usefulness of siRNA carrying the molecule 3 modification pattern, cycle 1 (sense strand, 5' gaaagagcau cuacggugau u SEQ. ID NO 38) silencing curves were performed, using either 17bp NSC4 duplexes carrying the molecule 3 modification pattern, or 19bp NSC4 duplexes carrying the modification pattern. Simultaneously, the ability of cyclo1 siRNA to compete with GAPDH 4 siRNA (19 bp, modified, sense, 5' ugguuuacau guuocaauau u SEQ. ID NO 39), and 17bp, modified, sense, 5' guuuacaugu uccaauauu SEQ. ID NO 40) was also assessed. In these experiments, the term "modified" refers to molecule 3 modifications. The competition experiments were performed in a similar fashion as those described previously (*i.*e., dose curve, 100nM total siRNA concentration) with the exception that studies were performed in HBK293 cells (5,000 cells per well) and 0.5 ug of Lipofectamine 2000 was used per well for transfection. BDNAs were performed twenty-four hours after transfection.

The results of these experiments are shown in **Figure 9** and demonstrate the following: in the case of experiments where cycle 1 was combined with either modified NSC4 17 mers or modified NSC4 19 mers, the cycle 1 dose curves in both reactions are nearly identical, suggesting that in this case, the molecules perform similarly. In the case where cyclo 1 is competing with either modified GAPDH 17mers or modified GAPDH 19meres, the modified 17meres are less capable of competing with cyclo 1. These experiments again show the value of this modification pattern in creating exaequo/bulking reagents that fail to compete with functional siRNA.

To further assess the quality of duplexes carrying molecule 3 modifications to act as transfection control reagents (*i.e*., reagents that fail to enter the RNAi pathway), siRNA that were unmodified or contained molecule 3 modifications were transfected into HeLa cells and assessed for changes in the gene expression profiles. Previous microarray studies have demonstrated that each siRNA generates a unique off-target gene expression signature. If siRNA molecule 3 modifications limit the ability of this molecule to enter RISC, the inventors predicted that the expression profile generated by modified to be only a subset of those that are generated by the unmodified counterpart.

To perform these studies, cells were plated in a 96 well plate format (20,000 cells per well) and transfected (Lipofectamine 2000) 24 hours later with cyclo52 siRNA (19bp sequence, sense, 5'-cagggcggag acuucacca), cyclo 52 siRNA with molecule 3 modifications, or 17 base pair cyclo 52 siRNA (sense, 5'-gggcggagac uucacca) containing molecule 3 modifications (100nM concentrations). Subsequently, total RNA was purified from both populations and hybridized to microArrays carrying 22,000 probes designed to genes of the human genome (Agilent A 1 arrays). In these experiments, mock-treated (lipid treated) cells were used as the reference control.

**Figure 10** shows a heatmap that displays all genes that are down regulated by 2-fold or more following transfection of modified or unmodified siRNA. As evident from this study, the expression profile of molecule 3 modified modifications is only a fraction of that generated by unmodified siRNA having the same sequence. Lanes **A** and **B** (unmodified Cyclo52) generate the greatest number of off-targets. This signature is suppressed when Cyclo52 siRNA (19bp) are modified with molecule 3 modification patterns, and even more reduced when Cyclo52 siRNA containing molecule 3 modification patterns are reduced in length by two base pairs (*e.g*., 17 bp duplexes with molecule 3 modification patterns). This result establishes that siRNA having molecule 3 modifications are useful as exaequo/bulking/transfection control reagents.

### EXAMPLE XII

### 5'-DEOXY MODIFIED SIRNA AS EXAEQUO REAGENTS

Previous studies have demonstrated the importance of a 5'phosphate group on the 5' terminus of the antisense strand to generate siRNA-induced silencing by RNAi. The inventors reasoned that given the importance of this position for silencing, construction of siRNA that were modified to eliminate the hydroxyl group that was necessary for phosphorylation might create excellent exaequo/bulking/transfection control reagents.

To test the value of 5'deoxy modified siRNA (molecule 4 modifications) to act as transfection controls, two experiments were performed. In the first, modified and unmodified siRNA targeting GAPDH (GAPDH4, sense strand 5'-uggtruuacau guuccaauau u) were transfected into HeLa cells and tested for the ability to knockdown GAPDH. The inventors predicted that if 5'-deoxy modifications prevented phosphorylation, then addition of these modifications to the termini of the sense and antisense strand should eliminate duplex functionality and create excellent exaequo/bulking/transfection control reagents.

The conditions for this experiment included the following: HeLa cells were plated at a density of 10,000 cells per well and transfected with siRNA (100, 50, 10, 1, and 0.1 nM) using Lipofectanine 2000 (0.2 ug per well). Twenty-four hours after transfection, the level of target silencing was measured using BDNA assays.

The results of these experiments are diyplayd in **Figure 11** and show that while unmodified GAPDH4 generates a typical dose-dependent silencing curve (high degrees of silencing at higher concentrations, low degrees of silencing at low concentrations), duplexes carrying molecule 4 modifications (*i.e*., 5'-deoxy) fail to silence the target at any of the concentrations tested. These results support the notion that siRNA carrying molecule 4 modifications can be used as transfection control reagents. Furthermore, these results suggest that addition of molecule 4 modifications to the sense strand of an siRNA duplex would eliminate the ability of that strand to participate in off-target silencing.

In a subsequent experiment, siRNA carrying molecule 4 modifications were assessed in a competition assay similar to those described previously. To accomplish this, HeLa cells (10,000 cells per well) were plated and subsequently transfected with varying concentrations of unmodified GAPDH4 (sense, 5'-ugguuuacau guuccaauau u SEQ. ID NO 41) plus modified (5'-deoxy, sense and antisense) GAPDH30 (sense, 5' ucaugggugu gaaccaugau u SEQ. ID NO 42) siRNA. For comparison, the performance of the 5'-deoxy modified molecule was compared with a second siRNA (17 bp, sense, 5'gcgacuaaac acaucaauu SEQ. ID NO 43) containing 2'-O-methyl modifications on positions 1 and 2 on the sense and antisense strands (*i.e*., molecule 3 modifications). The results of these studies are presented in **Figure 12** and bring out two critical points. First, siRNA containing the 5'-deoxy modification (M4) perform as well as 17bp duplexes containing 2'-O-methyl modifications on positions 1 and 2 of the sense and antisense strand (M3, this conclusion is further supported by data presented in **Figure 9**). Neither molecule inhibits the functionality of GAPDH4. Second, in the case of GAPDH4, it appears that both modified molecules enhance the functionality of the GAPDH4 siRNA at lower concentrations. The inventors attribute this to an increase in the delivery of the GAPDH4 siRNA at low concentrations when the bulking reagent is present.

### EXAMPLE XIII

### MOLECULE 5-OR MOLECULE-6- MODIFIED siRNA

To test the functionality of siRNAs containing molecule 5 modifications, side-by-side dose curves (100, 10, 1, 0.1, and 0.01 nM) were performed that compared siRNA that were (1) unmodified, (2) contained molecule 1 modifications, (3) contained molecule 5 modifications, or (4) contained molecule 6 modifications. Human cyclophilin 1 targeting siRNA (cyclo 1, 3, and 4) were transfected into HeLa cells at varying concentrations (0.01,0.1,1,10, or 100 nM) and the level of target silencing was assessed 24 hours post-transfection by BDNA. The sequences used in these studies included the following

| **TABLE IV** | | |
|---|---|---|
| **SEQUENCES FOR TESTING MOLECULE 5 MODIFICATIONS** | | |
| **SEQUENCE NAME** | **SEQUENCE (SENSE, 5'**→**3')** | **SEQ. ID NO.** |
| Cyclo 1 | guuccaaaaa caguggaua | 44 |
| Cyclo 3 | caaaaacagu ggauaauuu | 45 |
| Cyclo 4 | aaaacagugg auaauuuug | 46 |

As shown in **Figure 13**, the performance of molecules containing modification pattern 5 or 6 (referred to as SOS and SOS+, respectively) are roughly equivalent to unmodified molecules or those containing molecule 1 modifications (referred to as siSTABLE in the figure).

The inventors have also performed side-by-side studies where they compare the longevity of target silencing induced by siRNA that are (1) unmodified, (2) contain molecule 1 modifications. (3) contain molecule 5 modifications, or (4) contain molecule 6 modifications. To accomplish this, modified or unmodified siRNA were transfected into cells at 100nM using Lipofectamine 2000. Subsequently, the level of target knockdown on days 1, 2, 3, 7, and 14 was assessed using BDNA. The sequences used for these studies are given below.

| **TABLE V** | | |
|---|---|---|
| **SEQUENCES FOR TESTING MOLECULE 1, 5, AND 6 MODIFICATIONS** | | |
| **SEQUENCE NAME** | **SEQUENCE (SENSE,'5**→**3')** | **SEQ. ID NO.** |
| Cyclo 1 | guuccaaaaa caguggaua | 47 |
| Cyclo 2 | uccaaaaaca guggauaau | 48 |
| Cyclo 3 | caaaaacagu ggauaauuu | 49 |
| Cyclo 4 | aaaacagugg auaauuuug | 50 |
| Cyclo 14 | ggccuuagcu acaggagag | 51 |
| Cyclo 37 | uuccaucgug uaaucaagg | 52 |
| Cyclo 48 | ucaugaucca gggcggaga | 53 |
| Cyclo 71 | aaagagcauc uacggugag | 54 |

The results are presented in **Figures 14 a-d** and demonstrate that, in general, the performance of the modified molecules over an extended period is the same or better than those that are unmodified.

### ADDITIONAL ASPECTS OF THE DISCLOSURE

1. An RNA duplex comprising:
   (a) a sense strand, wherein said sense strand comprises
      (i) a first 5' terminal sense nucleotide and a second 5' terminal sense nucleotide, wherein said first 5' terminal sense nucleotide comprises a first 2'-O-alkyl sense modification and said second 5' terminal sense nucleotide comprises a second 2'-O-alkyl sense modification;
      (ii) at least one 2'-O-alkyl pyrimidine modified sense nucleotide, wherein said at least one 2'-O-alkyl pyrimidine modified sense nucleotide is a nucleotide other than said first 5' terminal sense nucleotide or said second 5' terminal sense nucleotide; and
   (b) an antisense strand, wherein said antisense strand comprises
      (i) at least one 2' halogen modified pyrimidine nucleotide; and
      (ii) a first 5' terminal antisense nucleotide, wherein said first 5' terminal antisense nucleotide is phosphorylated at its 5' carbon position,
   wherein the sense strand and the antisense strand are capable of forming a duplex of between 18 and 30 base pairs.
2. The RNA duplex of 1, wherein the sense strand and antisense strand are capable of forming a duplex of between 19 and 25 base pairs.
3. The RNA duplex of 1 further comprising a label.
4. The RNA duplex of 3, wherein said label is attached to said first 5' terminal sense nucleotide.
5. The RNA duplex of 4, wherein said label is a fluorescent dye.
6. The RNA duplex of 1, wherein all pyrimidines on said sense strand comprise a 2'-O-alkyl modification.
7. The RNA duplex of 1, wherein all pyrimidines on said antisense strand comprises 2'-fluoro modification:
8. The RNA duplex of 1, wherein said first 2'-O-alkyl sense modification, said second 2'-O-alkyl sense modification, and said at least one 2'-O-alkyl pyrimidine modified sense nucleotide each comprises 2'-O-methyl.
9. The RNA duplex of 1, wherein said halogen is fluorine.
10. The RNA duplex of 1, wherein the first 2'-O-alkyl sense modification is 2'-O-methyl, the second 2'-O-alkyl sense modification is 2'-O-methyl, the at least one 2'-O-alkyl pyrimidine modified sense nucleotide comprises 2'-O-methyl, the halogen is fluorine, and the first 5' terminal sense nucleotide further comprises a fluorescent dye.
11. The RNA duplex of 1 further comprising at least one phosphorothioate internucleotide linkage.
12. The RNA duplex of 1 further comprising at least one methylphosphonate internucleotide linkage.
13. The RNA duplex of 8, further comprising
   a 2'-O-methyl modification on each pyrimidine nucleotide of the sense strand;
   a 2'-fluorine modification on each pyrimidine nucleotide of the antisense strand;
   a 3' overhang on the 3' end of the antisense strand comprising a first overhang nucleotide and a second overhang nucleotide, wherein the first overhang nucleotide is attached to the RNA duplex by a phosphorothioate internucleotide linkage, and the first overhang nucleotide and the second overhang nucleotide are attached to each other by a phosphorothioate internucleotide linkage; and
   a blunt end at the 5' terminus of the antisense strand and the 3' terminus of the sense strand.
14. The RNA duplex of 13, further comprising a second 5' terminal antisense nucleotide, wherein said second 5' terminal antisense nucleotide comprises a 2'-O-methyl modification.
15. An RNA duplex comprising:
   (a) a sense strand, wherein said sense strand comprises
      (i) a first 5' terminal sense nucleotide and a second 5' terminal sense nucleotide, wherein said first 5' terminal sense nucleotide comprises a first 2'-O-alkyl sense modification and said second 5' terminal sense nucleotide comprises a second 2'-O-alkyl sense modification;
      (ii) at least one 2'-O-alkyl pyrimidine modified sense nucleotide, wherein said at least one 2'-O-alkyl pyrimidine modified sense nucleotide is a nucleotide other than said first 5' terminal sense nucleotide or said second 5' terminal sense nucleotide; and
   (b) an antisense strand, wherein said antisense strand comprises
      (i) a first 5' terminal antisense nucleotide and a second 5' terminal antisense nueleotide, wherein said first 5' terminal antisense nucleotide comprises and a first 2'-O-alkyl antisense modification and said second 5' terminal antisense nucleotide comprises a second 2'-O-alkyl antisense modification; and
      (ii) at least one 2'-O-alkyl pyrimidine modified antisense nucleotide, wherein said at least one 2'-O-alkyl modified antisense nucleotide is a nucleotide other than said first 5' terminal antisense nucleotide or said second 5' terminal antisense nucleotide,
   wherein the sense strand and antisense strand are capable of forming a duplex of between 16 and 50 base pairs.
16. The RNA duplex of 15, wherein the sense strand and antisense strand are capable of forming a duplex of between 18 and 30 base pairs.
17. The RNA duplex of 16, wherein the sense strand and antisense strand are capable of forming a duplex of between 19 and 25 base pairs.
18. The RNA duplex of 15, wherein each pyrimidine on said sense strand comprises a 2'-O-alkyl modification.
19. The RNA duplex of 15, wherein each pyrimidine on said antisense strand comprises a 2'-O-alkyl modification.
20. The RNA duplex of 15, wherein said first 2'-O-alkyl sense modification, said second 2'-O-alkyl sense modification, said first 2'-O-alkyl antisense modification, said second 2'-O-alkyl antisense modification, said at least one 2'-O-alkyl pyrimidine modified sense nucleotide, and said at least one 2'-O-alkyl pyrimidine modified antisense nucleotide each comprises 2'-O-methyl.
21. The RNA duplex of 15 further comprising a label.
22. The RNA duplex of 21, wherein said label is attached to said first 5' terminal sense nucleotide.
23. The RNA duplex of 21, wherein said label is a fluorescent dye.
24. The RNA duplex of 15 further comprising at least one phosphorothioate internucleotide linkage.
25. The RNA duplex of 15 further comprising at least one methylphosphonate internucleotide linkage.
26. A method of gene silencing comprising introducing the RNA duplex of 1 into a cell that is expressing or is capable of expressing a target gene.
27. A method of performing RNA interference, comprising introducing the RNA duplex of 15 into a cell in the presence or absence of an RNA duplex that is capable of silencing a target gene.
28. An RNA duplex comprising:
   (a) a sense strand, wherein said sense strand comprises
      (i) a first 5' terminal sense nucleotide and a second 5' terminal sense nucleotide, wherein said first 5' terminal sense nucleotide comprises a first 2'-O-alkyl sense modification, and said second 5' terminal sense nucleotide comprises a second 2'-O-alkyl sense modification;
   (b) an antisense strand, wherein said antisense strand comprises
      (i) a first 5' terminal antisense nucleotide and a second 5' terminal antisense nucleotide, wherein said first 5' terminal antisense nucleotide comprises a first 2'-O-alkyl antisense modification, and said second 5' terminal antisense nucleotide comprises a second 2'-O-alkyl antisense modification;
         wherein neither the sense strand nor the antisense strand have been phosphorylated at the 5' carbon position of their first 5' terminal nucleotides and the sense and/or antisense strand may comprise a blocking group at the 5' carbon position of the first 5' terminal nucleotide, and the sense strand and antisense strand are capable of forming a duplex of 16 to 30 base pairs.
29. An RNA duplex comprising:
   (a) a sense strand, wherein said sense strand comprises
      (i) a first 5' terminal sense nucleotide, a second 5' terminal sense nucleotide, and at least one 2'-O-alkyl pyrimidine modified sense nucleotide that is not the first 5' terminal nucleotide or second 5' terminal sense nucleotide, wherein said first 5' terminal sense nucleotide comprises a first 2'-O-alkyl sense modification and said second 5' terminal sense nucleotide comprises a second 2'-O-alkyl sense modification; and
   (b) an antisense strand, wherein said antisense strand comprises
      (i) a first 5' terminal antisense nucleotide, a second 5' terminal antisense nucleotide, and at least one 2' halogen modified pyrimidine antisense nucleotide that is not the first 5' terminal antisense nucleotide or second 5' terminal antisense nucleotide, wherein said first 5' terminal antisense nucleotide comprises a first 2'-O-alkyl antisense modification and said second 5' terminal antisense nucleotide comprises a second 2'-O-alkyl antisense modification;
         wherein neither the sense strand nor the antisense strand have been phosphorylated at the 5' carbon position of their first 5' terminal nucleotides and the sense and/or antisense strand may comprise a blocking group at the 5' carbon position of the first 5' terminal nucleotide, and the sense strand and antisense strand are capable of forming a duplex of 16 to 30 base pairs.
30. An RNA duplex comprising:
   (a) a sense strand, wherein said sense strand comprises
      (i) a first 5' terminal sense nucleotide, a second 5' terminal sense nucleotide, a third 5' terminal nucleotide, and at least one 2'-O-alkyl pyrimidine modified sense nucleotide that is not the first 5' terminal sense nucleotide, second 5' terminal sense nucleotide, or third 5' terminal sense nucleotide, wherein said first 5' terminal sense nucleotide comprises a first 2'-O-alkyl sense modification, said second 5' terminal sense nucleotide comprises a second 2'-O-alkyl sense modification, and said third 5' terminal sense nucleotide comprises a third 2'-O-alkyl sense modification; and
   (b) an antisense strand, wherein said antisense strand comprises
      (i) a first 5' terminal antisense nucleotide, a second 5' terminal antisense nucleotide, a third 5' terminal antisense nucleotide, and at least one 2' halogen modified pyrimidine antisense nucleotide that is not the first 5' terminal antisense nucleotide, or second 5' terminal antisense nucleotide, or third 5' terminal
         antisense nucleotide, wherein said first 5' terminal antisense nucleotide comprises a first 2'-O-alkyl antisense modification, said second 5' terminal antisense nucleotide comprises a second 2'-O-alkyl antisense modification, and said third 5' terminal antisense nucleotide comprises a third 2'-O-alkyl antisense modification;
   wherein neither the sense strand nor the antisense strand have been phosphorylated at the 5' carbon position of their first 5' terminal nucleotide and the sense and/or antisense strand may comprise a blocking group at the 5' carbon position of the first 5' terminal nucleotide, and the sense strand and antisense strand are capable of forming a duplex of 16 to 30 base pairs.
31. An RNA duplex comprising:
   (a) a sense strand, wherein said sense strand comprises
      (i) a first 5' terminal sense nucleotide and a second 5' terminal sense nucleotide, wherein said first 5' terminal sense nucleotide comprises a first 2'-O-methyl sense modification, and said second 5' terminal sense nucleotide comprises a second 2'-O-methyl sense modification; and
   (b) an antisense strand, wherein said antisense strand comprises
      (i) a first 5' terminal antisense nucleotide and a second 5' terminal antisense nucleotide, wherein said first 5' terminal antisense nucleotide comprises a first 2'-O-methyl antisense modification, and said second 5' terminal antisense nucleotide comprises a second 2'-O-methyl antisense modification;
         wherein neither the sense strand nor the antisense strand have been phosphorylated at the 5' carbon position of their first 5' terminal nucleotides and the sense and/or antisense strand may comprise a blocking group at the 5' carbon position of the first 5' terminal nucleotide, and the sense strand and antisense strand are capable of forming a duplex of 16 to 30 base pairs.
32. The RNA duplex of 31, further comprising a label.
33. The RNA duplex of 32, wherein said label is attached to said first 5' terminal sense nucleotide.
34. The RNA duplex of 32, wherein said label is a fluorescent dye.
35. The RNA duplex of 31, wherein all pyrimidines on said sense strand comprise a 2'-O-alkyl modification.
36. The RNA duplex of 31, wherein all pyrimidines on said antisense strand, except for any pyrimidines at the first 5' terminal antisense nucleotide and the second 5' terminal antisense nucleotide, comprise a 2'-fluoro modification.
37. The RNA duplex of 31 further comprising at least one phosphorothioate internucleotide linkage.
38. The RNA duplex of 31 further comprising at least one methylphosphonate internucleotide linkage.
39. An RNA duplex comprising:
   (a) a sense strand, wherein said sense strand comprises
      (i) a first 5' terminal sense nucleotide, a second 5' terminal sense nucleotide, and at least one .2'-O-methyl pyrimidine modified sense nucleotide that is not the first 5' terminal sense nucleotide or second 5' terminal sense nucleotide, wherein said first 5' terminal sense nucleotide comprises a first 2'-O-methyl sense modification and said second 5' terminal sense nucleotide comprises a second 2'-O-methyl sense modification; and
   (b) an antisense strand, wherein said antisense strand comprises
      (i) a first 5' terminal antisense nucleotide, a second 5' terminal antisense nucleotide, and at least one 2' fluorine modified pyrimidine antisense nucleotide that is not the first 5' terminal antisense nucleotide or second 5' terminal antisense nucleotide, wherein said first 5' terminal antisense nucleotide comprises a
         first 2'-O-methyl antisense modification and said second 5' terminal antisense nucleotide comprises a second 2'-O-methyl antisense modification;
   wherein neither the sense strand nor the antisense strand have been phosphorylated at the 5' carbon position of their first 5' terminal nucleotides and the sense and/or antisense strand may comprise a blocking group at the 5' carbon position of the first 5' terminal nucleotide, and the sense strand and antisense strand are capable of forming a duplex of 16 to 30 base pairs.
40. The RNA duplex of 39, further comprising a label.
41. The RNA duplex of 40, wherein said label is attached to said first 5' terminal sense nucleotide.
42. The RNA duplex of 40, wherein said label is a fluorescent dye.
43. The RNA duplex of 39, wherein all pyrimidines on said sense strand comprise a 2'-O-alkyl modification.
44. The RNA duplex of 39, wherein all pyrimidines on said antisense strand, except for any pyrimidines at the first 5' terminal antisense nucleotide and the second 5' terminal antisense nucleotide, comprise a 2'-fluoro modification.
45. The RNA duplex of 39, further comprising at least one phosphorothioate internucleotide linkage.
46. The RNA duplex of 39, further comprising at least one methylphosphonate internucleotide linkage.
47. An RNA duplex comprising:
   (a) a sense strand, wherein said sense strand comprises
      (i) a first 5' terminal sense nucleotide, a second 5' terminal sense nucleotide, a third 5' terminal sense nucleotide, and at least one 2'-O-methyl pyrimidine modified sense nucleotide that is not the first 5' terminal sense nucleotide, or second 5' terminal sense nucleotide, or third 5' terminal sense nucleotide, wherein said first 5' terminal sense nucleotide comprises a first 2'-O-methyl sense modification, said second 5' terminal sense nucleotide comprises a second 2'-O-methyl sense modification, and said third terminal sense nucleotide comprises a third 2'-O methyl sense modification; and
   (b) an antisense strand, wherein said antisense strand comprises
      (i) a first 5' terminal antisense nucleotide, a second 5' terminal antisense nucleotide, a third 5' terminal antisense nucleotide, and at least one 2' fluorine modified pyrimidine antisense nucleotide that is not the first 5' terminal antisense nucleotide, second 5' terminal antisense nucleotide, or third 5' terminal antisense nucleotide, wherein said first 5' terminal antisense nucleotide comprises a first 2'-O-methyl antisense modification, said second 5' terminal antisense nucleotide comprises a second 2'-O-methyl antisense modification, and said third 5' terminal antisense nucleotide comprises a third 2'-O-methyl antisense modification;
   wherein neither the sense strand nor the antisense strand have been phosphorylated at the 5' carbon position of their first 5' terminal nucleotides and the sense and/or antisense strand may comprise a blocking group at the 5' carbon position of the first 5' terminal nucleotide, and the sense strand and antisense strand are capable of forming a duplex of 16 to 30 base pairs.
48. The RNA duplex of 47, further comprising a label.
49. The RNA duplex of 48, wherein said label is attached to said first 5' terminal sense nucleotide.
50. The RNA duplex of 48, wherein said label is a fluorescent dye.
51. The RNA duplex of 47, wherein all pyrimidines on said sense strand comprise a 2'-O-alkyl modification.
52. The RNA duplex of 47, wherein all pyrimidines on said antisense strand, except for any pyrimidines at the first 5' terminal antisense nucleotide and the second 5' terminal antisense nucleotide, comprise a 2'-fluoro modification.
53. The RNA duplex of 47, further comprising at least one phosphorothioate internucleotide linkage.
54. The RNA duplex of 47, further comprising at least one methylphosphonate internucleotide linkage.
55. The RNA duplex of 1, further comprising a third 5' terminal sense nucleotide, wherein said third 5' terminal sense nucleotide comprises a third 2'-O-alkyl sense modification.
56. The RNA duplex of 1, further comprising a third 5' terminal antisense nucleotide, wherein said third 5' terminal antisense nucleotide comprises a third 2'-O-alkyl sense modification.
57. The RNA duplex of 1, further comprising a blocking group on said first terminal sense nucleotide.
58. A method of performing RNA interference, comprising introducing the RNA duplex of 28 into a cell in the presence or absence of an RNA duplex that is capable of silencing a target gene.
59. A method of performing RNA interference, comprising introducing the RNA duplex of 29 into a cell in the presence or absence of an RNA duplex that is capable of silencing a target gene.
60. A method of performing RNA interference, comprising introducing the RNA duplex ot 30 into a cell in the presence or absence of an RNA duplex that is capable of silencing a target gene.
61. A method of performing RNA interference, comprising introducing the RNA duplex of 31 into a cell in the presence or absence of an RNA duplex that is capable of silencing a target gene.
62. The RNA duplex of 28, further comprising a third 5' terminal sense nucleotide, wherein said third 5' terminal sense nucleotide comprises a third 2'-O-alkyl sense modification.
63. The RNA duplex of 28, further comprising a third 5' terminal antisense nucleotide, wherein said third 5' terminal antisense nucleotide comprises a third 2'-O-alkyl antisense modification.
64. The RNA duplex of 31, further comprising a third 5' terminal sense nucleotide, wherein said third 5' terminal sense nucleotide comprises a third 2'-O-methyl sense modification.
65. The RNA duplex of 31, further comprising a third 5' terminal antisense sense nucleotide, wherein said third 5' terminal antisense nucleotide comprises a third 2'-O-methyl antisense modification.

## Claims

1. An RNA duplex comprising:
(a) a sense strand, wherein said sense strand comprises
(i) a first 5' terminal sense nucleotide and a second 5' terminal sense nucleotide, wherein said first 5' terminal sense nucleotide comprises a first 2'-O-alkyl sense modification and said second 5' terminal sense nucleotide comprises a second 2'-O-alkyl sense modification;
(ii) at least one 2'-O-alkyl pyrimidine modified sense nucleotide, wherein said at least one 2'-O-alkyl pyrimidine modified sense nucleotide is a nucleotide other than said first 5' terminal sense nucleotide or said second 5' terminal sense nucleotide and such that each pyrimidine on said sense strand comprises a 2'-O-alkyl modification; and
(b) an antisense strand, wherein said antisense strand comprises
(i) a first 5' terminal antisense nucleotide and a second 5' terminal antisense nucleotide, wherein said first 5' terminal antisense nucleotide comprises a first 2'-O-alkyl antisense modification and said second 5' terminal antisense nucleotide comprises a second 2'-O-alkyl antisense modification; and
(ii) at least one 2'-O-alkyl pyrimidine modified antisense nucleotide, wherein said at least one 2'-O-alkyl modified antisense nucleotide is a nucleotide other than said first 5' terminal antisense nucleotide or said second 5' terminal antisense nucleotide and such that each pyrimidine on said antisense strand comprises a 2'-O-alkyl modification,
wherein the sense strand and antisense strand are capable of forming a duplex of between 16 and 50 base pairs;
wherein all nucleotides within the duplex other than said first 5' terminal sense nucleotide, second 5' terminal sense nucleotide, each pyrimidine on said sense strand, first 5' terminal antisense nucleotide, second 5' terminal antisense nucleotide and each pyrimidine on said antisense strand are unmodified;
and wherein the first 5' terminal antisense nucleotide is not phosphorylated.

2. The RNA duplex of claim 1, wherein the sense strand and antisense strand are capable of forming a duplex of between 18 and 30 base pairs.

3. The RNA duplex of claim 2, wherein the sense strand and antisense strand are capable of forming a duplex of between 19 and 25 base pairs.

4. The RNA duplex of claim 1, wherein each 2'-O-alkyl sense modification and each 2'-O-alkyl antisense modification comprises 2'-O-methyl.

5. The RNA duplex of claim 1 further comprising a label.

6. The RNA duplex of claim 5, wherein said label is attached to said first 5' terminal sense nucleotide.

7. The RNA duplex of claim 5, wherein said label is a fluorescent dye.

8. The RNA duplex of claim 1 further comprising at least one phosphorothioate internucleotide linkage.

9. The RNA duplex of claim 1 further comprising at least one methylphosphonate internucleotide linkage.

10. An RNA duplex comprising:
(a) a sense strand, wherein said sense strand comprises
(i) a first 5' terminal sense nucleotide and a second 5' terminal sense nucleotide, wherein said first 5' terminal sense nucleotide comprises a first 2'-O-alkyl sense modification, and said second 5' terminal sense nucleotide comprises a second 2'-O- alkyl sense modification;
b) an antisense strand, wherein said antisense strand comprises
(i) a first 5' terminal antisense nucleotide and a second 5' terminal antisense nucleotide, wherein said first 5' terminal antisense nucleotide comprises a first 2'-O-alkyl antisense modification, and said second 5' terminal antisense nucleotide comprises a second 2'-O-alkyl antisense modification;
wherein:
- neither the sense strand nor the antisense strand is phosphorylated at the 5' carbon position of their first 5' terminal nucleotides;
- the sense and/or antisense strand may comprise a blocking group at the 5' carbon position of the first 5' terminal nucleotide;
- the sense strand and antisense strand are capable of forming a duplex of 16 to 30 base pairs; and
- all nucleotides within the duplex other than said first 5' terminal sense nucleotide, second 5' terminal sense nucleotide, first 5' terminal antisense nucleotide and second 5' terminal antisense nucleotide are unmodified.

11. An RNA duplex comprising:
(a) a sense strand, wherein said sense strand comprises
(i) a first 5' terminal sense nucleotide, a second 5' terminal sense nucleotide, a third 5' terminal sense nucleotide, and at least one 2'-O- alkyl pyrimidine modified sense nucleotide that is not the first 5' terminal sense nucleotide, second 5' terminal sense nucleotide, or third 5' terminal sense nucleotide, wherein said first 5' terminal sense nucleotide comprises a first 2'-O-alkyl sense modification, said second 5' terminal sense nucleotide comprises a second 2'-O-alkyl sense modification, and said third 5' terminal sense nucleotide comprises a third 2'-O-alkyl sense modification; and
(b) an antisense strand, wherein said antisense strand comprises
(i) a first 5' terminal antisense nucleotide, a second 5' terminal antisense nucleotide, a third 5' terminal antisense nucleotide, and at least one 2' halogen modified pyrimidine antisense nucleotide that is not the first 5' terminal antisense nucleotide, or second 5' terminal antisense nucleotide, or third 5' terminal antisense nucleotide, wherein said first 5' terminal antisense nucleotide comprises a first 2'-O-alkyl antisense modification, said second 5' terminal antisense nucleotide comprises a second 2'-O-alkyl antisense modification, and said third 5' terminal antisense nucleotide comprises a third 2'-O-alkyl antisense modification;
wherein neither the sense strand nor the antisense strand is phosphorylated at the 5' carbon position of their first 5' terminal nucleotide and the sense and/or antisense strand may comprise a blocking group at the 5' carbon position of the first 5' terminal nucleotide, and the sense strand and antisense strand are capable of forming a duplex of 16 to 30 base pairs.

12. The RNA duplex of claim 11, wherein all of the pyrimidines of the antisense strand, other than said first 5' terminal antisense nucleotide, second 5' terminal antisense nucleotide and third 5' terminal antisense nucleotide, are 2' halogen modified.

13. A method of performing RNA interference, comprising introducing the RNA duplex of any one of claims 1-12 into a cell in the presence of an RNA duplex that is capable of silencing a target gene, wherein said method is not a method for treatment of the human or animal body by surgery or therapy or a diagnostic method practised on the human or animal body.

## Patentansprüche

1. RNA-Doppelstrang, umfassend:
(a) einen Sense-Strang, wobei der Sense-Strang umfasst:
(i) ein erstes 5'-terminales Sense-Nukleotid und ein zweites 5'-terminales Sense-Nukleotid, wobei das erste 5'-terminale Sense-Nukleotid eine erste 2'-O-Alkyl-Sense-Modifikation umfasst und das zweite 5'-terminale Sense-Nukleotid eine zweite 2'-O-Alkyl-Sense-Modifikation umfasst;
(ii) wenigstens ein 2'-O-Alkylpyrimidinmodifiziertes Sense-Nukleotid, wobei das wenigstens eine 2'-O-Alkylpyrimidinmodifizierte Sense-Nukleotid ein anderes Nukleotid als das erste 5'-terminale Sense-Nukleotid oder das zweite 5'-terminale Sense-Nukleotid ist und so ist, dass jedes Pyrimidin an dem Sense-Strang eine 2'-O-Alkyl-Modifikation umfasst, und
(b) einen Antisense-Strang, wobei der Antisense-Strang umfasst:
(i) ein erstes 5'-terminales Antisense-Nukleotid und ein zweites 5'-terminales Antisense-Nukleotid, wobei das erste 5'-terminale Antisense-Nukleotid eine erste 2'-O-Alkyl-Antisense-Modifikation umfasst und das zweite 5'-terminale Antisense-Nukleotid eine zweite 2'-O-Alkyl-Antisense-Modifikation umfasst, und
(ii) wenigstens ein 2'-O-Alkylpyrimidinmodifiziertes Antisense-Nukleotid, wobei das wenigstens eine 2'-O-Alkyl-modifizierte Antisense-Nukleotid ein anderes Nukleotid als das erste 5'-terminale Antisense-Nukleotid oder das zweite 5'-terminale Antisense-Nukleotid ist und so ist, dass jedes Pyrimidin an dem Antisense-Strang eine 2'-O-Alkyl-Modifikation umfasst,
wobei der Sense-Strang und der Antisense-Strang fähig sind, einen Doppelstrang von zwischen 16 und 50 Basenpaaren zu bilden;
wobei in dem Doppelstrang alle anderen Nukleotide als das erste 5'-terminale Sense-Nukleotid, das zweite 5'-terminale Sense-Nukleotid, jedes Pyrimidin an dem Sense-Strang, das erste 5'-terminale Antisense-Nukleotid, das zweite 5'-terminale Antisense-Nukleotid und jedes Pyrimidin an dem Antisense-Strang unmodifiziert sind,
und wobei das erste 5'-terminale Antisense-Nukleotid nicht phosphoryliert ist.

2. RNA-Doppelstrang gemäß Anspruch 1, wobei der Sense-Strang und der Antisense-Strang fähig sind, einen Doppelstrang von zwischen 18 und 30 Basenpaaren zu bilden.

3. RNA-Doppelstrang gemäß Anspruch 2, wobei der Sense-Strang und der Antisense-Strang fähig sind, einen Doppelstrang von zwischen 19 und 25 Basenpaaren zu bilden.

4. RNA-Doppelstrang gemäß Anspruch 1, wobei jede 2'-0-Alkyl-Sense-Modifikation und jede 2'-0-Alkyl-Antisense-Modifikation 2'-0-Methyl umfasst.

5. RNA-Doppelstrang gemäß Anspruch 1, der außerdem eine Markierung umfasst.

6. RNA-Doppelstrang gemäß Anspruch 5, wobei die Markierung an dem ersten 5'-terminalen Sense-Nukleotid befestigt ist.

7. RNA-Doppelstrang gemäß Anspruch 5, wobei die Markierung ein Fluoreszenzfarbstoff ist.

8. RNA-Doppelstrang gemäß Anspruch 1, der außerdem wenigstens eine Phosphorthioat-Internukleotid-Verknüpfung umfasst.

9. RNA-Doppelstrang gemäß Anspruch 1, der außerdem wenigstens eine Methylphosphonat-Internukleotid-Verknüpfung umfasst.

10. RNA-Doppelstrang, umfassend:
(a) einen Sense-Strang, wobei der Sense-Strang umfasst:
(i) ein erstes 5'-terminales Sense-Nukleotid und ein zweites 5'-terminales Sense-Nukleotid, wobei das erste 5'-terminale Sense-Nukleotid eine erste 2'-0-Alkyl-Sense-Modifikation umfasst und das zweite 5'-terminale Sense-Nukleotid eine zweite 2'-O-Alkyl-Sense-Modifikation umfasst;
(b) einen Antisense-Strang, wobei der Antisense-Strang umfasst:
(i) ein erstes 5'-terminales Antisense-Nukleotid und ein zweites 5'-terminales Antisense-Nukleotid, wobei das erste 5'-terminale Antisense-Nukleotid eine erste 2'-O-Alkyl-Antisense-Modifikation umfasst und das zweite 5'-terminale Antisense-Nukleotid eine zweite 2'-O-Alkyl-Antisense-Modifikation umfasst;
wobei:
- weder der Sense-Strang noch der Antisense-Strang an der 5'-Kohlenstoffposition ihrer ersten 5'-terminalen Nukleotide phosphoryliert ist;
- der Sense- und/oder Antisense-Strang eine Blockierungsgruppe an der 5'-Kohlenstoffposition des ersten 5'-terminalen Nukleotids umfassen kann;
- der Sense-Strang und der Antisense-Strang fähig sind, einen Doppelstrang von 16 bis 30 Basenpaaren zu bilden, und
- in dem Doppelstrang alle anderen Nukleotide als das erste 5'-terminale Sense-Nukleotid, das zweite 5'-terminale Sense-Nukleotid, das erste 5'-terminale Antisense-Nukleotid und das zweite 5'-terminale Antisense-Nukleotid unmodifiziert sind.

11. RNA-Doppelstrang, umfassend:
(a) einen Sense-Strang, wobei der Sense-Strang umfasst:
(i) ein erstes 5'-terminales Sense-Nukleotid, ein zweites 5'-terminales Sense-Nukleotid, ein drittes 5'-terminales Sense-Nukleotid und wenigstens ein 2'-O-Alkylpyrimidinmodifiziertes Sense-Nukleotid, das nicht das erste 5'-terminale Sense-Nukleotid, das zweite 5'-terminale Sense-Nukleotid oder das dritte 5'-terminale Sense-Nukleotid ist, wobei das erste 5'-terminale Sense-Nukleotid eine erste 2'-O-Alkyl-Sense-Modifikation umfasst, das zweite 5'-terminale Sense-Nukleotid eine zweite 2'-O-Alkyl-Sense-Modifikation umfasst und das dritte 5'-terminale Sense-Nukleotid eine dritte 2'-O-Alkyl-Sense-Modifikation umfasst, und
(b) einen Antisense-Strang, wobei der Antisense-Strang umfasst:
(i) ein erstes 5'-terminales Antisense-Nukleotid, ein zweites 5'-terminales Antisense-Nukleotid, ein drittes 5'-terminales Antisense-Nukleotid und wenigstens ein 2'-Halogen-modifiziertes Pyrimidin-Antisense-Nukleotid, das nicht das erste 5'-terminale Antisense-Nukleotid oder das zweite 5'-terminale Antisense-Nukleotid oder das dritte 5'-terminale Antisense-Nukleotid ist, wobei das erste 5'-terminale Antisense-Nukleotid eine erste 2'-O-Alkyl-Antisense-Modifikation umfasst, das zweite 5'-terminale Antisense-Nukleotid eine zweite 2'-0-Alkyl-Antisense-Modifikation umfasst und das dritte 5'-terminale Antisense-Nukleotid eine dritte 2'-O-Alkyl-Antisense-Modifikation umfasst;
wobei weder der Sense-Strang noch der Antisense-Strang an der 5'-Kohlenstoffposition ihres ersten 5'-terminalen Nukleotids phosphoryliert ist und der Sense- und/oder Antisense-Strang eine Blockierungsgruppe an der 5'-Kohlenstoffposition des ersten 5'-terminalen Nukleotids umfassen kann, und der Sense-Strang und der Antisense-Strang in der Lage sind, einen Doppelstrang von 16 bis 30 Basenpaaren zu bilden.

12. RNA-Doppelstrang gemäß Anspruch 11, wobei alle anderen Pyrimidine des Antisense-Strangs als das des ersten 5'-terminalen Antisense-Nukleotids, des zweiten 5'-terminalen Antisense-Nukleotids und des dritten 5'-terminalen Antisense-Nukleotids 2'-Halogen-modifiziert sind.

13. Verfahren zur Durchführung einer RNA-Interferenz, umfassend Einführen des RNA-Doppelstrangs gemäß einem der Ansprüche 1-12 in eine Zelle in Gegenwart eines RNA-Doppelstrangs, der fähig ist, ein Targetgen abzuschalten (silencing), wobei das Verfahren kein Verfahren zur Behandlung des Menschen- oder Tierkörpers durch Operation oder Therapie ist oder kein diagnostisches Verfahren ist, das am Menschen- oder Tierkörper durchgeführt wird.

## Revendications

1. Duplex d'ARN, comprenant :
a) un brin sens, lequel brin sens comprend :
i) un premier nucléotide sens 5'-terminal et un deuxième nucléotide sens 5'-terminal, lequel premier nucléotide sens 5'-terminal comporte une première modification sens 2'-O-alkyle, et lequel deuxième nucléotide sens 5'-terminal comporte une deuxième modification sens 2'-O-alkyle,
ii) et au moins un nucléotide sens modifié 2'-O-alkyl-pyrimidine, lequel nucléotide sens modifié 2'-O-alkyl-pyrimidine au nombre d'au moins un est un autre nucléotide que ledit premier nucléotide sens 5'-terminal ou ledit deuxième nucléotide sens 5'-terminal, de sorte que chaque base pyrimidine dudit brin sens comporte une modification 2'-O-alkyle,
b) et un brin anti-sens, lequel brin anti-sens comprend :
i) un premier nucléotide anti-sens 5'-terminal et un deuxième nucléotide anti-sens 5'-terminal, lequel premier nucléotide anti-sens 5'-terminal comporte une première modification anti-sens 2'-O-alkyle, et lequel deuxième nucléotide anti-sens 5'-terminal comporte une deuxième modification anti-sens 2'-O-alkyle,
ii) et au moins un nucléotide anti-sens modifié 2'-O-alkyl-pyrimidine, lequel nucléotide anti-sens modifié 2'-O-alkyl-pyrimidine au nombre d'au moins un est un autre nucléotide que ledit premier nucléotide anti-sens 5'-terminal ou ledit deuxième nucléotide anti-sens 5'-terminal, de sorte que chaque base pyrimidine dudit brin anti-sens comporte une modification 2'-O-alkyle,
étant entendu que ce brin sens et ce brin anti-sens sont capables de former un duplex d'une longueur de 16 à 50 paires de bases,
étant entendu que tous les nucléotides présents au sein de ce duplex, autres que lesdits premier nucléotide sens 5'-terminal et deuxième nucléotide sens 5'-terminal, que chaque base pyrimidine dudit brin sens, que lesdits premier nucléotide anti-sens 5'-terminal et deuxième nucléotide anti-sens 5'-terminal, et que chaque base pyrimidine dudit brin anti-sens, ne sont pas modifiés,
et étant entendu que le premier nucléotide anti-sens 5'-terminal n'est pas phosphorylé.

2. Duplex d'ARN conforme à la revendication 1, dans lequel le brin sens et le brin anti-sens sont capables de former un duplex d'une longueur de 18 à 30 paires de bases.

3. Duplex d'ARN conforme à la revendication 2, dans lequel le brin sens et le brin anti-sens sont capables de former un duplex d'une longueur de 19 à 25 paires de bases.

4. Duplex d'ARN conforme à la revendication 1, dans lequel chaque modification sens 2'-O-alkyle et chaque modification anti-sens 2'-O-alkyle sont des modifications 2'-O-méthyle.

5. Duplex d'ARN conforme à la revendication 1, qui comporte en outre un marqueur.

6. Duplex d'ARN conforme à la revendication 5, dans lequel ledit marqueur est attaché audit premier nucléotide sens 5'-terminal.

7. Duplex d'ARN conforme à la revendication 5, dans lequel ledit marqueur est un colorant fluorescent.

8. Duplex d'ARN conforme à la revendication 1, qui comporte en outre au moins un raccord inter-nucléotides de type phosphorothioate.

9. Duplex d'ARN conforme à la revendication 1, qui comporte en outre au moins un raccord inter-nucléotides de type méthyl-phosphonate.

10. Duplex d'ARN, comprenant :
a) un brin sens, lequel brin sens comprend :
i) un premier nucléotide sens 5'-terminal et un deuxième nucléotide sens 5'-terminal, lequel premier nucléotide sens 5'-terminal comporte une première modification sens 2'-O-alkyle, et lequel deuxième nucléotide sens 5'-terminal comporte une deuxième modification sens 2'-O-alkyle,
b) et un brin anti-sens, lequel brin anti-sens comprend :
i) un premier nucléotide anti-sens 5'-terminal et un deuxième nucléotide anti-sens 5'-terminal, lequel premier nucléotide anti-sens 5'-terminal comporte une première modification anti-sens 2'-O-alkyle, et lequel deuxième nucléotide anti-sens 5'-terminal comporte une deuxième modification anti-sens 2'-O-alkyle,
étant entendu
- que ni le brin sens, ni le brin anti-sens n'est phosphorylé au niveau de l'atome de carbone en 5' de leur premier nucléotide 5'-terminal,
- que le brin sens et/ou le brin anti-sens peut ou peuvent porter un groupe bloquant au niveau de l'atome de carbone en 5' de leur premier nucléotide 5'-terminal,
- que le brin sens et le brin anti-sens sont capables de former un duplex d'une longueur de 16 à 30 paires de bases,
- et que tous les nucléotides présents au sein de ce duplex, autres que lesdits premier nucléotide sens 5'-terminal, deuxième nucléotide sens 5'-terminal, premier nucléotide anti-sens 5'-terminal et deuxième nucléotide anti-sens 5'-terminal, ne sont pas modifiés.

11. Duplex d'ARN, comprenant :
a) un brin sens, lequel brin sens comprend :
i) un premier nucléotide sens 5'-terminal, un deuxième nucléotide sens 5'-terminal, un troisième nucléotide sens 5'-terminal et au moins un nucléotide sens modifié 2'-O-alkyl-pyrimidine qui n'est ni le premier nucléotide sens 5'-terminal, ni le deuxième nucléotide sens 5'-terminal, ni le troisième nucléotide sens 5'-terminal, lequel premier nucléotide sens 5'-terminal comporte une première modification sens 2'-O-alkyle, lequel deuxième nucléotide sens 5'-terminal comporte une deuxième modification sens 2'-O-alkyle, et lequel troisième nucléotide sens 5'-terminal comporte une troisième modification sens 2'-O-alkyle,
b) et un brin anti-sens, lequel brin anti-sens comprend :
i) un premier nucléotide anti-sens 5'-terminal, un deuxième nucléotide anti-sens 5'-terminal, un troisième nucléotide anti-sens 5'-terminal et au moins un nucléotide anti-sens modifié 2'-halogéno-pyrimidine qui n'est ni le premier nucléotide anti-sens 5'-terminal, ni le deuxième nucléotide anti-sens 5'-terminal, ni le troisième nucléotide anti-sens 5'-terminal, lequel premier nucléotide anti-sens 5'-terminal comporte une première modification anti-sens 2'-O-alkyle, lequel deuxième nucléotide anti-sens 5'-terminal comporte une deuxième modification anti-sens 2'-O-alkyle, et lequel troisième nucléotide anti-sens 5'-terminal comporte une troisième modification anti-sens 2'-O-alkyle,
étant entendu
- que ni le brin sens, ni le brin anti-sens n'est phosphorylé au niveau de l'atome de carbone en 5' de leur premier nucléotide 5'-terminal,
- que le brin sens et/ou le brin anti-sens peut ou peuvent porter un groupe bloquant au niveau de l'atome de carbone en 5' de leur premier nucléotide 5'-terminal,
- et que le brin sens et le brin anti-sens sont capables de former un duplex d'une longueur de 16 à 30 paires de bases.

12. Duplex d'ARN conforme à la revendication 11, dans lequel toutes les bases pyrimidines du brin anti-sens, autres que lesdits premier nucléotide anti-sens 5'-terminal, deuxième nucléotide anti-sens 5'-terminal et troisième nucléotide anti-sens 5'-terminal, comportent une modification 2'-halogéno.

13. Procédé de réalisation d'une interférence par ARN, comportant le fait d'introduire dans une cellule un duplex d'ARN conforme à l'une des revendications 1 à 12, en présence d'un duplex d'ARN qui est capable de rendre silencieux un gène cible, lequel procédé n'est ni un procédé de traitement chirurgical ou thérapeutique du corps d'un humain ou d'un animal, ni un procédé de diagnostic pratiqué sur le corps d'un humain ou d'un animal.


### SEQUENCE LISTING

<110> DHARMACON, INC LEAKE, Devin KHVOROVA, Anastasia FEDOROV, Yuriy DELANEY, Michael 0 ROBERTSON, Barbara ANDERSON, Emily
<120> Stabilized RNAs as Transfection Control
   and Silencing Reagents
<130> 13585PCT
<140> to be assigned
   <141> 2005-02-04
<150> 60/542646
   <151> 2004-02-06
<150> 60/543640
   <151> 2004-02-10
<150> 60/572270
   <151> 2004-05-18
<160> 55
<170> FastSEQ for Windows Version 4.0
<210> 1
   <211> 19
   <212> RNA
   <213> Artificial Sequence
<220>
   <223> siRNA strand
<400> 1
   gaaagagcau cuacgguga 19
<210> 2
   <211> 19
   <212> RNA
   <213> Artificial Sequence
<220>
   <223> siRNA strand
<400> 2
   gaaaggauuu ggcuacaaa 19
<210> 3
   <211> 19
   <212> RNA
   <213> Artificial Sequence
<220>
   <223> siRNA strand
<400> 3
   acagcaaauu ccaucgugu 19
<210> 4
   <211> 19
   <212> RNA
   <213> Artificial Sequence
<220>
   <223> siRNA strand
<400> 4
   ggaaagacug uuccaaaaa 19
<210> 5
   <211> 4
   <212> RNA
   <213> Artificial Sequence
<220>
   <223> siRNA strand
<400> 5
   uucg 4
<210> 6
   <211> 9
   <212> RNA
   <213> Artificial Sequence
<220>
   <223> siRNA strand
<400> 6
   uuuguguag 9
<210> 7
   <211> 10
   <212> RNA
   <213> Artificial Sequence
<220>
   <223> siRNA strand
<400> 7
   cuuccuguca 10
<210> 8
   <211> 19
   <212> RNA
   <213> Artificial Sequence
<220>
   <223> siRNA strand
<400> 8
   gcuuacauca acaaaguag 19
<210> 9
   <211> 19
   <212> RNA
   <213> Artificial Sequence
<220>
   <223> siRNA strand
<400> 9
   caaaguagaa gagcuaaag 19
<210> 10
   <211> 19
   <212> RNA
   <213> Artificial Sequence
<220>
   <223> siRNA strand
<400> 10
   agagagaucc ucauaaagg 19
<210> 11
   <211> 19
   <212> RNA
   <213> Artificial Sequence
<220>
   <223> siRNA strand
<400> 11
   guaacaaccg cgaaaaagu 19
<210> 12
   <211> 19
   <212> RNA
   <213> Artificial Sequence
<220>
   <223> siRNA strand
<400> 12
   ucaaguaaca accgcgaaa 19
<210> 13
   <211> 19
   <212> RNA
   <213> Artificial Sequence
<220>
   <223> siRNA strand
<400> 13
   aaagagcauc uacggugag 19
<210> 14
   <211> 19
   <212> RNA
   <213> Artificial Sequence
<220>
   <223> siRNA strand
<900> 14
   ucagagagau ccucauaaa 19
<210> 15
   <211> 19
   <212> RNA
   <213> Artificial Sequence
<220>
   <223> siRNA strand
<400> 15
   aaagacgaug acggaaaaa 19
<210> 16
   <211> 19
   <212> RNA
   <213> Artificial Sequence
<220>
   <223> siRNA strand
<400> 16
   uccaaaaaca guggauaau 19
<210> 17
   <211> 19
   <212> RNA
   <213> Artificial Sequence
<220>
   <223> siRNA strand
<400> 17
   cggugagcgc uuccccgau 19
<210> 18
   <211> 19
   <212> RNA
   <213> Artificial Sequence
<220>
   <223> siRNA strand
<400> 18
   uuuuguggcc uuagcuaca 19
<210> 19
   <211> 19
   <212> RNA
   <213> Artificial Sequence
<220>
   <223> siRNA strand
<400> 19
   ggcuacaaaa acagcaaau 19
<210> 20
   <211> 19
   <212> RNA
   <213> Artificial Sequence
<220>
   <223> siRNA strand
<400> 20
   uuugcggucg ccgucguug 19
<210> 21
   <211> 19
   <212> RNA
   <213> Artificial Sequence
<220>
   <223> siRNA strand
<400> 21
   aggggacgaa ggagggaag 19
<210> 22
   <211> 19
   <212> RNA
   <213> Artificial Sequence
<220>
   <223> siRNA strand
<400> 22
   gcacucugau ugacaaaua 19
<210> 23
   <211> 19
   <212> RNA
   <213> Artificial Sequence
<220>
   <223> siRNA strand
<400> 23
   ugaagucucu gauuaagua 19
<210> 24
   <211> 19
   <212> RNA
   <213> Artificial Sequence
<220>
   <223> siRNA strand
<400> 24
   gauaugggcu gaauacaaa 19
<210> 25
   <211> 19
   <212> RNA
   <213> Artificial Sequence
<220>
   <223> siRNA strand
<400> 25
   gaaagagcau cuacgguga 19
<210> 26
   <211> 19
   <212> RNA
   <213> Artificial Sequence
<220>
   <223> siRNA strand
<400> 26
   gaaaggauuu ggcuacaaa 19
<210> 27
   <211> 19
   <212> RNA
   <213> Artificial Sequence
<220>
   <223> siRNA strand
<400> 27
   acagcaaauu ccaucgugu 19
<210> 28
   <211> 19
   <212> RNA
   <213> Artificial Sequence
<220>
   <223> siRNA strand
<400> 28
   ggaaagacug uuccaaaaa 19
<210> 29
   <211> 19
   <212> RNA
   <213> Artificial Sequence
<220>
   <223> siRNA strand
<400> 29
   ggccuuagcu acaggagag 19
<210> 30
   <211> 19
   <212> RNA
   <213> Artificial Sequence
<220>
   <223> siRNA strand
<400> 30
   gaaaaaucag agagauccu 19
<210> 31
   <211> 19
   <212> RNA
   <213> Artificial Sequence
<220>
   <223> siRNA strand
<400> 31
   uaccggaaaa cucgacgca 19
<210> 32
   <211> 19
   <212> RNA,
   <213> Artificial Sequence
<220>
   <223> siRNA strand
<400> 32
   acgucgccag ucaaguaac 19
<210> 33
   <211> 19
   <212> RNA
   <213> Artificial Sequence
<220>
   <223> siRNA strand
<400> 33
   gauuacgucg ccagucaag 19
<210> 34
   <211> 19
   <212> RNA
   <213> Artificial Sequence
<220>
   <223> siRNA strand
<400> 34
   agagaucgug gauuacguc 19
<210> 35
   <211> 19
   <212> RNA
   <213> Artificial Sequence
<220>
   <223> siRNA strand
<400> 35
   uguuguuuug gagcacgga 19
<210> 36
   <211> 21
   <212> RNA
   <213> Artificial Sequence
<220>
   <223> siRNA strand
<400> 36
   uagcgacuaa acacaucaau u 21
<210> 37
   <211> 19
   <212> RNA
   <213> Artificial Sequence
<220>
   <223> siRNA strand
<400> 37
   gcgacuaaac acaucaauu 19
<210> 38
   <211> 21
   <212> RNA
   <213> Artificial Sequence
<220>
   <223> siRNA strand
<400> 38
   gaaagagcau cuacggugau u 21
<210> 39
   <211> 21
   <212> RNA
   <213> Artificial Sequence
<220>
   <223> siRNA strand
<400> 39
   ugguuuacau guuccaauau u 21
<210> 40
   <211> 19
   <212> RNA
   <213> Artificial Sequence
<220>
   <223> siRNA strand
<400> 40
   guuuacaugu uccaauauu 19
<210> 41
   <211> 21
   <212> RNA
   <213> Artificial Sequence
<220>
   <223> siRNA strand
<400> 41
   ugguuuacau guuccaauau u 21
<210> 42
   <211> 21
   <212> RNA
   <213> Artificial Sequence
<220>
   <223> siRNA strand
<400> 42
   ucaugggugu gaaccaugau u 21
<210> 43
   <211> 19
   <212> RNA
   <213> Artificial Sequence
<220>
   <223> siRNAs strand
<400> 43
   gcgacuaaac acaucaauu 19
<210> 44
   <211> 19
   <212> RNA
   <213> Artificial Sequence
<220>
   <223> siRNA strand
<400> 44
   guuccaaaaa caguggaua 19
<210> 45
   <211> 19
   <212> RNA
   <213> Artificial Sequence
<220>
   <223> siRNA strand
<400> 45
   caaaaacagu ggauaauuu 19
<210> 46
   <211> 19
   <212> RNA
   <213> Artificial Sequence
<220>
   <223> siRNA strand
<400> 46
   aaaacagugg auaauuuug 19
<210> 47
   <211> 19
   <212> RNA
   <213> Artificial Sequence
<220>
   <223> siRNA strand
<400> 47
   guuccaaaaa caguggaua 19
<210> 48
   <211> 19
   <212> RNA
   <213> Artificial Sequence
<220>
   <223> siRNA strand
<400> 48
   uccaaaaaca guggauaau 19
<210> 49
   <211> 19
   <212> RNA
   <213> Artificial Sequence
<220>
   <223> siRNA strand
<400> 49
   caaaaacagu ggauaauuu 19
<210> 50
   <211> 19
   <212> RNA
   <213> Artificial Sequence
<220>
   <223> siRNA strand
<400> 50
   aaaacagugg auaauuuug 19
<210> 51
   <211> 19
   <212> RNA
   <213> Artificial Sequence
<220>
   <223> siRNA strand
<400> 51
   ggccuuagcu acaggagag 19
<210> 52
   <211> 19
   <212> RNA
   <213> Artificial Sequence
<220>
   <223> siRNA strand
<400> 52
   uuccaucgug uaaucaagg 19
<210> 53
   <211> 19
   <212> RNA
   <213> Artificial Sequence
<220>
   <223> siRNA strand
<400> 53
   ucaugaucca gggcggaga 19
<210> 54
   <211> 19
   <212> RNA
   <213> Artificial Sequence
<220>
   <223> siRNA strand
<400> 54
   aaagagcauc uacggugag 19
<210> 55
   <211> 20
   <212> RNA
   <213> Artificial Sequence
<220>
   <223> siRNA strand
<400> 55
   auuguagcga ucgcagacuu 20